# EUROPEAN PATENT APPLICATION

(11) **EP 4 248 958 A2**
(43) Date of publication of application: **27.09.2023**
(21) Application number: 23191736.0
(22) Date of filing: 27.01.2022
(51) Int. Cl.: A61K 31/13

(54) **METHODS OF TREATMENT WITH S1P RECEPTOR MODULATORS**

(30) Priority: 28.01.2021 WO PCT/IB2021/000033; 27.09.2021 EP 21199256
(62) Divisional of application: 22702938.6
(71) Applicant: Priothera SAS, 68300 Saint-Louis (FR); Priothera Limited, D02 DK18 Dublin 2 (IE)
(72) Inventor: BUCHER, Christoph, 4402 FRENKENDORF (CH); DERTSCHNIG, Simone, 4054 BASEL (CH)
(74) Representative: Plasseraud IP

(57) **Abstract**

The present invention relates to S1P receptor modulators, preferably mocravimod, for use in treating patients suffering from acute myelogenous leukemia (AML) and who have undergone allogeneic hematopoietic stem cell transplantation (HSCT). The invention relates in particular to methods of treating AML in subjects undergoing HSCT, wherein said method comprises daily administering an efficient amount of S1P receptor modulator, preferably mocravimod, to said subject in need thereof, for at least 6 months, preferably at least 12 months.

## Description

### TECHNICAL FIELD

The present invention relates to S1P receptor modulators, preferably mocravimod, for use in treating patients suffering from acute myelogenous leukemia and who have undergone allogeneic hematopoietic stem cell transplantation.

### BACKGROUND ART

Acute myeloid leukemia (AML) is an aggressive, fast-growing, disease in which too many myeloblasts (immature white blood cells that are not lymphoblasts) are found in the bone marrow and blood. Also called acute myeloblastic leukemia, acute myelogenous leukemia, acute nonlymphocytic leukemia, AML, and ANLL. AML results when bone marrow begins making blasts instead of mature white blood cells. The immature blasts are unable to fight infections. AML is the most common acute leukemia, and it progresses rapidly. Left untreated, AML may lead to death within weeks or months.

Current treatments for AML include chemotherapy, including targeted chemotherapies, radiation therapy, and autologous hematopoietic stem cell transplantation or allogeneic hematopoietic stem cell transplantation. The side effects of each of these treatments are well documented. Typically, a newly diagnosed AML patient will be treated with an induction chemotherapy regimen to attempt to put the cancer in remission. Remission, however, is most often a temporary measure as most AML patients in remission eventually relapse. Post-remission therapies include consolidation chemotherapy, allogenic hematopoietic stem cell transplantation, or autologous hematopoietic stem cell transplantation.

To date, allogeneic hematopoietic stem cell transplantation (HSCT) remains the only treatment option for possible cure. However, major complications of HSCT include graft-versus-host disease (GVHD) and other life-threatening complications. Even with the use of immunosuppressive prophylaxis, 30 to 60% of patients will develop some level of acute GVHD (Abo-Zena and Horwitz, Curr Opin Pharmacol. 2002 Aug;2(4):452-7; Jagasia et al., Blood 2012 Jan 5;119(1):296-307; Ruggeri et al., J Hematol Oncol. 2016 Sep 17;9(1):89).

Immunosuppressive GVHD prophylaxis is based for example on Ciclosporin A (CsA) which is a Calcineurin inhibitor (CNI) and Methotrexate (MTX) as a well established immunosuppressive regimen. Criteria for decision making to proceed to allo-HSCT in patients in first Complete Remission (CR) include risk group classification at diagnosis based on prognostic factors, and donor availability, at the time of induction. Some criteria are added at the time of consolidation, such as the quality of remission and persistence of Measurable Residual Disease (MRD), co-morbidities (HCT-CI as index of comorbidity) and recipient-donor profile (Gratwohl score).

The European Leukemia Network (ELN) has issued a guidance for stratification based on prognostic in AML (Döhner et al., Blood 2017 Jan 26;129(4):424-447) with 3 groups: Favourable, Intermediate and Adverse.

Based on the stratification, the ELN has issued guidance for allo-HSCT eligibility in patients with intermediate/adverse risk and an additional stratification to assess post remission therapies: Low, Intermediate, High risk of relapse (Döhner et al., Blood 2017 Jan 26;129(4):424-447). For instance, HSCT is indicated for patients in first CR with adverse cytogenetic/ molecular characteristics and/ or high MRD, provided HCT-CI is good enough and provided a donor is available in a timely manner.

While acute GVHD is an inflammatory disease, chronic GVHD resembles an autoimmune syndrome and typically develops 4 to 6 months posttransplant due to antigen-specific donor immune cells that cause autoimmune clinical manifestations. Chronic GVHD can occur independently from acute GVHD as the classic form or as an overlap syndrome with the presence of acute features. The pathology of acute GVHD is characterized by T-cell infiltration causing tissue inflammation and damage and is a risk factor for the later development of chronic GVHD, which is a fibrotic and sclerotic disease. It remains, however, unknown if acute and chronic GVHD are directly connected, and if so, how they are mechanistically linked.

One potential mechanism, amongst others, that can facilitate the transition from acute to chronic GVHD, is defective thymic function in acute GVHD. Indeed, pre-clinical data has shown that a breakdown of tolerance induction during acute GVHD causes the emergence of autoreactive T cells from the thymus to the periphery (Dertschnig S, et al. Blood. 2013;122(5):837-841; Dertschnig S, et al. Blood. 2015;125(17):2720-2723).

Once these autoreactive T cells are in the periphery, they may have the potential to cause the autoimmune syndrome typically observed during chronic GVHD.

Sphingosine-1-phosphate (S1P) is a metabolite of sphingolipid, a component of biomembrane. S1P acts as a ligand for a family of five G-protein-coupled receptors, named S1P receptor types 1 to 5 (S1PR1-5). In preclinical studies, FTY720 (fingolimod), a multi-S1PR inhibitor of S1PR1, 3-5, has been shown to ameliorate acute GVHD after allogeneic hematopoietic stem cell transplantation (HSCT) by inhibiting donor T-cell infiltration to acute GVHD target organs and to facilitate rapid contraction of donor T-cell pool in association with an increased donor T-cell apoptosis (Kim et al. J Clin Invest. 2003; 111: 659-669. Hashimoto D, et al. Eur J Immunol. 2007; 37: 271-281).

In contrast to FTY720 which displays poor selectivity for S1PR1 versus S1PR3-5, the compound KRP203 (mocravimod) acts specifically on S1PR1 with a potentially milder toxicity profile. Yokoyama et al have shown that short-term administration of KRP203 alone induced apoptosis of donor T cells in the secondary lymphoid organs and ameliorated acute GVHD (Yokoyama E, et al. Bone Marrow Transplant. 2020; 55: 787-795). To the knowledge of the inventors, a role of S1P receptor modulator has never been shown in the prevention or treatment of chronic GVHD, in particular in human patients undergoing HSCT.

Calcineurin inhibitors such as CsA and tacrolimus (TAC) suppress donor T-cell activation and remain the most commonly used immunosuppressants for acute GVHD prophylaxis. WO2020/022507 discloses the results of a clinical study for treating patients with hematological malignancies, where said patients undergo HSCT and are treated with KRP203 daily for a maximum duration of 110 days, together with standard of care to prevent acute GVHD with immunosuppressants, such as CsA and MTX, or MTX and TAC.

These immunosuppressive agents, however, also inhibit leukemia-specific T-cell responses leading to impaired Graft vs Leukemia (GVL) effect. Indeed, Yokoyama E, et al. previously showed that prolonged administration of CsA after mouse HSCT significantly impaired GVL effects compared to KRP203 (Yokoyama E, et al. Bone Marrow Transplant. 2020; 55: 787-795).

WO2020/022507 suggested that short-term administration of KRP203 significantly reduced risk of acute GVHD and improved the survival of patients with hematological malignancies.

However, there is still a need to improve the survival of patients with high-risk of relapse. In particular, relapse is currently the leading cause of treatment failure after allogeneic HSCT, while transplant-related mortality and morbidity due to GVHD is still an issue, despite some progress in GVHD therapy.

There is also still a need to find an optimal stable formulation with a S1Pr drug, stability is an essential quality attribute for pharmaceutical formulations. Predictability of the optimal formulation, particularly for solid state form, remains a challenge in the industry (Curr Pharm Des. 2016;22(32):5019-5028).

In particular, in AML, there is still a need to provide a therapy capable of de-coupling GVL and acute GVHD for improving relapse rate in patients, while lowering chronic GVHD incidence and severity.

Without limiting the invention to any particular mechanism, the invention described here meets this need, as the described methods provide a synergistic therapeutic effect, preventing acute and chronic GVHD effects while further improving GVL effect and risk of relapse in high-risk AML patients.

### SUMMARY

Accordingly, a first object of the invention relates to an S1P receptor modulator, preferably mocravimod, for use in treating a human subject suffering from acute myelogenous leukemia (AML) and undergoing allogeneic hematopoietic stem cell transplant (HSCT).

Another object of the invention relates to an S1P receptor modulator, preferably mocravimod, for use in treating chronic GVHD, typically for delaying chronic GVHD onset, in a human subject undergoing allogeneic hematopoietic stem cell transplant (HSCT), preferably in a human subject in need of HSCT for treating acute myelogenous leukemia.

Another object of the invention relates to a pharmaceutical composition comprising an S1P receptor modulator of formula (II) or formula (IIa) or formula (IIb): 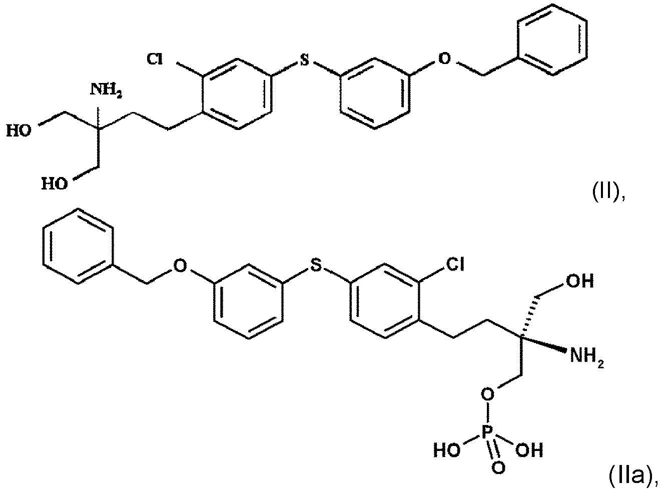 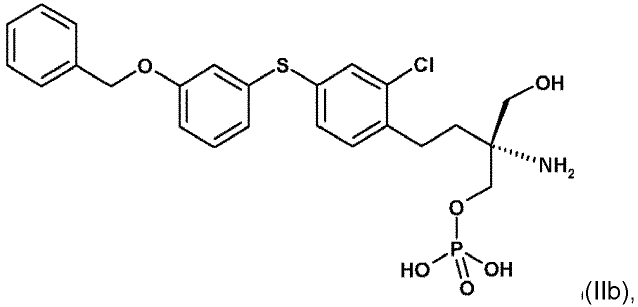
or pharmaceutically acceptable salts thereof, and
at least one filler selected from mannitol, microcrystalline cellulose and mixtures thereof, sodium starch glycolate as disintegrant, magnesium stearate as lubricant and, colloidal silicon dioxide as glidant.

The present disclosure relates in particular to various methods of treating acute myelogenous leukemia in a subject undergoing allogeneic hematopoietic stem cell transplant (HSCT) comprising:
1) administering to the subject an effective amount of an S1P receptor modulator,
2) conditioning said subject for destroying substantially the bone marrow and immune system wherein said conditioning includes treatment of said subject with an effective amount of a chemotherapeutic agent and/or performing total body irradiation;
3) transplanting allogeneic hematopoietic stem cells from a donor to said subject; and,
4) optionally, co-administering an efficient amount of one or more immunosuppressants to prevent acute GVHD.

The present disclosure further relates to a method of preventing chronic GvHD in a subject undergoing allogeneic hematopoietic stem cell transplant (HSCT) comprising:
1) administering to the subject an effective amount of an S1P receptor modulator,
2) conditioning said subject for destroying substantially the bone marrow and immune system wherein said conditioning includes treatment of said subject with an effective amount of a chemotherapeutic agent and/or performing total body irradiation;
3) transplanting allogeneic hematopoietic stem cells from a donor to said subject; and,
4) optionally, co-administering an efficient amount of one or more immunosuppressants to prevent acute GVHD.

The disclosure further relates to the use of an S1P receptor modulator in the manufacture of a medicament for treating acute myelogenous leukemia in a subject undergoing allogenic hematopoietic stem cell transplant.

The disclosure also relates to the use of an S1P receptor modulator in the manufacture of a medicament for preventing chronic GVHD, typically delaying chronic GVHD onset, in a patient undergoing allogeneic hematopoietic stem cell transplant.

In some embodiments of the various methods and use described herein, the S1P receptor modulator is selected among mocravimod, FTY720, siponimod, fingolimod, ozanimod, ponesimod, etrasimod, AKP-11, cenerimod, amiselimod, CBP-307, OPL-307, OPL-002, BMS-986166, SCD-044, BOS-173717, CP-1050, typically selected among mocravimod, FTY720 and siponimod, and most preferably mocravimod. Preferably said S1P receptor modulator is a S1P receptor agonist. More preferably, the S1P receptor modulator is of formula (I) or (II) : 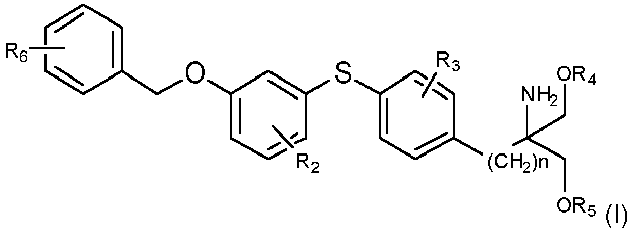 wherein
R₂ is H, halogen, trihalomethyl, C₁₋₄alkoxy, C₁₋₇alkyl, phenethyl or benzyloxy;
R₃ is H, halogen, CF₃, OH, C₁₋₇alkyl, C₁₋₄alkoxy, benzyloxy, phenyl or C₁₋₄alkoxymethyl;
each of R₄ and R₅, independently is H or a residue of formula (a) 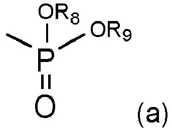
wherein each of R₈ and R₉, independently, is H or C₁₋₄alkyl optionally substituted by halogen;
and n is an integer from 1 to 4; and
R₆ is hydrogen, halogen, C₁₋₇alkyl, C₁₋₄alkoxy or trifluoromethyl, 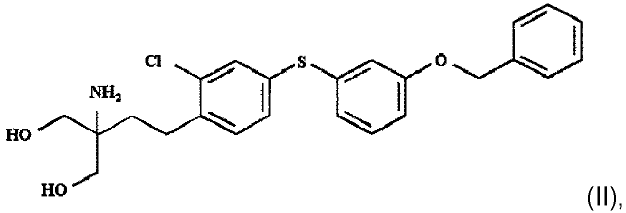
or pharmaceutically acceptable salts thereof, and more preferably mocravimod or pharmaceutically acceptable salts thereof.

In some embodiments of the various methods and use described herein, the S1P receptor modulator is selected to have a pharmacokinetic half-life over 40, 50, 60, 70, 90 hours, preferable over 100 hours, such as mocravimod or fingolimod, hence maximizing drug exposure in patients, thereby allowing to skip a S1Pr administration's dose over one or more days in case of toxicities caused by the conditioning procedure, such as bone marrow suppression, alopecia, nausea, vomiting, parotid swelling and erythema. In particular nausea and/or vomiting is causing patients not able to swallow therapies such as oral dosage of a S1Pr drug, hence using a long pharmacokinetic half-life S1Pr drug may be advantageous for the patients.

In some embodiments, the administration of the S1P receptor modulator, preferably mocravimod, is started prior to allogenic HSCT, preferably 7, 8, 9, 10, 11, 12, 13, or 14 days prior to HSCT, more preferably 11 days prior to HSCT.

In some embodiments, the S1P receptor modulator, preferably mocravimod, is daily administered for a period set to at least 80 days, or at least 100 days or more, after the HSCT.

For preferred embodiments, the S1P receptor modulator, most preferably mocravimod, is daily administered for at least 6, 7, 8, 9, 10, 11, 12, 18, or 24 months, or more, preferably during the life of the subject or until relapse, i.e. chronic daily treatment.

For example, the S1P receptor modulator, most preferably mocravimod, is daily administered from 1 to 14 days prior to HSCT, preferably from 11 days prior to HSCT, for at least 6, 7, 8, 9, 10, 11, 12, 18, or 24 months, or more, or until relapse, after HSCT.

In any of the methods and use described herein, the amount of S1P receptor modulator, most preferably mocravimod, can be administered per day at a fixed amount. Preferably said fixed daily dosage is 0,05 mg to 40 mg per day, preferably 0,1 mg to 35 mg, more preferably 0,5 mg to 30 mg, even more preferably 1 mg to 15 mg per day, even more preferably 1,5 mg to 7mg, even more preferably 2 mg to 5 mg, even more preferably about 3 mg per day or about 1 mg per day.

For example, the S1P receptor modulator can be mocravimod and said mocravimod may be administered at a daily dose of about 1 mg per day. Alternatively, mocravimod may be administered at a dose of about 3mg per day, preferably as three solid dosage forms of about 1mg or as one solid dosage form of about 3mg. Alternatively, mocravimod may be administered at a dose of about 2mg per day, preferably as two solid dosage forms of about 1mg or as one solid dosage form of about 2mg.

In specific embodiments, the conditioning regimen at step 2) includes a chemotherapy with a chemotherapeutic agent selected from the group consisting of cyclophosphamide or thiotepa, cytarabine, etoposide, busulfan or melphalan, fludarabine, and mixtures thereof such as a combined administration of fludarabine/busulfan, busulfan/cyclophosphamide, or fludarabine/melphalan.

In specific embodiments, the conditioning regimen is as follow:
- intravenous administration of an effective amount of Fludarabine; preferably at 30 mg/m2 once daily for 5 days on Day -8 to -4 (total 150 mg/m2)
- intravenous administration of an effective amount of Thiotepa; preferably at 5 mg/kg IV twice daily for 1 day on Day -7 (total 10 mg/kg)
- intravenous administration of Melphalan; preferably at 60 mg/m2 IV once daily for 2 days on Day -2 and -1 (total 120 mg/m2)
wherein the day numbers are relative to the day of HSCT.

In other specific embodiments, the conditioning regimen at step 2) consists in :
i. the administration of cyclophosphamide followed by total body irradiation, or
ii. the administration of busulfan and cyclophosphamide, or
iii. the administration of fludarabine and busulfan, and optionally, total body irradiation with a low dosage.

In specific embodiments, the hematopoietic stem cells are selected in step 3) from HLA-matched related or unrelated donor with 8/8 or higher matches at the HLA-A, -B, -C, -DRB1, and/or-DQB1 loci, as determined by high resolution HLA typing.

In specific embodiments, at step 4) of the methods, the one or more immunosuppressants is selected from the group consisting of ciclosporin A, sirolimus, tacrolimus, methotrexate, and mycophenolate mofetil, preferably ciclosporin A or a mixture and methotrexate or tacrolimus or a mixture of tacrolimus and methotrexate. In a specific embodiment, the immunosuppressant is at least ciclosporin A. In another specific embodiment, the immunosuppressant is at least tacrolimus.

For example, ciclosporin A can be administered within a period between the starting day of administration of the S1P receptor modulator, preferably mocravimod, and the day of HSCT, preferably 3 days prior to HSCT. In specific embodiments, the ciclosporin A is administered intravenously or orally at an initial dosage of 2.5 mg/kg over 2 hours every 12 hours and optionally adjusted between 150 to 400 mg/L.

For example, tacrolimus can be administered within a period between the starting day of administration of the S1P receptor modulator, preferably mocravimod, and the day of HSCT, preferably 3 days prior to HSCT. In specific embodiments, the tacrolimus is administered intravenously or orally at an initial dosage of 0.02-0.03 mg/kg/day either as continuous infusion or divided between 2 bolus infusions twice a day. In an embodiment the dose injected should be so as to maintain target blood concentrations of 5-10 ng/mL.

In specific embodiments, methotrexate is administered together with ciclosporin A or tacrolimus the day of HSCT at a dosage of 10 mg/kg of and optionally 2 and 5 days after the first administration and the 16th day therefrom, at a dosage of 6 mg/kg. In another embodiment, methotrexate is administered the day after HSCT at a dosage of 15 mg/m2 IV once, and then 3 days, 6 days and 11 days after HSCT at a dosage of 10 mg/m2 IV once. In the latter embodiment, the use of leucovorin rescue is allowable and encouraged at the same dose as methotrexate given every 6 hours for 3 doses starting 24 hours after methotrexate dose; given orally or IV. Modifications of this regimen can be done to accommodate decreased clearance or methotrexate toxicity.

In a preferred embodiment, the one or more immunosuppressants used in step 4) do not include tacrolimus. In another preferred embodiment, the one or more immunosuppressants used in step 4) do not include ciclosporin A.

In specific embodiments of the methods and use, the S1P receptor modulator, preferably mocravimod, is administered for a period of at least 6, 7, 8, 9, 10, 11, 12, 18, 24 months, or more, preferably during the life of the subject or until relapse, said subject being further treated in step 4) with an efficient amount of immunosuppressants including at least ciclosporin A, and said immunosuppressants treatment is reduced or stopped prior to 6 months following HSCT, preferably within a period from 3 to 6 months, or from 3 to 5 months, or from 3 to 4 months following HSCT. For example, said immunosuppressants treatment is reduced of at least 10%, 20%, 30%, 40%, 50%, 60%, 70%, 80%, 90%, 100% compared to the starting dose of said immunosuppressant.

In specific embodiments of the methods and use disclosed herein, said subject is with refractory or relapsed AML after one or more AML therapy.

In specific embodiments, said subject is selected among the patients suffering of acute myelogenous leukemia and being either in first complete morphological remission but in the adverse-risk group called "CR1 high risk", or in second and subsequent complete morphological remission called "CR2", wherein said CR1 high risk, CR2 patients are defined according to ASBMT RFI 2017 - Disease Classifications Corresponding to CIBMTR Classifications of the American Society for Blood and Marrow Transplantation, preferably patients classified as CR1 high risk and CR2.

In specific embodiments, the subject is measurable residual disease (MRD)-positive prior to being administered the method of the present disclosure (e.g.HSCT with mocravimod). Accordingly, in specific embodiment, prior to step (1), the method includes detecting the MRD status of a subject.

In specific embodiments, said S1P receptor modulator is administered in an amount sufficient to treat AML and to prevent acute GVHD.

In specific embodiments, said S1P receptor modulator is administered in an amount sufficient to treat AML and to prevent acute and chronic GVHD.

Preferably, said patient is refractory GVHD-free, and relapse free at 3, 4, 5, 6, 7, 8, 9, 10, 11, 12, 13, 14, 15, 16, 17, 18, 19, 20, 21, 22, 23, or 24 months from HSCT.

In specific embodiments of the methods and use, said subject does not present one or more of the following:
- grade III/IV acute graft-versus-host disease (GVHD) refractory to at least 2 lines of immunosuppressive treatment,
- extensive chronic GVHD refractory to systemic immunosuppressive treatment,
- disease relapse,
- death,
after at least 3, 4, 5, 6, 7, 8, 9, 10, 11, 12, 13, 14, 15, 16, 17, 18, 19, 20, 21, 22, 23, or 24 months from HSCT.

In specific embodiments, said methods and use described herein improves morbidity or mortality in a population of subjects, in particular via refractory GVHD-free, relapse-free survival (rGRFS) and both relapse-related mortality and transplant-related mortality at 3, 4, 5, 6, 7, 8, 9, 10, 11, 12, 13, 14, 15, 16, 17, 18, 19, 20, 21, 22, 23, 24 months from HSCT.

For example, said methods and use reduce the occurrence, or severity of either GVHD, refractory GVHD, relapse or mortality during the next 12, 18 or 24 months after HSCT in a population of subjects.

Also disclosed herein are methods for improving overall survival of subjects, said method comprising, for each subject, treating the subject with any of the method using the S1P receptor modulator as disclosed herein.

In specific embodiments, said method improves the quality of life in a population of subjects, preferably as measured by the Fundation for the Accreditation of Cellular Therapy Bone Marrow Transplantation (FACT-BMT) questionnaire and/or the MD Anderson symptom inventory (MDASI), at 3, 6, 12 or 24 months as compared with the same method of HSCT without administration of said S1PR modulator. Improvement may be significant, and for example the quality of life may increase of at least 10%, 20%, 30%; 40%, 50%, 60%, 70%, 80%, 90%, 100%, or more.

### DETAILED DESCRIPTION

The disclosure relates to an S1P receptor modulator, preferably mocravimod, for use in treating a human subject suffering from acute myelogenous leukemia (AML) and undergoing allogeneic hematopoietic stem cell transplant (HSCT).

### General Definitions

As used herein, a "modulator" is a compound which, when administered to a subject, provides the desired interaction with the target receptor, either by way of the compound acting directly on the receptor itself, or by way of a metabolite of the compound acting on the receptor. Upon administration to a subject, the S1P receptor modulator, preferably mocravimod, interacts with the S1P receptor by either activating or inhibiting the receptor for signal transduction.

As used herein, "S1P agonist" refers to a compound which initiates a physiological response when combined with the S1P receptor. Preferably the physiological response initiated is the agonist-induced internalization of the S1P receptor. The kinetics of agonist-induced internalization from cell membranes, and the recycling of the S1P receptors to the cell membrane after said compound shedding depends on the compound. Such S1P receptor agonists may also be referred as functional antagonists. The persistence of the internalization conditions the agonist's "functional antagonism" properties.

As used herein, HSC refers to hematopoietic stem cell.

As used herein, HSCT refers to allogeneic hematopoietic stem cell transplantation.

The term "pharmaceutically acceptable salts thereof" includes both acid and base addition salts. Non-limiting examples of pharmaceutically acceptable acid addition salts include chlorides, hydrochlorides, bromides, sulfates, nitrates, phosphates, sulfonates, methane sulfonates, formates, tartrates, maleates, citrates, benzoates, salicylates, and ascorbates. Non-limiting examples of pharmaceutically acceptable base addition salts include sodium, potassium, lithium, ammonium (substituted and unsubstituted), calcium, magnesium, iron, zinc, copper, manganese, and aluminum salts. Pharmaceutically acceptable salts may, for example, be obtained using standard procedures well known in the field of pharmaceuticals. For mocravimod, pharmaceutically acceptable salts would typically be acid-addition salts, since mocravimod is itself a base. Preferably, the pharmaceutically acceptable salts thereof is hydrochloride salt.

The term "excipient", as used herein, refers to a non-active substance that is added alongside the drug substance, and is part of the formulation mixture. Pharmaceutically acceptable excipient are for example fillers, solvents, diluents, carriers, auxiliaries, distributing and sensing agents, delivery agents, such as preserving agents, disintegrants, moisteners, emulsifiers, suspending agents, thickeners, sweeteners, flavoring agents, aromatizing agents, antibacterial agents, fungicides, lubricants, and prolonged delivery controllers, antioxidants, glidants. The choice and suitable proportions of them are depended on the nature and way of administration and dosage.

An "average particle size" refers here to the D50 which means that 50% of the particles have size less than or equal to the indicated value. For instance, an average particle size less than or equal to 8 µm refers to a D50 of 8 µm, i.e. 50% of the particles have a particle size less than or equal to 8 µm. The term D90 means that 90% of the particles have a particle size less than or equal to the indicated value. For instance, a D90 less than or equal to 25 µm means that 90% of the particles have a particle size less than or equal to 25 µm. The D50 as well as the D90 are determined by laser light diffraction using the liquid route, e.g. on a BECKMAN-COULTER laser diffraction particle size analyzer LS 230, equipped with its small volume dispersion module (liquid route), following the technical manual and the manufacturer's instructions.

The terms "effective amount" or "therapeutically effective amount" refers to an amount of an active principle ingredient, for example, an S1P receptor modulator, preferably mocravimod, that when administered to a subject, either as a single dose or as part of a series of doses, is effective to produce at least one therapeutic effect, either alone or in combination with other active agent.

The term "about" has herein the meaning that the following value may vary for ± 20%, preferably ± 10%, more preferably ± 5%, even more preferably ± 2%, even more preferably ±1%.

The terms "patient", "subject", "individual", and the like, are used interchangeably herein, and refer to a human. In some embodiments, the patient, subject or individual in need of treatment includes those who already have the disease, condition, or disorder, i.e. Acute Myelogenous Leukemia.

"Combination" refers to either a fixed combination in one dosage unit form, or a combined administration where a compound of the present disclosure and a combination partner (e.g. another drug as explained below, also referred to as "therapeutic agent" or "co-agent") may be administered independently at the same time or separately within time intervals, especially where these time intervals allow that the combination partners show a cooperative, e.g. synergistic effect. The single components may be packaged in a kit or separately. One or both of the components (e.g., powders or liquids) may be reconstituted or diluted to a desired dose prior to administration.

The terms "co-administration" or "combined administration" or the like as utilized herein are meant to encompass administration of the selected combination partner to a single subject in need thereof (e.g. a patient), and are intended to include treatment regimens in which the agents are not necessarily administered by the same route of administration or at the same time.

As used herein, unless specified otherwise, the term "treating" or "treatment", denotes reversing, alleviating, inhibiting the progress of, or preventing the disorder or condition to which such term applies, or reversing, alleviating, inhibiting the progress of, or preventing one or more symptoms of the disorder or condition to which such term applies.

As used herein, the term "AUClast" is defined as the concentration measured from time 0 to the last measurable concentration.

### S1P receptor modulators for use as active principal ingredients in the treatment methods of the disclosure

S1P receptors are divided into five subtypes related G-coupled protein receptors (i.e., S1P₁, S1P₂, S1P₃, S1P₄ and S1P₅), which are expressed in a wide variety of tissues and exhibit different cell specificity.

In certain embodiments, a modulator of the S1P receptor for use according to the present methods of the disclosure is a compound which modulates one or more of the five S1P receptor types 1 to 5 (S1PR₁₋₅) by activating or inhibiting the receptor for signal transduction. Such compounds are also referred to herein as "S1P agonists" and "S1P inhibitor" respectively.

In specific embodiments, said S1P receptor modulator for use in the treatment methods of the present disclosure is selected among KRP203 (mocravimod), FTY720 (fingolimod), siponimod (Mayzent^{®}), fingolimod (Gilenya^{™}), ozanimod (Zeposia^{®}), ponesimod, etrasimod, AKP-11, cenerimod, amiselimod, CBP-307, OPL-307, OPL-002, BMS-986166, SCD-044, BOS-173717, CP-1050. Preferably, said S1P receptor modulator for use in the treatment methods of the present disclosure is selected among KRP203 (mocravimod), FTY720 (fingolimod), and Mayzent^{®} (siponimod), most preferably mocravimod.

In an embodiment, said S1P receptor modulator for use in the treatment methods of the present disclosure is a S1P agonist. Examples of such S1P agonist is KRP203 (mocravimod), a S1PR₁ selective agonist, or FTY720 (fingolimod), a multi-S1PR agonist of S1PR₁, ₃₋₅ or siponimod, a S1PR₃ agonist, or any of their pharmaceutically acceptable salts. Preferably, in certain embodiments, said S1P agonist is selected among those S1P agonists activating selectively S1PR₁.

In a preferred embodiment, the S1P receptor modulator for use according to the present disclosure is the compound of formula (I): 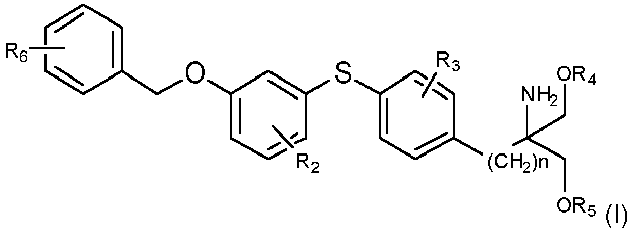 Wherein
R₂ is H, halogen, trihalomethyl, C₁₋₄alkoxy, C₁₋₇alkyl, phenethyl or benzyloxy;
R₃ is H, halogen, CF₃, OH, C₁₋₇alkyl, C₁₋₄alkoxy, benzyloxy, phenyl or C₁₋₄alkoxymethyl;
each of R₄ and R₅, independently is H or a residue of formula (a) 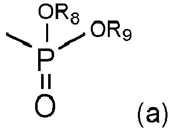
wherein each of R₈ and R₉, independently, is H or C₁₋₄alkyl optionally substituted by halogen;
and n is an integer from 1 to 4; and
R₆ is hydrogen, halogen, C₁₋₇alkyl, C₁₋₄alkoxy or trifluoromethyl.

In specific embodiments, said compoud of formula (I) is an S1P agonist, preferably an S1PR₁ selective agonist. Typically, in preferred embodiments, R₃ is chlorine. More preferably, R₂ is H, R₃ is chlorine and R₆ is hydrogen. For example, R₂ is H, R₃ is chlorine, R₆ is hydrogen, and each of R₃ and R₅, independently is H.

In a more preferred embodiment, the S1P receptor modulator for use according to the present disclosure, preferably an S1P agonist, preferably S1PR₁ selective agonist, is 2-amino-2- [4- (3-benzyloxyphenylthio) -2-chlorophenyl] ethyl-propane-1,3-diol, of formula (II) (also referred as mocravimod or KRP203): 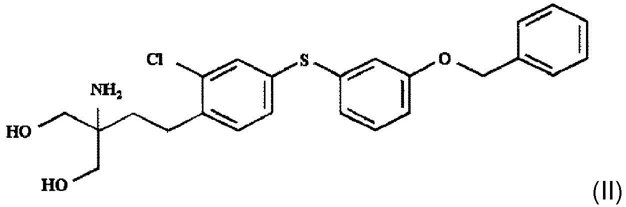 or pharmaceutically acceptable salts thereof.

Other S1P receptor modulator for use according to the present disclosure, preferably S1PR₁ selective agonist, includes the phosphate derivatives of the following formulae: 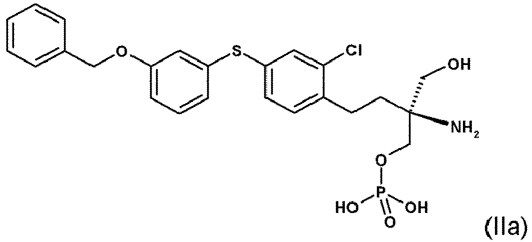 or

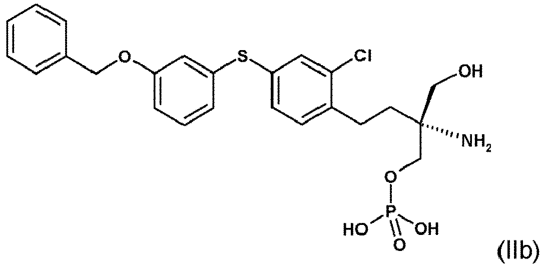

In some embodiments of the various methods and use described herein, the S1P receptor modulator is selected to have a pharmacokinetic half-life over 40, 50, 60, 70, 90 hours, preferable over 100 hours, such as mocravimod or fingolimod, hence maximizing drug exposure in patients, thereby allowing to skip a S1Pr administration's dose over one or more days in case of toxicities caused by the conditioning procedure, such as bone marrow suppression, alopecia, nausea, vomiting, parotid swelling and erythema. In particular nausea and/or vomiting is causing patients not able to swallow therapies such as oral dosage of a S1Pr drug, hence using a long PK half-life S1Pr drug may be advantageous for the patients.

Said compounds and their synthesis methods are also disclosed in WO03/029205, WO2004/074297, WO2006/009092, WO2006/041019 and WO2014128611A1 (which disclosures are incorporated herein by reference).

Mocravimod is particularly preferred. Indeed, comparing pharmacodynamic effects of different S1P modulators, such as mocravimod, FTY720 and BAF312 established in healthy volunteers reveals differences in efficacy of lymphocyte sequestration. A measurable parameter that determines maintenance of the mode of action, i.e., sequestering of lymphocytes in secondary lymphoid organs and bone marrow, is reduction of peripheral lymphocyte counts. Recovery of absolute lymphocyte counts to 80% of normal counts after a single 1 mg dose of FTY720, multiple dose applications of BAF312 for 28 days, or a single 3 mg dose of KRP203 was reached after 8, 7 and more than 10 days, respectively. Thus, the lymphocyte recovery time for KRP203 is significantly longer than for BAF312 and FTY720.

### Particle size of the S1P receptor modulator

In the pharmaceutical industry, particle characterization of powder materials has become one of the crucial aspects in drug product development and quality control of solid oral dosage forms. The particle size distribution of the drug substance may have significant effects on final drug product performance (e.g., dissolution, bioavailability, content uniformity, stability, etc.). Furthermore, the particle size distributions of the drug substance can affect drug product manufacturability such as flowability, blend uniformity, compactibility, and have profound influence on almost every step of manufacturing processes for solid oral dosage forms, including pre-mixing/mixing, granulation, drying, milling, blending, coating, encapsulation, and compression. The particle size of the drug substance can therefore ultimately impact safety, efficacy, and quality of the drug product.

In an embodiment the S1P receptor modulator of the present disclosure has an average particle size (D50) of less than or equal to 8µm, preferably 6µm, more preferably 5µm. In another embodiment, the S1P receptor modulator of the present disclosure has a D90 of less than or equal to 25µm, preferably 22µm, more preferably 19µm. Indeed, it has been found by the inventors that an average particle size (D50) of greater than 8 µm, preferably greater than 6µm, more preferably greater than 5µm and/or a D90 greater than 25 µm, preferably greater than 22µm, more preferably greater than 19µm, impairs the dissolution of the drug by diminishing the surface contact with the solvent resulting in lowering bioavailability and in vivo performance.

### Pharmaceutical composition comprising the S1P receptor modulator

The present disclosure also relates to a pharmaceutical composition of said S1P receptor modulator, preferably mocravimod, as described above, in particular for their use in the treatment methods as disclosed.

In an embodiment, the pharmaceutical composition of the present disclosure comprises the S1P receptor modulator, preferably mocravimod, and one or more pharmaceutically acceptable excipients.

Any suitable excipients known to those of ordinary skill in the art for use in pharmaceutical compositions may be employed in the compositions described herein.

The pharmaceutical composition may be administered in any manner appropriate to the disease or disorder to be treated as determined by persons of ordinary skill in the medical arts. An appropriate dose and a suitable duration and frequency of administration will be determined by such factors as discussed herein, including the condition of the patient, the type and severity of the patient's disease, the particular form of the active ingredient, and the method of administration. In general, an appropriate dose (or effective dose) and treatment regimen provides the pharmaceutical composition in an amount sufficient to provide a therapeutic effect, for example, an improved clinical outcome, such as more frequent complete or partial remissions, or longer disease-free and/or overall survival, or a lessening of symptom severity or other benefit as described in detail herein.

The pharmaceutical compositions described herein may be administered to a subject in need thereof by any of several routes that can effectively deliver an effective amount of the compound. The pharmaceutical composition may be administered orally, rectally, parenterally, intracisternally, intravaginally, intraperitoneally, topically, bucally, or as an oral or nasal spray. In a preferred embodiment, the pharmaceutical composition is suitable to be administered orally.

In another embodiment, the pharmaceutical composition may be a solid dosage form suitable for oral administration. Solid dosage forms for oral administration include capsules, tablets, pills, powders, and granules. In a preferred embodiment, the pharmaceutical composition is a capsule or a tablet. The capsule may be a soft or hard gelatin capsule, preferably a hard gelatin capsule. For example, the capsule is HGC Crushed or HPMC capsules Crushed.

In an embodiment, the release of the capsule or tablet content may be immediate or modified such as delayed, targeted or extended. In a preferred embodiment the solid dosage form is an immediate release dosage form.

In an embodiment, the pharmaceutical composition comprises the S1P receptor modulator, preferably mocravimod, and one or more pharmaceutically acceptable excipients, and particularly, at least one filler and mixtures thereof, a disintegrant, a lubricant and, a glidant.

### Fillers

Fillers, (also referred to as a diluents, dilutants or thinners) are substance which are added to the drug substance in order to make the latter suitable for oral administration (e.g., capsules, tablets). Fillers themselves should not produce any pharmacological effect on human being. Examples of fillers include mannitol, microcrystalline cellulose, lactose monohydrate, anhydrous lactose, corn starch, xylitol, sorbitol, sucrose, dicalcium phosphate, maltodextrin, and gelatin. The pharmaceutical composition of the present disclosure comprises at least one filler selected from mannitol, microcrystalline cellulose and mixtures thereof. In a preferred embodiment, the pharmaceutical composition of the present disclosure comprises a mixture of mannitol and microcrystalline cellulose.

### Disintegrants

Disintegrants are added to oral solid dosage forms to aid in their deaggregation. Disintegrants are formulated to cause a rapid break-up of solids dosage forms when they come into contact with moisture. Disintegration is typically viewed as the first step in the dissolution process. Examples of disintegrants include the modified starch such as sodium starch glycolate, sodium carboxymethyl starch, and pre-gelatinized starch, crosslinked polymers, such as crosslinked polyvinylpyrrolidone (crospovidone) or crosslinked sodium carboxymethyl cellulose (croscarmellose sodium), and calcium silicate. The pharmaceutical composition of the present disclosure comprises sodium starch glycolate as disintegrant.

### Lubricants

Lubricants are substances that we use in tablet and capsule formulations in order to reduce the friction. Lubricant can facilitate extrusion of tablets from matrix, thus preventing formation of scratches on their surfaces. By nature lubricants can be divided into two groups: a) fats and fat-like substances; b) powdery substance. Powdery substances are more applicable then the fat-like ones, because the latter impact on solubility and chemical stability of the tablets. Powdery lubricants are introduced by powdering of granulate. They provide constant-rate outflow of mass for tabletizing from hopper into matrix that guaranties accuracy and constancy of the drug substance dosage.

Examples of lubricants include magnesium stearate, hydrogenated castor oil, glyceryl behenate, calcium stearate, zinc stearate, mineral oil, silicone fluid, sodium lauryl sulfate, L-leucine, and sodium stearyl fumarate. The pharmaceutical composition of the present disclosure comprises magnesium stearate as lubricant.

### Glidants

Glidants are blended with the formulation to enhance the tablet-core blend-material flow property. During the early stage of compression, glidants are mixed within the particle arrangement of the tablet powder blend to improve flowability and uniformity within the die cavity of tablet presses. Glidants encourage the flow of tablet granulation by diminishing friction between particles. The effect of glidants on the flow of the granules depends on the size and shape of the particles of the granules and the glidants. Above a certain concentration, the glidant will in fact function to inhibit flowability. In tablet manufacture, glidants are usually added just prior to compression. Examples of glidants include colloidal silicon dioxide, starch, magnesium stearate and talc. The pharmaceutical composition of the present disclosure comprises colloidal silicon dioxide as lubricant.

The pharmaceutical composition of the present disclosure comprises the S1P receptor modulator, and at least one filler selected from mannitol, microcrystalline cellulose and mixtures thereof, sodium starch glycolate as disintegrant, magnesium stearate as lubricant and, colloidal silicon dioxide as glidant.

Any suitable excipients known to those of ordinary skill in the art in pharmaceutical compositions may be further employed in the compositions described herein.

In an embodiment the dosage strength of the S1P receptor modulator, preferably the hydrochloride salt of formula (I), in the solid dosage form is between 0.05 mg to 15 mg/unit, preferably between 0.1mg to 10mg/unit, for example about 0.1mg/unit, or about 0.4mg/unit, or about 1 mg/unit, or about 10 mg/unit, more preferably about 1 mg/unit.

More specifically, the pharmaceutical composition of the present disclosure further comprises the following ingredients:
- mannitol, preferably at a content from 48 to 88 mg/unit, more preferably from 58 to 78mg/unit, even more preferably at a content about 68 mg/unit;
- microcrystalline cellulose, preferably at a content from 5 to 45 mg/unit, more preferably from 15 to 35 mg/unit, even more preferably at a content about 25 mg/unit;
- sodium starch glycolate, preferably at a content from 1 to 8 mg/unit, more preferably from 2 to 6 mg/unit, even more preferably at a content about 4 mg/unit;
- magnesium stearate, preferably at a content from 0.025 to 4 mg/unit, more preferably from 0.5 to 2 mg/unit, even more preferably at a content about 1 mg/unit; and
- colloidal silicon dioxide, preferably at a content from 0.125 to 2 mg/unit, more preferably from 0.25 to 1 mg/unit, even more preferably at a content about 0.5 mg/unit.

In another embodiment, the pharmaceutical composition of the present disclosure comprises the following ingredients:
- the S1P receptor modulator, preferably mocravimod, preferably at a content from 0.05% to 15%, more preferably from 0.1% to 10% mg/unit, even more preferably at a content about 0.1% or 0.4% or 1% or 10%;
- mannitol, preferably at a content from 48% to 88%, more preferably from 58% to 78%, even more preferably at a content about 68%;
- microcrystalline cellulose, preferably at a content from 5% to 45%, more preferably from 15% to 35%, even more preferably at a content about 25%;
- sodium starch glycolate, preferably at a content from 1% to 8%, more preferably from 2% to 6%, even more preferably at a content about 4%;
- magnesium stearate, preferably at a content from 0.025% to 4%, more preferably from 0.5% to 2%, even more preferably at a content about 1%;
- colloidal silicon dioxide, preferably at a content from 0.125 to 2 mg/unit, more preferably from 0.25 to 1 mg/unit, even more preferably at a content about 0.5 mg/unit,

Percentage being expressed mg/mg of the total composition in dry weight.

A preferred stable formulation of the present disclosure is as follows (deriving from Blend 17 of the examples 4 and 5 and detailed in example 6):

| Excipient | Amount %w/w | Amount (kq) |
|---|---|---|
| KRP203 hydrochloride | 1.08 | 0.119 |
| mannitol | 68.23 | 7.505 |
| microcrystalline cellulose | 25.19 | 2171 |
| sodium starch glycolate | 4.00 | 0.440 |
| magnesium stearate | 1.00 | 0.110 |
| colloidal silicon dioxide | 0.50 | 0.055 |

Evaluation of drug stability can prevent toxicity and increase safety, efficacy and quality of the final drug product. This is especially important for treatment of patients in countries with variable temperatures (humid high temperatures), because patients are taking the composition capsule at home once they are discharged from hospital, which happens a few weeks after the HSCT therapy, once the patient's condition is stabilized.

In an embodiment, the pharmaceutical composition of the disclosure is stable for at least 1 month at 50°C, preferably 2 months at 50°C.

In another embodiment, the pharmaceutical composition of the disclosure is stable at least 24 months at 5°C.

In another embodiment, the pharmaceutical composition of the disclosure is stable at least 24 months at 25°C/ 60% relative humidity.

As used herein, stability of a composition is measured according to the following method: high-pressure liquid chromatography (HPLC) which is widely known in the field. A composition is stable if the sum of impurities is less than or equal to 0.7% with a confidence interval of [98-102]%.

### Process of preparing the pharmaceutical composition

Another aspect of the present disclosure relates to the process of preparing the above-mentioned pharmaceutical composition, wherein it comprises the step of:
a. Blending the S1P receptor modulator with microcrystalline cellulose, colloidal silicon dioxide, preferably at 22rpm for 18 mins,
b. adding mannitol and blending the resulting mixture, preferably at 22rpm for 9 mins,
c. adding sodium starch glycolate and blending the resulting mixture, preferably at 22rpm for 5 mins,
d. adding magnesium stearate, blending the resulting pharmaceutical composition, preferably at 22rpm for 5 mins,
e. recovering the pharmaceutical composition of the present disclosure.

In a preferred embodiment, the process further comprises the step of:
f. filling the resulting pharmaceutical composition into capsules,
g. recovering the resulting capsules filled with the pharmaceutical composition.

### The patient population to be preferably targeted by the treatment methods

The treatment methods disclosed herein are suitable for patients having acute myeloid leukemia (AML) and that, as per standard medical practice, requires myeloablative conditioning followed by allogeneic hematopoietic stem cell transplant.

The present method of treatments disclosed herein improves the GVL effect while reducing acute and chronic GVHD, and, optionally side effects of immunosuppressants, in particular calcineurin inhibitors, such as ciclosporin A, in particular for patients with high risk of relapse as defined below, and more specifically in combination with a chronic administration of S1P receptor modulator and/or tapering of immunosuppressant treatment as described in the next sections.

Accordingly, the methods of the present disclosure are particularly suitable for subject with refractory or relapsed AML after one or more AML therapy.

As used herein, the term "refractory" means a subject which failed to achieve complete remission (e.g., wherein less than 5% of the cells in the bone marrow are blasts, and there is an absence of blasts with Auer rods in the bone marrow, an absence of extramedullary disease, and full hematologic recovery (e.g, absolute neutrophil count (ANC) >1,000/pL and platelet count >100,000/pL), and/or CRi following treatment for a disease, or achieved a CR e.g, wherein less than 5% of the cells in the bone marrow are blasts, and there is an absence of blasts with Auer rods in the bone marrow, an absence of extramedullary disease, and full hematologic recovery (e.g, absolute neutrophil count (ANC) >1,000/pL and platelet count >100,000/pL), and/or CRi lasting less than 90 days following treatment for the disease.

As used herein, the term "relapse" or "relapsed" has its ordinary meaning in the art, and may refer to the return of AML or the signs and symptoms of AML after a period of complete remission (e.g, initial complete remission) due to treatment.

In some embodiments, relapse may refer to the recurrence of disease after complete remission meeting one or more of the following criteria (i) > 5% blasts in the marrow or peripheral blood, and/or (ii) extramedullary disease, and/or disease presence determined by a physician upon clinical assessment. In some embodiments, "relapse" refers to reoccurrence of a disease following a CR e.g, wherein less than 5% of the cells in the bone marrow are blasts, and there is an absence of blasts with Auer rods in the bone marrow, an absence of extramedullary disease, and full hematologic recovery (e.g, absolute neutrophil count (ANC) >1,000/pL and platelet count >100,000/pL), and/or CRi) lasting 90 days or longer.

As used herein, the term "remission" has its ordinary meaning in the art, and may refer to a decrease in or disappearance of signs and symptoms of cancer. In partial remission, some, but not all, signs and symptoms of cancer have disappeared. In complete remission (CR), all signs and symptoms of cancer have disappeared, although cancer still may be in the body.

"Complete remission" as used herein, is measured by complete morphologic response (CMR). Complete morphological response is defined as leukaemia clearance (< 5% marrow blasts and no circulating peripheral blasts) in conjunction with normal values for absolute neutrophil count and platelet count, no extramedullary manifestation of leukaemia and no need for repeat blood transfusions, and includes CR, CRi and CRh, as defined below.

In some embodiments, complete remission of AML means the disease has been treated, and the following are true: (i) the complete blood count is normal; (ii) less than 5% of the cells in the bone marrow are blasts (leukemia cells); and (iii) there are no signs or symptoms of leukemia in the brain and spinal cord or elsewhere in the body. In some embodiments, complete remission of AML means less than 5% of the cells in the bone marrow are blasts, and there is an absence of blasts with Auer rods in the bone marrow, an absence of extramedullary disease, and full hematologic recovery ( e.g ., absolute neutrophil count (ANC) >1,000/pL and platelet count >100,000/pL).

CR as used herein, is defined as leukaemia clearance (less than 5% marrow blasts and no circulating peripheral blasts) in conjunction with normal values for absolute neutrophil count and platelet count, no extramedullary manifestation of leukaemia and no need for repeat blood transfusions.

CRi as used herein, is defined as meeting all CMR criteria except for an absolute neutrophil count < 1,000/pL or platelet count < 100,000/µL.

CRh as used herein, means less than 5% of the cells in the bone marrow are blasts, and there is an absence of blasts with Auer rods in the bone marrow, an absence of extramedullary disease, and partial hematologic recovery of both peripheral blood cell types (e.g, ANC >500/pL and platelet count >50,000/pL).

"Partial remission" as used herein, means greater than or equal to 5% to less than or equal to 25% of the cells in the bone marrow are blasts, and a decrease of at least 50% in the percentage of blasts. In some embodiments, partial remission of AML means (i) greater than or equal to 5% to less than or equal to 25% of the cells in the bone marrow are blasts; (ii) a decrease of at least 50% in the percentage of blasts; and (iii) the complete blood count is normal.

In certain embodiments, the patient population is selected among the patients suffering from acute myeloid leukemia and classified as CR1 or CR2 according to CIBMTR classification.

In specific embodiments, said subject is selected among the patients suffering of acute myelogenous leukemia and being either in first complete morphological remission but in the adverse-risk group called "CR1 high risk", or in second and subsequent complete morphological remission called "CR2".

First complete remission or CR1 is defined according to "ASBMT RFI 2017 - Disease Classifications Corresponding to CIBMTR Classifications of the American Society for Blood and Marrow Transplantation" as patients having a treatment response, i.e. complete remission following the first treatment.

CR1 patients are classified in a 3-group classification (favorable, intermediate, adverse). CR1 high risk patients corresponds to the adverse group as defined according to the 2017 European Leukaemia Network (ELN) genetic risk stratification (Döhner H, et al. Diagnosis and management of AML in adults: 2017 ELN recommendations from an international expert panel. Blood. 2017 Jan 26;129(4):424-447. doi: 10.1182/blood-2016-08-733196. Epub 2016 Nov 28. PMID: 27895058; PMCID: PMC5291965*)* and which corresponds to the genetic abnormality selected from the group consisting of t(6;9)(p23;q34.1), DEK-NUP214t(v;11q23.3), KMT2A rearranged t(9;22)(q34.1;q11.2), BCR-ABL1 inv(3)(q21.3q26.2) or t(3;3)(q21.3;q26.2), GATA2,MECOM(EVI1 -5 or del(5q); -7; -17/abn(17p), complex karyotype, monosomal karyotypell, wild-type NPM1 and FLT3-ITD^{high}, mutated RUNX1, mutated ASXL1, and Mutated TP53.

Second complete remission or CR2 is defined according to "ASBMT RFI 2017 - Disease Classifications Corresponding to CIBMTR Classifications of the American Society for Blood and Marrow Transplantation" (https://higherlogicdownload.s3.amazonaws.com/ASBMT/43a1f41f-55cb-4c97-9e78-c03e867db505/Uploadedlmages/ASBMT_RFI 2018B_CIBMTR_Disease Classificati ons.pdf) as patients in complete remission (CR) for the second time and subsequent time. CR2 patients achieved CR as defined above, relapsed and achieved at one complete remission again. Final pre-HSCT status must be complete remission.

As used herein, CR2 includes CR2+ as defined according to "ASBMT RFI 2017 - Disease Classifications Corresponding to CIBMTR Classifications of the American Society for Blood and Marrow Transplantation", i.e. patients in complete remission (CR) for more than the second time. CR2+ patients achieved CR twice as defined above, relapsed and achieved complete remission again.

Stratification between CR1 and CR2 is needed for interpretation of the S1P receptor modulator trial outcome, since HSCT basic performance may vary depending on CR status.

In an embodiment, CR1 high risk, CR2 patients are male or female subjects aged 18-70 years with acute myeloid leukaemia.

All CR1 high risk and CR2 patients are in need for haematopoietic stem cell transplantation (HSCT) and have a very high risk for relapse.

In specific embodiments, the subject in need of the methods of treatment or use of the present disclosure is measurable residual disease (MRD)-positive prior to being administered the methods of treatment with HSCT and S1P receptor modulator described herein (preferably , HSCT with mocravimod).

In hematological cancers, such as AML, measurable residual disease and minimal residual disease refer to the post-therapy persistence of leukemic cells at levels below morphologic detection. Although not wishing to be bound by any particular theory, MRD is thought to be a strong prognostic indicator of increased risk of relapse or shorter survival in patients with hematologic cancers, such as AML. MRD testing for AML is preferably conducted using one of three techniques: immunophenotypic detection by multiparameter flow cytometry (MFC), real-time quantitative PCR (RT-qPCR) and next-generation sequencing technology. MFC uses panels of fluorochrome-labeled monoclonal antibodies to identify aberrantly expressed antigens of leukemic cells. RT-qPCR can be used to amplify leukemia-associated genetic abnormalities. Next-generation sequencing technology can be used to evaluate a few genes or an entire genome. Together, RT-qPCR and next-generation sequencing technology represent molecular approaches to MRD testing. Each of the foregoing methods of detecting MRD status in a subject is described in Ravandi, F., et al, Blood Advances 12 June 2018, vol. 2, no. 11, and Schuurhuis, G. J., et al. , Blood 2018 March 22, 131(12): 1275-1291, the relevant contents of which are incorporated herein by reference in their entireties.

To guide the development of a standardized approach to MRD testing, the European LeukemiaNet (ELN) has issued consensus recommendations for the measurement of MRD in AML. According to the ELN, a percentage of cancer (e.g., AML) cells to leukocytes of 0.1% or greater in a subject's bone marrow, measured by MFC according to the ELN's recommendations for MRD testing by MFC, indicates the subject is MRD positive (MRD+) by MFC according to the ELN's recommendations for MRD testing by MFC. A percentage of cancer cells to leukocytes of less than 0.1% in a subject's bone marrow, measured by MFC according to the ELN's recommendations for MRD testing by MFC, indicates the subject is MRD negative (MRD-) by MFC according to the ELN's
The ELN has also issued guidelines for molecular MRD testing in AML. The ELN defines complete molecular remission as complete morphologic remission plus two successive negative MRD samples obtained within an interval of >4 weeks at a sensitivity level of at least 1 in 1,000, wherein the samples are collected and measured according to the ELN guidelines for molecular MRD testing. The ELN defines molecular persistence at low copy numbers, which is associated with a low risk of relapse, as MRD with low copy numbers (<100-200 copies/104 ABL copies corresponding to <1-2% of target to reference gene or allele burden) in patients with morphologic CR, and a copy number or relative increase <1 log between any two positive samples collected at the end of treatment, wherein the samples are collected and measured according to the ELN guidelines for molecular MRD testing. The ELN defines molecular progression in patients with molecular persistence as an increase of MRD copy numbers >1 log 10 between any two positive samples collected and measured according to the ELN guidelines for molecular MRD testing. The ELN defines molecular relapse as an increase of the MRD level of >1 log 10 between two positive samples in a patient who previously tested negative, wherein the samples are collected and measured according to the ELN guidelines for molecular MRD testing. Both molecular persistence and molecular relapse are indicators of an MRD-positive subject by RT-qPCR conducted according to the ELN guidelines for MRD testing by RT-qPCR. Thus, patients in complete molecular remission and patients labelled as having molecular persistence at low copy numbers are MRD-negative by RT-qPCR conducted according to the ELN guidelines for MRD testing by RT-qPCR. RT-qPCR is the recommended molecular approach to MRD testing, as discussed in Ravandi, F., el al. and Schuurhuis, G. J., el al. Specific recommendations for collecting and measuring samples (e.g. bone marrow samples) for MRD testing are described in Ravandi, F , et al, Blood Advances 12 June 2018, vol. 2, no. 11 and Schuurhuis, G. J., et al, Blood 2018 March 22, 131(12): 1275-1291, the relevant contents of which are incorporated herein by reference in their entireties.

When a subject having a hematologic cancer, such as AML, is described herein as being "measurable residual disease negative", "minimal residual disease negative", "MRD-negative" or "MRD" without a further modifier, such as by MFC or by RT-qPCR, the subject is MRD negative according to at least one of the ELN's criteria described herein (e.g. MFC, molecular biology). In some embodiments, the subject is MRD-negative by MFC conducted according to ELN guidelines for MRD testing. In some embodiments, the subject is MRD-negative by RT-qPCR conducted according to ELN guidelines for MRD testing. In some embodiments, the subject is MRD-negative by both MFC and RT-qPCR conducted according to ELN guidelines for MRD testing. In some embodiments, the subject is MRD-negative by MFC conducted according to ELN guidelines for MRD testing, and is MRD-positive by RT-qPCR conducted according to ELN guidelines for MRD testing.

In some embodiments, the subject is MRD-positive by MFC conducted according to ELN guidelines for MRD testing, and is MRD-negative by RT-qPCR conducted according to ELN guidelines for MRD testing. When a subject is MRD-negative according to one of the ELN's criterion described herein (e.g. the criterion for MFC), but MRD-positive according to another of the ELN's criterion described herein (e.g. the criterion for RT-qPCR), that subject can still be described as MRD-negative according to the use of that term herein because the subject is MRD negative according to at least one of the ELN's criteria described herein.

When a subject having a hematological cancer, such as AML, is described herein as being "measurable residual disease positive", "minimal residual disease positive", "MRD-positive" or "MRD+", the subject is MRD positive by the ELN's criteria for MFC and RT-qPCR described herein. For example, a subject that is MRD positive for AML can be MRD-positive by MFC conducted according to ELN guidelines for MRD testing in AML, and MRD-positive by RT-qPCR conducted according to ELN guidelines for MRD testing in AML.

Thus, in some embodiments of the methods described herein, the method further comprises detecting the MRD status of a subject (e.g. prior to administering the HSCT methods of treatment with S1P receptor modulator as described herein).

### The methods of treatment

The S1P receptor modulator as disclosed in the previous section, and more preferably mocravimod, and their pharmaceutical compositions, are useful as a drug in methods for treating AML in subjects undergoing HSCT, and more particularly in the subpopulation of patients as defined above.

The S1P receptor modulator as disclosed in the previous section, and more preferably mocravimod, and their pharmaceutical compositions, are also useful as a drug in methods for preventing chronic GVHD in subjects undergoing HSCT, in particular in subjects undergoing HSCT for treating AML, and more particularly in the subpopulation of patients as defined above.

Detailed embodiments of such methods are disclosed hereafter.

The method of the present disclosure comprises at least the following steps:
1) administering to the subject an effective amount of an S1P receptor modulator, for example, a compound of formula (I) or (II), or a pharmaceutically acceptable salt thereof;
2) conditioning said subject for destroying substantially the bone marrow and immune system wherein said conditioning includes treatment of said subject with an effective amount of a chemotherapeutic agent such as cyclophosphamide and/or treating said subject with a high-dose chemoradiation therapy;
3) transplanting allogeneic hematopoietic stem cells from a donor to said subject; and,
4) optionally, co-administering an efficient amount of one or more immunosuppressants to prevent acute GVHD, in particular during the first 3 months following the start of S1P receptor modulator treatment.

Detailed embodiments of each step are described hereafter.

### Step (1) of administering the S1P receptor modulator

To perform the methods of treatment as disclosed herein, an effective amount of S1P receptor modulator (preferably mocravimod or other S1P receptor modulator as disclosed above) is administered to the subject in need of such treatment.

"An effective amount" as used herein, refers to the amount of S1P receptor modulator (such as mocravimod) required to confer therapeutic effect on the subject, either alone or in combination with one or more other active agents. Effective amounts vary, as recognized by those skilled in the art, depending on, for example, route of administration, excipient usage, and co-usage with other active agents. In the case of treating a particular disease or condition, the desired therapeutic effect is inhibiting the progression of the disease. This may involve only slowing the progression of the disease temporarily, although more preferably, it involves halting the progression of the disease permanently. This can be monitored by routine methods or can be monitored according to diagnostic methods discussed herein. The desired response to treatment of the disease or condition also can be delaying the onset or even preventing the onset of the disease or condition. In specific embodiments, the desired response can be delaying or preventing the onset of acute GVHD, chronic GVHD, relapse AML or death. In specific embodiments, the desired response can be delaying or preventing the onset of AML relapse while delaying or preventing the onset of GVHD (grade III/IV) refractory to systemic immunosuppressive treatment. In other embodiments, the desired response can be increasing survival at 3, 6, 12 and 24 months after HSCT. In other embodiments, the desired response can be improving the quality of life, in particular at 3, 6, 12 and 24 months after HSCT, as compared to same HSCT treatment without administration of S1P receptor modulator.

The administration of the S1P receptor modulator, preferably mocravimod, is started prior to allogenic hematopoietic stem cells transplantation (HSCT). Preferably, administration is started at least 7 days prior to HSCT, for example 14, 13, 12, 11, 10, 9, 8, or 7 days, preferably 11 days prior to HSCT.

The S1P receptor modulator, preferably mocravimod, is then daily administered for a period set to at least 80 days, or at least 100 days or more, after the HSCT.

In a preferred embodiment, the S1P receptor modulator, preferably mocravimod, is daily administered for a longer period (as chronic use), for example for at least 6, 7, 8, 9, 10, 11, 12, 18, 24 months, or more, typically during the life of the subject, or until relapse, after the HSCT. In specific embodiments, the S1P receptor modulator, preferably mocravimod, is daily administered for a period between 6 and 24 months, for example for 12 months after the HSCT.

The daily dosage amount and dose of a S1P receptor modulator, preferably mocravimod, described herein may depend upon the subject's condition, that is, stage of the disease, severity of symptoms caused by the disease, general health status, as well as age, gender, and weight, and other factors apparent to a person of ordinary skill in the medical art. Similarly, the dose of the S1P modulator, preferably mocravimod, for treating a human subject suffering from AML and undergoing allogeneic HSC transplant may be determined according to parameters understood by a person of ordinary skill in the medical art.

In some embodiments, the amount of S1P receptor modulator, preferably mocravimod, administered per day is a fixed amount. In some embodiments, the fixed daily dosage is 0,05 mg to 40 mg per day, preferably 0,1 mg to 35 mg, more preferably 0,5 mg to 30 mg, even more preferably 1 mg to 15 mg per day, even more preferably 1,5 mg to 7mg, even more preferably 2 mg to 5 mg, even more preferably about 3 mg per day or about 1 mg per day.

In some embodiments, the S1P receptor modulator is mocravimod, and said mocravimod is daily administered for at least 6, 7, 8, 9, 10, 11, 12, 18, 24 months, or more, after the HSCT at a dosage of 3 mg per day.

The daily dosage may be administered as one dose per day or in multiple doses in a single day. In a preferred embodiment, the daily dosage is administered once a day. In some embodiments, the doses are administered several times daily, preferably 3 times daily. The minimum dose that is enough to provide effective therapy may be used in some embodiments.

In another embodiment, said S1P receptor modulator is mocravimod, and said mocravimod is administered at a daily dose of 3mg, e.g. three solid dosage form a day of 1mg. Indeed, 3 solid dosage form, such as capsules or tablets, of 1mg administered in a spaced-apart manner is easier to swallow than a single solid dosage form of 3 mg.

### Step (2) of myeloablative conditioning

Before allogenic hematopoietic stem cell transplantation, patients have to be conditioned by preferably using high dose of chemotherapy and/or performing total body irradiation (TBI) according to national guidelines adapted to institutional practices, also referred as chemoradiation therapy. Preferably, the standard of care is myelo-ablative regimen. However, non myelo-ablative regimen or reduced intensity conditioning may also be used for some subset population of patients (Jethava et al., Bone Marrow Transplant. 2017 Nov;52(11):1504-1511).

High-dose chemotherapy, high-dose total body irradiation (TBI), or a combination thereof is carried out.

As used herein, "chemotherapeutic agent" and "chemotherapy" refer to agents and therapies, respectively, that inhibit (e.g., arrest) the growth of cancer cells as, for example, by killing the cells or inhibiting cell division. "Chemotherapeutic agent for AML" and "chemotherapy for AML" refer to chemotherapeutic agents and chemotherapies, respectively, administered to a subject with the purpose of treating AML in the subject.

As examples of chemotherapeutic agents, mention may be made of cyclophosphamide (CY), cytarabine (CA), etoposide (ETP), busulfan (BU), fludarabine (FLU), melphalan (MEL), methotrexate (MTX), ciclosporin A (CsA), and the like, and mixtures thereof such as fludarabine/busulfan, busulfan/cyclophosphamide and fludarabine/melphalan.

For example, one of the following conditioning regimens may be administered (day numbers are relative to the day of HSCT).

As examples of conditioning regimens, mention may be made of, for example:
- Fludarabine; 30 mg/m2 IV once daily for 5 days on Day -8 to -4 (150 mg/m2)
- Thiotepa; 5 mg/kg IV twice daily for 1 day on Day -7 (10 mg/kg)
- Melphalan; 60 mg/m2 IV once daily for 2 days on Day -2 and -1 (120 mg/m2)

Dose variations in the conditioning regimen may occur to accommodate for patient's condition and/or local practice.

According to institutional practices, the 3 following conditioning regimens may be selected next to the standard regimen described hereabove:
- Melphalan can be substituted by busulfan.
- Thiotepa can be substituted by cyclophosphamide

1. Busulfan + fludarabine + cyclophosphamide:
   - Busulfan 110 mg/m2 IV on Day -7 to -4 (440 mg/m2)
   - Fludarabine 25 mg/m2 for 5 days from Day -6 to -2 (125 mg/m2)
   - Cyclophosphamide 14.5 mg/kg IV on Day -3 and -2 (29 mg/kg)
2. Busulfan + fludarabine + thiotepa:
   - Busulfan 3.2 mg/kg/day IV for 3 days on Day -5 to -3 (9.6 mg/kg)
   - Fludarabine 50 mg/m2/day IV for 3 days on Day -5 to -3 (150 mg/m2)
   - Thiotepa 5 mg/kg/day IV for 2 days on Day -7 and -6 (10 mg/kg)
3. Melphalan + thiotepa + fludarabine:
   - Melphalan 100 or 140 mg/m2 IV on Day -6
   - Fludarabine 40 mg/m2 IV for 4 days on Days -5 to -2 (160 mg/m2)
   - Thiotepa 5-10 mg/kg IV on Day -7
   (day numbers are relative to the day of HSCT)

For example, a treatment consisting of administration of cyclophosphamide followed by total body irradiation, a treatment consisting of administration of busulfan and cyclophosphamide and the like may be mentioned.

### Step (3) of HSC transplant

Conditioning is followed by allogeneic HSCT whose goal is two-fold: firstly, to replace the patient's diseased hematopoietic system with new HSC stemming from a genetically disparate healthy donor, and secondly, to exploit the immunotherapeutic effect of the donor graft, i.e. graft-versus-leukaemia effect called "GVL".

For example, the allogenic HSCT may comprise transplanting HSCs from a donor to said patient. HSCs are collected from the donor and administered to the patient by intravenous infusion.

HSCs can be harvested from different sources. HSC mobilized by granulocyte colony-stimulating factor (G-CSF) into the peripheral blood are the preferred source for transplantation today. Alternative sources for HSCs used clinically are bone marrow and umbilical cord blood. A suitable donor should be HLA-matched related or unrelated donor with 8/8 or higher matches at the HLA-A, -B, -C, -DRB1, and/or -DQB1 loci, as determined by high resolution HLA typing.

Allogeneic hematopoietic stem cell transplant may be performed as the standard of care. Suitable methods are described for example in Boglarka Gyurkocza, Andrew Rezvani & Rainer F Storb (2010) Allogeneic hematopoietic cell transplantation: the state of the art, Expert Review of Hematology, 3:3, 285-299, DOI: 10. 1586/ehm. 10.21*.*

Preferably, stem cell source is mobilized peripheral blood collected via apheresis by a compatible donor. The minimum recommended CD34+ cell dose in the graft may be 2 × 10⁶ / kg, with a recommended target dose of about 5 × 10⁶ / kg.

### Step (4) of immunosuppressant treatment

The S1P receptor modulator, preferably mocravimod, may also be used alone and/or in appropriate association, as well as in combination, with other pharmaceutically active compounds.

In order to reduce the risks of acute GVHD, the S1P receptor modulator, preferably mocravimod, is co-administered in conjunction with an immunosuppressive agent, in particular during at least the first three months following HSCT.

Examples of immunosuppressive agents include without limitation ciclosporin A, sirolimus, tacrolimus, methotrexate, and mycophenolate.

In an embodiment, the immunosuppressant agent comprises or essentially consists of an efficient amount of ciclosporin A.

Ciclosporin A or related immunosuppressant administration may be started, for example, within a period between the starting day of administration of the S1P receptor modulator, preferably mocravimod, and the day of HSC transplant, preferably 3 or 2 days prior to HSC transplant or 1 day prior to HSC transplant.

For example, intravenous administration of ciclosporin A may be carried out at an initial dosage of between 2 to 6 mg/kg/day, preferably 3 to 5 mg/kg/day. Dosage adjustments are carried out, based on the toxicity or the concentration of ciclosporin A relative to the target trough concentration (150 to 400 mg / L). The administration of ciclosporin A can be changed to oral administration if the patient can tolerate the oral administration. The initial dosage for oral administration may be set to the current dosage for intravenous administration. The dosage of ciclosporin A is monitored at least weekly and changed to a clinically appropriate dosage.

In an embodiment, the dosage level between 2 to 6 mg/kg/day, preferably 3 to 5 mg/kg/day may be continued during the day of HSC transplant for a period of up to 2 weeks before a change is made to oral maintenance therapy. Preferably, when the change to oral maintenance therapy is made, the daily doses of cyclosporin A may be about 12.5 mg/kg. Preferably the maintenance therapy is continued for at least 3 months, preferably for 6 months before decreasing the dose. Particularly, the dose of cyclosporin A may be gradually decreased to zero by 1 year after transplantation.

The use of S1Pr drug facilitates the acceleration of Calcineurin inhibitors' dose reduction, such as ciclosporin A (CsA) and tacrolimus (TAC), which is a major advantage for patients, minimizing toxicities of the used Calcineurin inhibitor, and which may be advantageous for fostering the reconstituted immune system (HSCT) to better fight against cancer.

In the case where methotrexate is used together therewith, the dosing schedule and the dosage of methotrexate will be adapted to the hospital standards. For example, on the day of administration of HSCT (e.g. 11 days after first administration of S1PR modulator, preferably mocravimod), 10 mg/kg of methotrexate is administered, and 2 and 5 days after first administration and the 16th day therefrom, 6 mg/kg of methotrexate is administered, respectively. The use of S1Pr drug also facilitates the acceleration of methotrexate dose reduction, which is major advantage for patients, minimizing toxicities of methotrexate, which may be advantageous for fostering the reconstituted immune system (HSCT) to better fight against cancer.

The present methods advantageously enable to prevent acute and chronic GVHD and to preserve the graft versus leukemia effect while lowering immunosuppressor amount usually administered after an allogenic HSC transplantation, which thus lower adverse effects of such immunosuppressant treatments.

In another embodiment, the immunosuppressants used in the method do not include tacrolimus. In another embodiment, the immunosuppressants used in the method do not include ciclosporin A.

Calcineurin inhibitors such as ciclosporin A (CsA) and tacrolimus (TAC) suppress donor T-cell activation and remain the most commonly used immunosuppressants for acute GVHD prophylaxis. These agents, however, also inhibit leukemia-specific T-cell responses leading to impaired GVL effect.

With the present methods, the chronic administration of an S1P receptor modulator, preferably mocravimod, provides a significant prevention of chronic GVHD and enables to lower (taper) the amount of ciclosporin A or other immunosuppressive drugs to be administered to the subject in need thereof, thereby lowering the adverse effects of such immunosuppressive compound, while still preventing chronic GVHD and improving the GVL effect.

In a preferred embodiment where S1P receptor, preferably mocravimod, is administered for a long period (chronic use), for example at least 6 months, preferably 12, 18 or 24 months, typically during the life of the subject or until relapse, said subject is further treated with an efficient amount of immunosuppressants including at least ciclosporin A, and said immunosuppressants treatment is reduced or stopped prior to 6 months following HSCT, preferably, within a period from 3-6 months, or from 3-5 months, or from 3-4 months following HSCT.

In a specific embodiment, said immunosuppressants treatment (for example cyclosporine A treatment) is reduced substantially (tapered), for example of at least about 10%, 20%, 30%, 40%, 50%, 60%, 70%, 80%, 90%, 100% compared to the starting dose of said immunosuppressant.

Tapering of immunosuppressant treatment may be performed rapidly or gradually, for example with a gradual decrease of the dose for 1, 2, 3, 4, 5, or 6 weeks after 1, 2, or 3 months after HSC transplant, and/or discontinued treatment after at least 3, 4, 5, or 6 months after HSC transplant. For example, the immunosuppressive dose may be gradually reduced by 6 weeks to two months, and discontinued in 3 to 4 months after HSC transplant. The physician will determine the most appropriate tapering regimen depending on the conditions of the patient.

In another embodiment, the method of the present disclosure comprises the administration of the S1P receptor modulator, preferably mocravimod, in order to achieve: reduction in incidence of refractory GVHD, delay in relapse or increase in relapse-free survival, increase in overall survival or progression free survival, or improvement in quality of life.

In specific embodiments, the methods of the present disclosure delays or prevents the onset of acute GVHD, chronic GVHD, relapse AML or death, in particular for at least 3, 6, 9, 12, 18, or 24 months after HSCT. In specific embodiments, the methods of the present disclosure delays or prevents the onset of AML relapse while delaying or preventing the onset of GVHD (grade III/IV) refractory to systemic immunosuppressive treatment, in particular for at least 3, 6, 9, 12, 18, or 24 months after HSCT.

In specific embodiments, the methods reduce the morbidity or mortality in a population of subjects, in particular via GVHD-free, relapse-free, survival and both relapse-related mortality and transplant-related mortality. For example, said methods reduce the occurrence, or severity of either GVHD, refractory GVHD, relapse or mortality in particular for at least 3, 6, 9, 12, 18, or 24 months after HSCT, in a population of subjects.

In specific embodiments, said patient is refractory GvHD-free, relapse free (rGRFS) after at least 3, 4, 5, 6, 7, 8, 9, 10, 11, 12, 13, 14, 15, 16, 17, 18, 19, 20, 21, 22, 23, or 24 months from HSCT.

As used herein, rGRFS is calculated similarly to conventional GRFS treating grade III to IV acute GVHD, chronic GVHD requiring systemic treatment, and disease relapse/progression as events, except that GVHD that resolved and do not require systemic treatment at the last evaluation is excluded as an event in rGRFS.

Accordingly, in specific embodiments of the methods, said subject does not present one or more of the following:
- grade III/IV acute graft-versus-host disease (GVHD) refractory to at least 2 lines of immunosuppressive treatment,
- extensive chronic GVHD refractory to systemic immunosuppressive treatment,
- disease relapse,
- death,
after at least 3, 4, 5, 6, 7, 8, 9, 10, 11, 12, 13, 14, 15, 16, 17, 18, 19, 20, 21, 22, 23, or 24 months from HSCT.

Also disclosed herein are methods for improving overall survival of subjects, said method comprising for each subject treating the subject with any of the method using the S1P receptor modulator as disclosed herein.

In specific embodiments, said method improves the quality of life of subjects, preferably as measured by the Foundation for the Accreditation of Cellular Therapy (FACT-BMT) Bone Marrow Transplantation questionnaire and/or the MD Anderson symptom inventory (MDASI), at 3, 6, 12 or 24 months as compared with subjects treated without S1P receptor modulator administration according to the standard of care for HSCT. Improvement may be significant, and for example an increase of at least 10%, 20%, 30%, 40%, 50%, 60%, 70%, 80%, 90%, 100%, or more.

In another embodiment, the S1P receptor modulator for use according to the present disclosure has
- maximum plasma levels (Cmax) of between 0.4 ng/mL and 1 ng/mL, preferably between 0.55 ng/mL and 0.85 ng/mL, more preferably between 0.65 ng/mL and 0.75 ng/mL,
- time to maximum plasma level (Tmax) between 2h and 10h, preferably between 4h and 8h, more preferably between 5h and 7h; and,
- area under the plasma concentration curve (AUClast) of between 60h*ng/mL and 74h*ng/mL, preferably between 64h*ng/mL and 70 h*ng/mL, more preferably between 66.5 h*ng/mL and 67.5 h*ng/mL.

In another embodiment, the S1P receptor modulator for use according to the present disclosure has
- maximum plasma levels (Cmax) between 0.6 ng/mL and 1.20 ng/mL, preferably between 0.75 ng/mL and 1.05 ng/mL, more preferably between 0.85 ng/mL and 0.95 ng/mL,
- time to maximum plasma level (Tmax) between 6h and 14h, preferably between 8h and 12h, more preferably between 9h and 11h, and
- area under the plasma concentration curve (AUClast) of between 63.5 h*ng/mL and 77.5 h*ng/mL, preferably between 67.5 h*ng/mL and 73.5 h*ng/mL, more preferably between 69.5 h*ng/mL and 71.5 h*ng/mL

In another embodiment, the S1P receptor modulator for use according to the present disclosure has a half time (T ½) of between 110h and 134h, preferably between 116h and 128h, more preferably between 121h and 123h.

In another embodiment, the S1P receptor modulator for use according to the present disclosure has a half time (T ½) of between 100h and 122h, preferably between 105h and 117h, more preferably between 110h and 112h.

### FIGURES

**Figure 1****:** Incidence of Mild chronic GVHD in patients who have received KRP 203 1mg+ CsA, KRP203 3mg + CsA and KRP203 3 mg + TAC.
**Figure 2****:** Favourable relapse incidence in patients who have received KRP 203 1mg+ CsA, KRP203 3mg + CsA and KRP203 3 mg + TAC.
**Figure 3****:** Incidence of Moderate chronic GVHD in patients who have received KRP 203 1mg+ CsA, KRP203 3mg + CsA and KRP203 3 mg + TAC.
**Figure 4A****:** KRP203 as prophylactic treatment for chronic GVHD. Clinical score of skin pathology in mice with GVHD, who either received KRP203 as prophylactic treatment (black) or vehicle (grey).
**Figure 4B****:** KRP203 as prophylactic treatment for chronic GVHD. Schirmer test result to measure aqueous tear production on day 42 in mice with (black) or without (grey) prophylactic KRP203 treatment.
**Figure 5A****:** KRP203 treatment combined with short- or long-term CsA. Overall survival of mice who received either T-cell depleted bone marrow (TCDBM) alone, or TCDBM + T cells and different treatment combinations.
**Figure 5B****:** KRP203 treatment combined with short- or long-term CsA. Tumor count in mice who received TCDBM alone or TCDBM + T and different treatment combinations.

### EXAMPLES

### Example 1: HSCT with mocravimod for the treatment of AML

The methods of the present disclosure may be carried out according to the following way:
Human patients in first complete remission (CR1) or second complete remission or more (CR2) and who are eligible for a HSCT collected from peripheral blood stem cells of sibling or matched unrelated donor can be subject to this therapy.

The therapy includes a daily chronic administration starting at day -11 prior to HSCT of an oral composition of mocravimod at a dose of 3mg per day, for a duration of at least 6 months, and preferably at least 12 months, or during the whole life of the subject (chronic administration), until relapse.

Together with KRP203, patients receive standard of care for GVHD prophylaxis starting preferably on day -4 prior allogeneic HSCT and which will include a combination of ciclosporin A and methotrexate.

The patient is subject to the following conditioning regimen: Fludarabine; 30 mg/m² i.v. once daily for 5 days on day -8 to -4 (150 mg/m2), Thiopeta; 5 mg/kg i.v. twice daily for 1 day on day -7 (10mg/kg) and Melphalan; 60 mg/m² i.v. once daily for 2 days on day -2 and -1 (120 mg/m²). Conditioning is followed by allogeneic HSCT. Granulocyte colony-stimulating factor (G-CSF)-mobilized peripheral blood stem cells (PBSC) will be the preferred source for hematopoietic stem cells (HSC). Donor should be HLA-matched related or unrelated with 8/8 or higher matches at the HLA-A, -B, -C, -DRB1, and/or -DQB1 loci, as determined by high resolution HLA typing.

### Example 2: Clinical Study for treating AML patients with high risk of relapse

Provided herein is a prophetic example describing a prospective randomized, open label, multi-center phase II comparative study to evaluate the efficacy and safety of mocravimod in patients with acute myeloid leukaemia undergoing allogeneic hematopoietic stem cell transplantation (HSCT) .

### Synopsis

| Investigational drug | Mocravimod (KRP203) | |
|---|---|---|
| Study phase | IIb | |
| Trial title | A prospective randomized, open label, multi-center phase II comparative study to evaluate the efficacy and safety of mocravimod in patients with acute myeloid leukaemia undergoing allogeneic hematopoietic stem cell transplantation | |
| Trial Background | Mocravimod is a sphingosine 1-phosphate receptor modulator (S1P). developed as an adjunctive therapy to allogeneic hematopoietic stem cell transplantation (HSCT) with the aim of improving immunology driven outcomes in patients with Acute Myeloid Leukaemia (AML). | |
| | The mechanism of action of mocravimod is related to active phosphorylated metabolites which block lymphocytes egress from secondary lymphoid organs thereby reducing the number of circulating lymphocytes. | |
| | Extensive preclinical and Proof of Concept clinical data suggest that S1P has marked effects on the outcomes that most impair overall outcome of HSCT for aggressive malignant hematological diseases: Relapse and GvHD. The profile of mocravimod is promising for being developed in combination as adjunctive treatment to HSCT resulting in delayed disease progression, reduced GvHD, and improved overall survival in patients with AML. | |
| | For the current trial, treatment with mocravimod, starting 11 days prior to and lasting up to 1 year after HSCT may trigger two positive effects: 1) Graft vs Host effect (GvH) to be reduced because allo-reactive T cells are retained in the lymphoid tissues and undergo activation induced cell death and do not migrate into the peripheral tissues 2) Graft vs Leukemia effect (GvL) to be increased through the eradication of residual disease by allo-T cells sequestered in the lymphoid tissues such as lymph nodes, the bone marrow and | |
| | the spleen white pulp. Leukemic cells (blasts) originate in the bone marrow and venture to peripheral blood and other tissues when morphologic relapse occurs. However, at the time patients reach complete remission, further to remission induction/consolidation, a minimal number of blasts surviving in the bone marrow and lymphoid tissues are sufficient to eventually result in relapse. Alloreactive lymphocytes co-transferred with the apheresis stem cell product play a key role in GvL effect. Progressive immune reconstitution after HSCT supports GvL by allo-reactive T cells in the bone marrow and the lymphoid tissues. Thus, since mocravimod is not immune-suppressive, allo-reactive T cells can retain anti-leukemia reactivity to boost GvL without off target reactivity translating into GVHD. A clinically relevant improvement of Morbi-Mortality in patients receiving HSCT is expected from the adjunction of mocravimod to SoC via GVHD-free, Relapse-free Survival and both Relapse-Related Mortality (RRM) and Transplant-Related Mortality (TRM). | |
| | Compared to alternative therapeutic options such as personalized therapies focusing on molecular targets determined by tumor genetic profile at diagnosis or after induction, alloreactive T cells have a broad polyclonal specificity profile. Thus, controlling escape mutant leukemia via HSCT with adjunctive mocravimod on a broader target population not limited by molecular target may happen to address some strong limitations of personalized therapies or other therapies such as hypomethylating agents used for complete remission maintenance. In addition, maintenance therapies aiming at improving Progression-Free Survival (PFS) do not address GVHD issues in post-transplant settings. | |
| | As no clinically relevant comparator is approved, the assessment will be conducted with or without mocravimod on top of standard of care (SoC). | |
| | The positive effects of mocravimod on stem cell engraftment enhancement and GVH effect inhibition have been demonstrated in different animal models and in study CKRP203A2105. Based on the known S1P biology, the findings are attributed both to modulation of effector T cell migration to GVHD target tissues and to migration of Hematopoietic Stem Cells from the bone marrow niche. | |
| | Safety pharmacology and toxicology studies for both mocravimod and other S1PR modulators show some commonalities in terms of mechanistic effects The broad clinical experience with other S1PR modulators in MS provides support for the in-class safety to be expected with use of mocravimod that is further supported by the very similar healthy volunteer safety data of the two compounds . | |
| | The clinical experience with mocravimod includes dosing in healthy volunteers with single doses up to 40 mg, and multiple doses up to 3 mg/day in healthy volunteers and in patients. A Phase 1b study CKRP203A2105, and in which mocravimod (3 mg/day) was administered in combination to an immunosuppressive regimen based on cyclosporin (CsA) and methotrexate (MTX) in patients with hematologic malignancies receiving HSCT, showed promising results regarding mocravimod's potential to decrease GVH effect while preserving GVL. **There is a good rationale based on Phase 1b trial analysis for a longer duration of treatment which may improve survival.** | |
| | These data support proceeding with a pivotal clinical trial to assess mocravimod as adjunctive therapy in HSCT in combination with SoC. SoC regimens for GVHD prophylaxis vary according to local practice and may include the use of a calcineurin inhibitor (CNI), such as cyclosporine A (CsA), in conjunction with another immunosuppressant, such as methotrexate. However, no specific GVHD prophylaxis regimen is well established as the only therapeutic option. | |
| | In the present trial, SoC for GVHD prophylaxis will include a combination of CsA and MTX in the active arm whereas the control arm will allow the local institutional GVHD prophylaxis usual regimen. | |
| | Mocravimod is to be used for the whole duration of the treatment phase in the active arm starting in the initial pre- transplantation period (11 days before HSCT). | |
| Trial rationale | The primary purpose of this open label-controlled study is to confirm the efficacy of mocravimod treatment for a duration of 1 year after HSCT from sibling or Matched Unrelated Donor, in patients with AML in High risk first remission or in second or subsequent remission. | |
| | Mocravimod is the active ingredient and the trial investigational medicinal product (IMP). | |
| | The dosing regimen of mocravimod in the setting of HSCT for hematologic malignancies in adults has been established in the Phase 1b study CKRP203A2105, in which mocravimod (3 mg/day) was administered for a treatment duration around 110 days in combination with various immunosuppressive GVHD prophylaxis regimen including a regimen based on ciclosporin (CsA) and methotrexate (MTX). | |
| | Mocravimod as adjunctive treatment in HSCT is a promising approach for protection against disease relapse, while lowering the risk of severe GVHD (grade III/IV) refractory to therapy in patients with AML. | |
| Objectives | **Primary objectives** | |
| | To confirm mocravimod treatment effect for 1 year as adjunctive treatment to HSCT in order to improve the safety and efficacy of HSCT. HSCT is performed with an apheresis product collected from peripheral blood stem cells from HLA-matched sibling or | |
| | unrelated donor, in subjects with AML in first (CR1) or second and subsequent Complete Morphologic Remission (CR2). | |
| | **Secondary objectives** | |
| | To confirm mocravimod effect on overall survival at 2 years, disease free survival at 2 years, and Quality of Life (QoL) | |
| Trial Endpoints | **Primary endpoint:** | |
| | Refractory GVHD-free, relapse-free survival (rGRFS). | |
| | rGRFS is defined as time from HSCT until whatever occurs first: | |
| | • grade III/IV acute graft-versus-host disease (GVHD) refractory to at least 2 lines of treatment | |
| | • extensive chronic GVHD refractory to systemic immunosuppressive treatment | |
| | • disease relapse | |
| | • death | |
| | This endpoint captures both safety and efficacy. | |
| | rGRFS is calculated similarly to conventional GRFS treating grade III to IV acute GVHD, chronic GVHD requiring systemic treatment, and disease relapse/progression as events, except that GVHD that resolved and do not require systemic treatment at the last evaluation is excluded as an event in rGRFS | |
| | **Key secondary endpoint:** | |
| | • Overall survival | |
| | **Secondary efficacy endpoints:** | |
| | • Progression-free survival | |
| | • Cumulative incidence of Relapse | |
| | **Safety endpoints** | |
| | • Incidence of macular edema | |
| | • Incidence of Liver function abnormality leading to dose reductions/treatment discontinuation | |
| | • Incidence of Post-Transplant Lymphoproliferative Disease | |
| | • Incidence of viral, bacterial and fungal severe infectious complications including EBV/CMV and incidence of pre-emptive, curative use of anti-viral therapy | |
| | • Cumulative incidence of NCI CTCAE grade 3-5 adverse events. | |
| | **Quality of Life endpoint** | |
| | The Foundation for the Accreditation of Cellular Therapy- Bone Marrow Transplantation questionnaire (FACT-BMT, version 4), the Short Form 36-item health survey (SF-36, version 2), and the MD Anderson Symptom Inventory (MDASI) will be scored at Screening, Month 3, Month 6, Month 12, and Month 24, provided that validated translations in local language are available. | |
| | **Exploratory endpoints** | |
| | • Time to engraftment. | |
| | • Time to acute GvHD and Grade of aGvHD | |
| | • Time to chronic GvHD and Grade of chronic GvHD | |
| | • Immunosuppressants free survival at one year (IFS) | |
| | • Antibodies titers Response to Flu Vaccination | |
| | • Median duration and Average dose of GvHD prophylaxis over the first year of treatment | |
| | • Incidence of concomitant therapy and Cumulative dose of corticosteroids during the first year of treatment | |
| | • Incidence of concomitant therapy and Cumulative dose of Ruxolitinib over the first year of treatment | |
| | • Cumulative incidence of NCI CTCAE grade 2-5 and grade 3-5 infections | |
| Overall trial design | This study is designed as a prospective, multicenter randomized, 1-year treatment period open label-controlled parallel group design, followed by an additional 1-year extension period using a single-arm, open label design. | |
| | Subjects will be randomly assigned using an interactive response system to either mocravimod or control group (1:1 ratio) and stratified by CR2 vs CR1 | |
| | After being randomized every subject start study treatment for 1 year unless safety concerns require treatment interruption or discontinuation as per investigator judgement. Subjects who would have completed the 1^{st} year on treatment (on either arm) will be proposed to receiving study treatment for an additional year in an open label setting. The analysis of primary endpoint will take place when all subjects will have completed the double-blind period or when the trial reach the pre-determined number of event for analysis , whichever occurs first.. | |
| | Recruitment period (FPFV to LPFV) is 12 months. | |
| | Duration of the study for each subject is 2 years. | |
| | The estimated time from start of enrolment until completion of the data analysis of the primary endpoint amounts to 27 months. The estimated time from start of enrolment until completion of the final data analyses amounts to approximately 40 months. | |
| | Safety review will be performed by an Independent Safety Monitoring Board (SMB), and adjudication of Death, GvHD, Macular edema, Heart Rhythm Disorders and Liver abnormalities | |
| | will be performed by an independent adjudication committee. The SMB may recommend | |
| | • to continue the study as planned or with amendments; | |
| | • to prematurely stop the study for unfavorable risk/benefit of mocravimod compared to control; | |
| | • to prematurely stop the study as lack in adherence to the study protocol will not allow to meet the primary study objective; | |
| | • to prematurely stop the study for substantial, robust and compelling evidence for a favorable risk/benefit relationship of mocravimod compared to control. | |
| Trial Phases | The trial for each subject will consist of four phases: | |
| | • Informed consent | |
| | • Screening and enrolment phase | |
| | • Treatment phase | |
| | • Follow-up phase, with an end of trial (EOT) visit at 24 months post-HSCT | |
| | Informed consent: | |
| | The Investigator will pre-screen potentially eligible AML subjects in CMR (including complete remission with incomplete blood recovery [CRi]) | |
| | Screening and enrolment phase: | |
| | After providing informed consent to participate in the trial, potentially eligible subjects will first undergo a BM harvest to be screened for AML disease assessment. Enrolment and registration will be performed before the start of the conditioning | |
| | regimen, after all eligibility assessments have been completed and the subject is confirmed to be eligible for the trial | |
| | Treatment phase | |
| | Enrolled subjects in the mocravimod group will receive a daily oral dose of 3 capsules for 1 year starting from Day -11 before planned HSCT, and therefore starting before conditioning regimen to be administered on Days -6 to-3 prior to HSCT. Enrolled subjects in the control group will not receive mocravimod. | |
| | Follow-up phase: | |
| | All subjects having completed the treatment phase, regardless of the duration will start a Follow-up phase in an open label setting for one extra year before an EOT visit. | |
| Trial Population | Male or female subjects aged 18-75 years with AML in Complete remission (CR1, CR2) who are eligible for a HSCT collected from peripheral blood stem cells of sibling or matched unrelated donor, | |
| | CMR is defined as leukaemia clearance (< 5% marrow blasts and no circulating peripheral blasts) in conjunction with normal values for absolute neutrophil count and platelet count, no extramedullary manifestation of leukaemia and no need for repeat blood transfusions. | |
| | CRi is defined as meeting all CMR criteria except for an absolute neutrophil count < 1 ,000/pL or platelet count < 100,000/µL | |
| Number of subjects | In total, it is planned to enrol 160 subjects with AML in CMR (including CRi) with indication for HSCT. | |
| Inclusion and exclusion criteria | Inclusion Criteria | |
| | Each patient must meet the following criteria to be enrolled in this study: | |
| | • Subjects with a diagnosis of AML and related precursor neoplasms according to the WHO 2016 classification (excluding acute promyelocytic leukaemia), including secondary AML after an antecedent haematological disease (e.g. myelodysplastic syndrome) and therapy-related AML | |
| | • Subject is planned to undergo allogeneic HSCT from fully matched sibling donor or Unrelated Donor with an 8/8 match at HLA-A, -B, -C and DRB1 at high resolution by DNA-based typing., whereas Stem cell source is mobilized peripheral blood collected via apheresis by a compatible Donor. The minimum recommended CD34+ cell dose in the graft should be 2 × 10⁶/kg, and the recommended target dose should be 5 × 10⁶/kg. | |
| | • Subjects with AML in first cytomorphological remission (CR1) and ELN adverse classification or subjects in subsequent cytomorphological remission (CR2). CR is defined as leukaemia clearance (< 5% marrow blasts and no circulating peripheral blasts) in conjunction with normal values for absolute neutrophil count and platelet count, no extramedullary manifestation of leukaemia and no need for repeat blood transfusions. CRi is defined as meeting all CMR criteria except for an absolute neutrophil count < 1,000/pL or platelet count < 100,000/µL. | |
| | • Life expectancy ≥ 6 months at screening | |
| | • Karnofsky Performance Status (KPS) ≥ 70% | |
| | • Male or female, age ≥ 18 years and ≤ 75 years _ | |
| | | Subjects ≥ 65 years must have a Sorror Score ≤ 3 |
| | • Able and willing to provide written informed consent and comply with the trial protocol and procedures | |
| | • For females of childbearing potential who are sexually active and males who have sexual contact with a female of childbearing potential: willingness to use reliable methods of | |
| | contraception (oral contraceptives, intrauterine device, hormone implants, contraceptive injection or abstinence) during study participation | |
| | A female is considered of childbearing potential following menarche and until becoming post-menopausal unless permanently sterile. Women are considered post-menopausal and not of child bearing potential if they have had 12 months of natural (spontaneous) amenorrhea with an appropriate clinical profile (e.g. age appropriate, history of vasomotor symptoms) or have had surgical bilateral oophorectomy (with or without hysterectomy) or tubal ligation at least six weeks ago. In the case of oophorectomy alone, only when the reproductive status of the woman has been confirmed by follow up hormone level assessment, she is considered not of child bearing potential. | |
| | • Affiliation to a national health insurance scheme (according to applicable local requirements) | |
| | Exclusion Criteria | |
| | Patients who meet any of the following criteria will be excluded from the study: | |
| | • Subjects having received prior allogeneic HSCT or recipient of a solid organ transplant. | |
| | • Subjects with acute promyelocytic leukaemia | |
| | • Diagnosis of any previous or concomitant malignancy is an exclusion criterion, except when the subject completed treatment (chemotherapy and/or surgery and/or radiotherapy) with curative intent for this malignancy at least 6 months prior to enrolment | |
| | • Blast crisis of chronic myeloid leukaemia | |
| | • Concurrent severe and/or uncontrolled medical condition including | |
| | | ∘ Clinically significant pulmonary fibrosis |
| | | ∘ Tuberculosis, except for history of successfully treated tuberculosis or history of prophylactic treatment after positive PPD skin reaction |
| | | ∘ Patients receiving chronic (daily) therapies for asthma |
| | | ∘ Patients with any other types of clinically significant bronchoconstructive disease |
| | | ∘ . Uncontrolled diabetes mellitus as assessed by the investigator or diabetes complicated with organ involvement such as diabetic nephropathy or retinopathy. |
| | | ∘ 7. Uncontrolled seizure disorder |
| | | ∘ 8. Uncontrolled depression or history of suicide attempts/ideation |
| | • Cardiac dysfunction as defined by: | |
| | | ∘ Myocardial infarction within the last 3 months of trial entry, or |
| | | ∘ Reduced left ventricular function with an ejection fraction < 40% as measured by multi-gated acquisition (MUGA) scan or echocardiogram (echo) within 28 days before screening, or |
| | | ∘ History or presence of stable or unstable ischemic heart disease (IHD), myocarditis, or cardiomyopathy, or |
| | | ∘ New York Heart Association (NYHA) Class II-IV congestive heart failure, or |
| | | ∘ Unstable cardiac arrhythmias including history of or presence of symptomatic bradycardia, |
| | | ∘ Resting heart rate (physical exam or 12 lead ECG) <60 bpm |
| | | ∘ History or current diagnose of ECG abnormalities indicating significant risk of safety such as: Concomitant clinically significant cardiac arrhythmias, e.g. sustained ventricular tachycardia, presence of a clinically relevant |
| | | impairment of cardiac conduction including sick sinus syndrome, or sino-atrial heart block, clinically significant AV block, bundle branch block or resting QTc (Fridericia preferred, but Bazzet acceptable) > 450 msec for males and > 470 msec for females at Screening or Baseline ECG |
| | | ∘ History or presence of symptomatic arrhythmia or arrhythmia requiring treatment or being otherwise of clinical significance |
| | | ∘ Uncontrolled arterial hypertension; if controlled, the medication must be stable for three (3) months prior to baseline visit |
| | | ∘ Treatment with medication that impairs cardiac conduction (e.g., beta blockers, verapamil-type and diltiazem-type calcium channel blockers, or cardiac glycosides) |
| | | ∘ Concomitant use of agents known to prolong the QT interval unless they can be permanently discontinued for the duration of the study |
| | | ∘ Treatment with quinidine |
| | | ∘ History of syncope of suspected cardiac origin |
| | | • History of familial long QT syndrome or known family history of Torsades de Pointes |
| | • Pulmonary dysfunction as defined by oxygen saturation < 90% on room air. Pulmonary function test (PFT) is required only in the case of symptomatic or prior known impairments within 28 days before screening - with pulmonary function < 50% corrected diffusing capacity of the lung for carbon monoxide (DLCO) and forced expiratory volume in 1 second (FEV₁) | |
| | • Significant liver disease or liver injury or known history of alcohol abuse, chronic liver or biliary disease | |
| | • Hepatic dysfunction as defined by AST and/or ALT> 5 x ULN | |
| | • Renal dysfunction with Serum creatinine > 2.0 mg/dL (176 µmol/L) | |
| | • Vaccination with live, attenuated vaccines within 4 weeks prior to screening | |
| | • Immunosuppressive drugs for concomitant disease. Subjects must be able to be off prednisone or other immunosuppressive medications for at least 3 days prior to the start of treatment of the study | |
| | • History of stroke or intracranial haemorrhage within 6 months prior to screening | |
| | • Active infections (viral, bacterial or fungal) that requires specific therapy. Acute anti-infectious therapy must have been completed within 14 days prior to trial treatment | |
| | • History of human immunodeficiency virus (HIV) or active infection with hepatitis B virus (HBV) or hepatitis C virus (HCV) defined as a positive HIV antibody, Hepatitis B surface antigen or Hepatitis C | |
| | • History of a previous malignancy, treated or untreated, within the past 5 years, regardless of whether there is evidence of local recurrence or metastases, with the exceptions of localized basal cell carcinoma of the skin or in-situ cervical cancer | |
| | • Current concomitant chemotherapy, radiation therapy, or immunotherapy | |
| | • Major surgery within 4 weeks prior to screening or a major wound that has not fully healed | |
| | • Positive pregnancy test or breastfeeding of subject (women of childbearing age only) | |
| | • Estimated probability of surviving less than 3 months | |
| | • Known allergy to any of the components of mocravimod (e.g., excipient) | |
| | • Any contraindication for GVHD prophylaxis with ciclosporin A, or Methotrexate | |
| | • Diagnosis of macular edema during screening. Patients with a history of macular edema will be allowed to enter the study provided they do not have macular edema at the ophthalmic examination at screening | |
| | • Participation in another interventional clinical trial within 4 weeks prior to trial enrolment or participation in a concomitant interventional clinical trial | |
| | • Any other condition that, in the opinion of the investigator, makes the patient or donor ineligible for the study | |
| | • Subjects under legal protection measure (guardianship, trusteeship or safeguard of justice) and/or inability or unwillingness to comply with the requirements and procedures of this trial | |
| Trial Duration | Each subject is planned to be in the trial for approximately 25 months, i.e. from signing informed consent to last visit. | |
| | All subjects will be followed up until 24 months after the start of treatment phase. | |
| | The expected duration of accrual is 12 months, | |
| | From enrolment of the first subject in the treatment phase to the last visit of the last subject in the Follow-up phase , the trial duration is expected to be up to 37 months (including a 12-month enrolment period). | |
| | The trial is planned to start in Q3 2021 and completion (including the final clinical study report) is expected in Q1 2025. | |
| Investigational and control | The Investigational medicinal product is mocravimod and is presented a capsule for oral use. | |
| Medicinal products | Each capsule in the trial contains 1 mg of mocravimod. | |
| IMP dose, mode of administration and control group | The IMP will be administered per os at a dose of 3 mg per day for 1 year: | |
| | Justification for the selected dose and duration of is based on the previous clinical experience in trials conducted with mocravimod, especially in post-transplant setting. | |
| | Dosing of the IMP in the present trial is not body weight-related as no dose-dependent effects on safety and preliminary signs of efficacy were observed in the CKRP2105 trial. | |
| | Having completed the screening period, subjects enrolled and randomized to mocravimod will begin study treatment from Day-11 until one-year post transplant or until all immunosuppressants have been tapered and successful weaning for at least 4 weeks. | |
| | When the subject has signed the informed consent form, the investigator or his/her staff will call the Interactive Response System (IxRS) and provide the requested identifying information for the subject. The IxRS will then assign the subject number and the procedure will be completed for treatment allocation. | |
| | No restrictions on concomitant medications for GvHD treatment will be made. | |
| | However, remission maintenance therapy such as targeted treatment FLT3 inhibitor, Hypo Methylating agents, Donor Lymphocyte Infusion prophylaxis are not allowed, regardless of the trial arm. | |
| Efficacy assessment(s) | Efficacy measurements will include the Event-Free Survival and Cumulative Incidence of Relapse | |
| | rGRFS is a primary efficacy endpoint and is a composite index for the evaluation of the impact of the IMP on the HSCT performance. | |
| | The dominant components of rGRFS are Relapse and death events. | |
| | Overall Survival at EOT is a key secondary efficacy endpoint. | |
| | Quality of Life assessment will support the efficacy analysis. | |
| | Pharmaco Kinetic blood samples will be collected in the active group at the start of the study at Day+2 and Day +7 and post-HSCT, | |
| Safety assessment(s) | Safety will be monitored up to 24 months after HSCT and separately evaluated for IMP and for the overall trial treatment (HSCT complications) | |
| | Safety will be assessed, as indicated, by means of: | |
| | • Macular edema screening: Fundoscopy before treatment initiation and at Week 4, Month 3 ,Month 12 and EoT | |
| | • Routine safety laboratory tests, including standard serology panel, pregnancy, haematology, clinical chemistry, and urinalysis tests | |
| | • Viral monitoring (cytomegalovirus [CMV, EBV) | |
| | • Karnofsky performance status | |
| | • Physical examination and vital signs | |
| | • Cardiac function evaluation by ECG, echo or MUGA and Cardiac monitoring by ECG before and 24 hours after first dose for Bradycardia screening | |
| | • Renal function evaluation by monitoring of the creatinine clearance | |
| | • Concomitant medication, therapy, and illness | |
| | • Collection of AEs (including monitoring of infection, and GVHD) | |
| | • PK of immunosuppressive backbone will be recorded throughout and will be measured centrally at visit dates to be able to cross compare | |
| Patient reported outcomes | The Foundation for the Accreditation of Cellular Therapy- Bone Marrow Transplantation questionnaire (FACT-BMT, version 4), and the MD Anderson Symptom Inventory (MDASI) will be scored at Screening, Day +14, Day +28, Day +60, Month 3, Month 6, Month 12, and Month 24, provided that validated translations in local language are available. | |
| Exploratory assessment | Engraftment, Immunosuppressants free survival at one year (IFS) Antibodies titers Response to Flu Vaccination, duration and dose of GvHD prophylaxis over the first year of treatment Incidence of concomitant therapy and Cumulative dose of corticosteroids during the first year of treatment Incidence of concomitant therapy and Cumulative dose of Ruxolitinib over the first year of treatment, Immune phenotyping of lymphocyte subpopulation by Cell Type-Specific Epigenetic DNA Markers. CD3+,CD4+,CD8+,Treg, B cells by Epiontis technology (epigenetic methylation), ST2 | |

### INTRODUCTION

### Rationale for use of Mocravimod

Mocravimod is a sphingosine 1-phosphate receptor modulator (S1P) developed by the applicant as an adjunctive therapy to allogeneic hematopoietic stem cell transplantation (allo-HSCT). S1P is a class of products associated with effect on the immune system. Some products are in development or are approved already as immunotherapy for the treatment of auto-immune diseases. Fingolimod (Gilenya; FTY720) is approved for the treatment of multiple sclerosis (MS). Mocravimod mechanism of action is similar to fingolimod. Both are prodrugs and their active phosphorylated metabolites block lymphocytes egress from lymph nodes thereby reducing the number of circulating lymphocytes. However, the mocravimod profile is attractive for being developed in conjunction with allo-HSCT and standard of care (SoC) to improve disease progression rate, overall survival and Quality of Life in patients with Acute Myelogenous Leukemia. Treatment with mocravimod (starting 11 days prior to and lasting up to 1 year post HSCT) will lead to sequestration of lymphocytes into secondary lymphoid organs. Through the sequestration process two positive effects are triggered: 1) Graft vs Host effect (GvH) is reduced because allo-reactive T cells do not migrate into the peripheral tissues 2) Graft vs Leukemia effect (GvL) is increased through the eradication of residual disease by allo-T cells sequestered in the lymphoid tissues such as lymph nodes and the spleen white pulp. Leukemic cells (blasts) originate in the bone marrow and venture to peripheral blood and other tissues when morphologic relapse occurs. However, only a minimal number of blasts can survive in the bone marrow and lymphoid tissues further to remission induction/consolidation therapy when patients reach complete remission. Progressive immune reconstitution after HSCT supports GvL by allo-reactive cells in the bone marrow and the lymphoid tissues. Lymphocytes such as allo-T cells play a key role in GvL effect and reside primarily in lymphoid tissues. Thus, since mocravimod is not immune-suppressive, allo-reactive T cells can retain anti-leukemia reactivity to boost GvL without off target reactivity translating into GVHD. A strong improvement of Morbi-Mortality in patients receiving HSCT is expected from the adjunction of mocravimod to SoC, via both Relapse-Related Mortality (RRM) and Transplant-Related Mortality (TRM).

Compared to alternative therapeutic options such as targeted therapies focusing on molecular biomarkers determined by tumor genetic profile at diagnosis, alloreactive T cells have a broader specificity profile. Thus controlling escape mutant leukemia via HSCT with adjunctive mocravimod on a broader target population may happen to address some strong limitations of targeted therapies. As no clinically relevant comparator is approved, the assessment of mocravimod is conducted versus placebo. These data and other data support proceeding with a pivotal clinical trial to assess mocravimod as adjunctive therapy in allo-HSCT in combination with SoC. SoC regimens vary according to local practice and may include the use of a calcineurin inhibitor (CNI), such as CsA, in conjunction with another immunosuppressant, such as MTX. The Treatment of the study is to be administered for the whole treatment phase (1 year) starting in the initial pre- transplantation period (11 days before HSCT) and in conjunction with a GVHD prophylaxis regimen based on CsA and MTX in the mocravimod arm, and according to the clinical site institutional practice in the control arm.

### Allogeneic hematopoietic stem cell transplantations in High-risk AML

Allo-HSCT is a key component of the current standard of care in AML management and is indicated for induction failure (refractory), after Relapse, and for Consolidation of Complete Remission.

Compared to chemotherapy, Allo-HSCT reduces the risk of relapse, but with a higher risk of treatment-related mortality. In AML, allo-HSCT is indicated if the risk of relapse is > 35% (Döhner et al., Blood 2017 Jan 26;129(4):424-447) and if the patient's age, comorbidities and frailty makes him fit for the procedure.

Allo-HSCT in High-risk AML is currently the only potential curative option. Although benefiting from GvL effect, this procedure is currently associated with a high risk of non-relapse morbidity and mortality due to GVHD. The risk of GVHD is currently related to the intensity of the GvL effect and is mitigated by immunosuppressant prophylaxis, which in turn decreases the GvL effect. Investigational treatment competing to HSCT in AML, such as molecularly targeted drugs, monoclonal antibodies and immunoconjugates, Checkpoint Inhibitors, T-cells engagers and CAR-T cells may lead to durable remissions and thus may compete with HSCT through extending survival without the prospect of a cure. Mocravimod's potential to decrease GvH effect while preserving GvL is promising as a way to keep the prospect of a cure while decreasing transplant-related mortality and morbidity.

### HSCT and GVHD.

Graft vs Host Disease (GVHD) is actually regarded as the off-target effect of the expected Graft vs Leukemia effect, involving the donor T-cells.

Alloreactivity is the basis of Graft vs Leukemia effect (GvL). GvL limits the post transplant relapse probability.

Alloreactivity stems from mismatch: alloantigens, minor HLA mismatch (donor-recipient polymorphism), major HLA mismatch (A,B,C,DR,DQ,DP), and Tumor alloantigens. GvHD may happen to be acute or chronic (aGvHD, cGvHD).

GvHD is common after alloHSCT with a high prevalence: aGvHD 30-70%, cGvHD 20-50% (Magenau et al., Br J Haematol. 2016 Apr;173(2):190-205). GvHD is a leading cause of post-transplant mortality and morbidity and a leading cause of post-transplant Quality of Life impairment. Despite some recent improvement in GvHD management, GvHD is still an issue. T-cell suppression via immunosuppressive prophylaxis by CNI and MTX, or first line therapy via systemic corticosteroids are associated with opportunistic infection, organ toxicity and risk of minimizing GvL effect. Alternatively, manipulation of specific immune modulators can decrease effector T cells involved in GvHD, and GvL, while increasing regulatory T cells and downregulating inflammatory pathways.

The combination of CsA and MTX has demonstrated a lower incidence of Grade II-IV aGvHD (Storb et al., N Engl J Med 1986 Mar 20;314(12):729-35) while efficacy remains suboptimal around 50% and tolerability is associated with nephrotoxicity and infection. Alternative prophylaxis regimen including tacrolimus, mycophenolate mofetil (MMF), Post-Transplant Cyclophosphamide, alpha 1 Anti Trypsine (AAT) are proposed in various settings. JAK inhibitors such as Ruxolitinib show a modest efficacy and have recently been approved for Steroid-Refractory GvHD therapy. Second line treatment include anti-TNF antibodies, anti-thymocyte globulin (ATG), sirolimus and MMF.

Extra-Corporeal Photopheresis (ECP) has been proposed as well in high risk/ Refractory aGvHD, possibly in combination with Mesenchymal Stem cells (MSC). Moreover, Fecal microbiota Transplant is investigative in aGvHD.

cGvHD management includes systemic corticosteroids with extended taper in first line, and ECP, rituximab or ibrutinib in second line

Some High risk biomarkers of GvHD have been identified: ST2 (IL-1R) and REG3alpha (intestinal injury biomarker).

### HSCT and Post-transplant Relapse

Post-transplant relapse in AML is biologically and clinically heterogenous. Some factors may impact the incidence of post-transplant relapse in AML.

The following risk factors are identified:
- The type of conditioning regimen: Reduced Intensity Conditioning is associated with high risk of relapse,
- The type of GvHD prophylaxis: intensive regimen based on Anti-Thymocyte Globulins (ATG) or ant-T cell antibodies, T cells depleted grafts are associated with high risk of relapse,
- Absence of Chronic GVHD is associated with high risk of relapse via poor GvL,
- HLA mismatch and loss of complete chimerism,
- Post transplant measurable residual disease (MRD): persistence or reappearance.

All patients beyond first relapse (CR2) are in need for HSCT and have a very high risk for relapse.

Clinical outcomes of HSCT vary depending on the depth of remission, and comorbidities, such as a fungal infection or organ dysfunction during induction.

HSCT outcome in CR2 AML is limited by the substantial number of patients who acquire comorbidities and cumulative chemotherapy toxicity compared to patients with HSCT in CR1.

Stratification CR2 vs CR1 is needed for interpretation of the mocravimod trial outcome, since HSCT basic performance may vary depending on CR status.

Standardization of relapse definitions and MRD assessment methods are needed.

Post-transplant clonal profile often happens to be different from the clonal profile at diagnosis.

Due to the polyclonal nature of AML, clones that evade prior molecular testing may determine relapse.

Various preventive and therapeutic approaches are being investigated to reduce relapse rate and include:
- High dose conditioning regimen! new agents,
- Early discontinuation of Immuno-Suppressive therapy,
- Pre-transplant therapy to change MRD positive status into negative status.

However, there is a need to combine tailored molecular monitoring (NGS), Multiparameter Flow Cytometry and routine Chimerism assessment to evaluate the MRD status in AML.
- Prophylactic DLI,
- Maintenance of remission via targeted therapy, epigenetic treatment, immune-based approaches.

In higher risk AML patients, the relapse rate is still over 60%, leading to an overall median disease-free survival (DFS) <1 year (range: 4-11 months).

Except HSCT, no maintenance therapy has demonstrated sufficient efficacy to be considered standard in AML.

Small molecules such as FLT3-inhibitors, Hypo Methylating agents, or Prophylactic Donor Lymphocyte Infusions are maintenance therapies.

Some progress has been made to reduce non-relapse mortality (NRM), but the risk of relapse after HSCT has not significantly decreased in the last decade.

**Disconnecting GvL and GvH, via limiting off target effect and improving the availability of T-cell on target is a desired approach to reduce the risk of relapse without increasing the risk of GVHD. Mocravimod is currently the only investigational product in clinical development with a suitable pharmacological profile and scientific rationale to meet this objective.**

### HSCT and Survival, GVHD-Free, Relapse-Free Survival (GRFS) Endpoint

Post- transplant survival is mostly related to AML relapse after HSCT, but Non-Relapse mortality is significant. Various strategies have been investigated to lower relapse and therefore improve OS.

Dose intensity of conditioning regimen matters, as relapse rate is higher with reduced Intensity conditioning, whereas Myelo Ablative Conditioning improves Survival and reduces Relapse. However, the impact of dose intensity may depend on genomics.

Immune Checkpoint blockade (CTLA4, PD1, PDL1, etc..), targeted therapies (TKIs), maintenance hypomethylating agents (HMA), and vaccines are investigational and may be associated with poor tolerability.

Making allo-HSCT as safe and simple as autologous HSCT while retaining allogeneic GvL effect is the objective of mocravimod therapy.

The performance of HSCT is assessed by GRFS, which is a composite endpoint for the evaluation of investigative medicinal products proposed as adjunctive to HSCT. GVHD-free/relapse-free survival (GRFS) in which events include grade 3-4 acute GVHD, systemic therapy-requiring chronic GVHD, relapse, or death in the first post-HSCT year.(Holtan et al., Biol Blood Marrow Transplant. 2015 Jun;21(6):1029-36)

More recently Refractory GRFS (rGRFS) was proposed as an accurate endpoint for Long-Term HSCT assessment. rGRFS is calculated similarly to conventional GRFS treating grade III to IV acute GVHD, chronic GVHD requiring systemic treatment, and disease relapse/progression as events, except that GVHD that resolve and do not require systemic treatment at the last evaluation is excluded as an event in rGRFS.

rGRFS is more accurate than GRFS for Mocravimod assessment: death, relapse or persistent severe GVHD.

### Study Intervention and IMP development program.

### Relevant Nonclinical Findings

KRP203 (mocravimod) is an immunomodulator that selectively targets sphingosine 1-phosphate (S1P) receptors via its phosphorylated active metabolite. KRP203-phosphate is a potent agonist on human S1P1 and S1P4 and a potent but partial agonist on hS1P3 and S1P5. The therapeutic effects are believed to be due to the prevention of effector lymphocyte recirculation from lymphatic tissue to susceptible target organs such as the gut, skin or CNS.

KRP203-phosphate induces a long-lasting and complete internalization of the S1P1, which renders these cells unresponsive to S1P, depriving them of an obligatory signal to egress from lymphoid organs. KRP203 does not inhibit allogeneic-stimulated T cell proliferation in mixed lymphocyte reactions (MLR), exhibit hematological cytotoxicity nor induces apoptosis of CD4+ T cells at therapeutic concentrations in vitro. KRP203 reduces lymphocyte counts in peripheral blood by more than 80%.

Using off-target binding assays, comprising GPCRs, ion channels, transporters, nuclear receptors and enzymes, large safety margins were calculated compared to an estimated KRP203-P free plasma concentration of 7 × 10-3 nM at maximum intended human dose of 3 mg/day. Thus it is unlikely that secondary pharmacology effects should arise due to non-specific receptor activation at therapeutic doses.

Safety pharmacology investigations revealed no significant effects on respiratory function in dogs at doses up to 20 mg/kg (highest dose tested) and no significant effects on bronchoconstrictor response induced by challenge agents in rats pre-treated with 3 mg/kg of KRP203. Except some piloerection, no adverse neuropharmacological effects were seen in rats at doses up to 2000 mg/kg. In a VEGF-induced vascular leakage model, KRP203 proved to be vasoprotective (barrier-protective), while it mildly decreased pulmonary endothelial barrier after single oral dose. Assessment of cardiovascular safety pharmacology in vitro did show a slight inhibitory potential of KRP203 on hERG channels (between 0.5 and 10 µM); however, the active moiety did not show significant effects up to the highest concentration tested, which was 3 µM. No APD prolongation was seen in a guinea pig papillary muscle preparation with concentrations of 3 µM of KRP203 or its phosphate. No indication for QT prolongation was observed in telemetered dogs or anaesthetized guinea pigs, but KRP203 was shown to have the potential to decrease heart rate (isolated rabbit heart or guinea pig; extrapolated exposure ≥0.1 µM) or to increase blood pressure (dogs; extrapolated exposure ≥0.3 µM). Based on the available in vitro and in vivo data, except for the potential to cause a negative chronotropic effect and some mild increase in blood pressure, there is no indication that KRP203 would cause arrhythmia or QT prolongation in man.

After oral administration of [14C]KRP203, radioactivity Tmax was 7 hours and 9.3 hours in male rats and dogs, respectively, indicating slow absorption. Blood distribution of [14C]KRP203 related radioactivity was species dependent and plasma protein binding of [14C]KRP203 was found to be high in all investigated species (-incl.>99% in human plasma). Following intravenous administration of [14C]KRP203, radioactivity was readily distributed to rat and dog tissues with similar volume of distribution (Vss = 8.0 L/kg in rat).

After p.o. dosing (0.5 mg/kg) of [14C]KRP203 to Wistar rats compound-related radioactive material showed a maximum tissue concentration at 8 and 24 hours post-dosing. Highly exposed tissues were liver and to a smaller extent kidney, small intestine, adrenal gland, lungs, spleen and mesenteric lymph nodes. Radioactivity in CNS tissues (cerebrum, cerebellum and medulla oblongata) was lower than in plasma until 24 h post-dose after single administration. The radioactivity was eliminated very slowly from these tissues and [14C]KRP203 related radioactivity was 5 to 6 times higher in the CNS tissues than in plasma 168 h post-dose. After 14-day multiple oral dose of [14C]KRP203 (0.5 mg/kg/day), total radioactivity accumulated more in brain relative to plasma and eliminated much slower.

Investigations of the in vitro metabolite profile of KRP203 showed that in blood from rats, dogs and humans, only phosphorylated KRP203 was detected in all species with the highest formation rate in rat blood followed by dog and human blood. In human hepatocytes, quantitatively the phosphorylated KRP203 was among the more prominent metabolites.

The in vivo ratio of phosphorylated KRP203 to KRP203 in plasma was up to 45 in rat. As the metabolic activities on KRP203 in human blood and hepatocytes were lower than those of other species, the metabolic clearance of KRP203 in man is expected be lower than that in rat and dog. Using in vitro tests, KRP203 did not show relevant potential in inhibiting the activity of human cytochrome P450 isoenzymes. KRP203 and KRP203-phosphate did not show significant induction of CYP3A4 via the human PXR receptor up to concentrations of 6.15 µM and 50 µM, respectively. KRP230 formed low amounts of covalent drug-protein adducts in human hepatocytes in vitro. Due to the low levels detected (68pmol/106 cells at 3 hours) in-vitro adduct formation is unlikely to be associated with clinical relevant findings at low dose levels (e.g. below 10 mg).

Plasma toxicokinetic data in dog following single and multiple doses (4 and 13 weeks), demonstrated that there was a trend for over proportionality between exposure and dose at higher doses for KRP203 and a trend for an under proportionality at all dose levels for KRP203-phosphate. In rat, there was an apparent proportionality between exposure and dose for both KRP203 and KRP203-phosphate after single and multiple administrations. KRP203-phosphate/KRP203 ratios (based on AUC0-24h) of 1.4-6.6 and 10.9-19.1 were observed in dog and rat, respectively. There was no evidence of accumulation for either KRP203 and KRP203-phosphate after repeated dosing (4 and 13 weeks) in dog, but the exposure increased in rat by 2- to 5-fold at the end of the 13-week treatment compared to single dose. No gender difference was noted in exposure for both KRP203 and KRP203-phosphate in rat and in dog.

Acute toxicity studies showed that KRP203 was well tolerated up to high dose levels, producing lethality only at oral doses of 1000 mg/kg in rats or intravenous doses above 50 mg/kg in rats or mice. No mortality was seen following single oral dosing in dogs up to 2000 mg/kg.

Repeat-dose toxicity studies in mice (2 weeks) revealed mortality in this species at doses ≥ 45 mg/kg/day, whilst in rats and dogs (up to 26 and 52-week, respectively) no mortality was seen up to daily doses of 50 mg/kg and 15 mg/kg, respectively. As expected from the pharmacological mode-of-action, effects related to lymphocyte depletion and characteristic changes in lymphoid organs were noted in all species at all dose levels tested in repeat dose toxicity studies. Besides these effects, the main target organs of toxicity were the lungs (mice, rats and dogs), the liver (mice and rats) and kidneys (mice).

The lungs were shown to be a consistent target organ in all animal species tested. At the end of the 13, 26 and 52-week rat and/or dog studies, no histopathological changes were observed at systemic exposures that were similar to those at which the lung effects occurred in the 4-week toxicity studies. Transitory activation of phagocytic cells and increased secretion of soluble markers by these cells is considered a main contributing factor to smooth muscle hypertrophy/hyperplasia.

Effects on the liver (bile duct hyperplasia, fluid retention in bile ducts (rats only), cholangiofibrosis and focal necrosis) were seen in rats and mice, but not in dogs. The rat liver findings did not considerably worsen upon prolongation of treatment from 4 to 26 weeks.

In general, the hepatic effects were mild and did only occur at high dose levels in some studies in combination with general clinical signs (e.g., body weight and food consumption), indicating that the MTD may have been exceeded. In addition, KRP203 does not show.

in silico structural alerts for human liver side effects and genomic profiling of the liver in a 2-week exploratory toxicity study in rats did not indicate any hepatotoxicity-related changes.

The kidney was a target organ of toxicity in mice only and only in a 2-week study at doses ≥ 45 mg/kg. Minimal to severe lesions were noted in both sexes and consisted of tubular vacuolation, dilatation, degeneration, regeneration, hyaline droplets, hyaline casts, increase of mitotic figures, glomerular degeneration with fibrinoid deposits and/or glomerulopathy.

Kidney impairment was most likely the primary cause of death/moribundity seen in this study.

No kidney changes were seen in a 13-week mouse study up to the highest dose level of 20 mg/kg, resulting in a systemic exposure (AUC0-24h) of approximately 7250 ng·h/mL.

There is no evidence for a genotoxic potential of KRP203 or its phosphate from two in vitro genotoxicity tests (bacterial mutation and chromosome aberration), and from an in vivo micronucleus study in mice with KRP203.

Reproductive toxicity studies in rats and rabbits showed a teratogenic potential and effects on the reproductive tract, resulting in embryo lethality and malformations at low doses (no NOAEL in the rat study). No effects on male fertility up to the highest dose of 20 mg/kg were observed in the reproductive toxicology study or from histopathology assessment in the 4- or 13-week toxicity studies up to the highest dose tested.

There is no indication for impaired local tolerability induced by KRP203 in the gastro-intestinal tract when given by oral dosing in rats or dogs up to 26 or 13 weeks, respectively. In the 26- and 52-week dog toxicity studies gastro-intestinal tract disturbance with soft/mucoid stools and diarrhea occurred in males only at the high dose level. KRP203 causes marked eye irritation but is not irritating to rabbit skin and does not have a contact sensitizing potential.

Overall, the preclinical studies completed to date, have not generated data that would preclude the use of this compound in investigational clinical studies.

### Relevant Clinical Research and dosing rationale

Table 1 summarizes the clinical studies with the number of subjects exposed to KRP203.

Overall, 325 HV as well as 66 patients (27 patients with ulcerative colitis, 10 with subacute cutaneous lupus erythematosus, and 29 with Crohn's disease) were included in placebo-controlled trials of KRP203 where KRP203 was well tolerated, demonstrating a favourable safety profile.

A clinical trial with KRP203 in patients undergoing allogeneic HSCT for hematological malignancies (Study KRP203A2105) has been completed.

**Table 1 Summary of completed human studies**

| Study | Population (No. of enrolled subjects) | Subjects exposed to KRP203/ Placebo | Study Title | Dose/Frequency/ Formulation |
|---|---|---|---|---|
| Healthy volunteer studies | | | | |
| CKRP203A 2101 | 136+9 | Part 1:109/27 | A single center, randomized, double-blind, | Single OS: 0.01, |
| | | Part 2: 9/0 | placebo-controlled, parallel group, ascending single oral dose study to assess the safety, tolerability, pharmacokinetics and pharmacodynamics of KRP203 in healthy subjects | 0.03, 0.1, or 0.3mg |
| | | | | Single Caps: 1, 2, 3, 4.5, 6, 9, 15, 25, or 40mg |
| CKRP203A 2102 | 60 | 45/15 | A randomized, parallel, double-blind, placebo-controlled, time-lagged, ascending, multiple-dose, pharmacokinetic, pharmacodynamic, safety and tolerability study of KRP203 in healthy volunteers | Caps: 0.3, 0.6, 1.2, 2.0, or 3.0mg/day |
| CKRP203A 2103 | 56 | 41/15 | A double blind, placebo controlled, parallel group study to investigate the effect of two different dose-titration regimens on the initial negative chronotropic effect of KRP203 in healthy subjects | Caps: 0.3, 0.5, 0.6, 0.9, 1.2, or 2.0mg/day |
| CKRP203C 101* | 40+24 | Part 1: 32/8 Part 2: 18/6 | A single center, randomized, double-blind, placebo-controlled, ascending single and multiple oral dose study to assess safety, pharmacokinetics, and pharmacodynamics of KRP-203 in healthy adults | Caps: Study Part 1 0.3, 1.0, 2.0, or 3.0mg |
| | | | | Caps: Study Part 2: 1.2 or 2.0mg |
| Patient studies | | | | |
| CKRP203A 2201 | 27 | 17/10 | A multi-center, double-blind, placebo controlled, parallel group, proof of concept study to evaluate the efficacy, safety and tolerability of KRP203 in subjects with | Caps: 0.1, 0.4, or 1mg uptitration to 1.2 |
| | | | moderately active refractory ulcerative colitis | mg/daily |
| CKRP203A 2202 | 10 | 8/2 | A multi-center, double-blind, placebocontrolled, proof-of-concept study to evaluate the efficacy and tolerability of KRP203 in patients with active subacute cutaneous lupus erythematosus | Caps: 0.1, 0.4, or 1mg uptitration to 1.2 mg/daily |
| KRP203C2 01 | 29 | 20/9 | KRP-203 Exploratory Study, Phase II | Once daily, oral administration in all regimens |
| | | | A study to evaluate the efficacy and safety in patients with active Crohn's disease | |
| | | | | Dose-titration period (Days 1 to 12) |
| | | | | Days 1 to 4: KRP-203 0.3 mg or placebo |
| | | | | Days 5 to 8: KRP-203 0.6 mg or placebo |
| | | | | Days 9 to 12: KRP-203 0.9 mg or placebo |
| | | | | Fixed-dose period (from Day13 onward) |
| | | | | KRP-203 1.2 mg or placebo was orally |
| | | | | administered once a day. |
| KRP203A2105 | 25 | 23 | Phase Ib study two-part, open-label study to evaluate the PK, safety, tolerability, pharmacokinetics and efficacy (in Part 2 only) in patients undergoing allogeneic HSCT for hematological malignancies | In part I, 10 subjects received 3 mg KRP203 + CSA and in part II, 6 subjects received 1 mg KRP203 + CsA and 7 subjects received 3 mg + Tac |

| | | | | |
|---|---|---|---|---|
| *conducted by Kyorin Pharmaceutical Co., Ltd. in Japanese healthy volunteers | | | | |

### Study CKRP203A2105 in HSCT for hematologic malignancies

Phase Ib study two-part, open-label study to evaluate the PK, safety, tolerability, pharmacokinetics and efficacy (in Part 2 only) in patients undergoing allogeneic HSCT for hematological malignancies.
- Part 1 is a single arm open label study to investigate the safety of 3 mg/day KRP203 added to a standard of care GvHD prophylaxis (cyclosporine A (CsA)/methotrexate) in HSCT patients in 10 patients.
- Part 2 is a randomized two arm open label study to compare the safety, efficacy and PK of 3 mg/day of KRP203 in combination with tacrolimus/methotrexate to 1 mg/day of KRP203 in combination with CsA/methotrexate in 20 patients.

The study population comprised of male or female subjects who were diagnosed with a hematological malignancy and qualified for a standard allogeneic HSCT where HLA matched stem cell source was available. Such malignancies included but were not limited to acute myeloid leukemia (AML), acute lymphocytic leukemia (ALL), myelodysplastic syndrome (MDS, chronic lymphocytic leukemia (CLL), marginal zone and follicular lymphomas, large-cell lymphoma, lymphoblastic, Burkitt's and other high grade lymphomas; mantle-cell lymphoma, lymphoplasmacytic lymphoma; prolymphocytic leukemia or multiple myeloma.

Part 1 comprised of a screening period (Days -50 to -2), Baseline (Day -1), treatment period from Day 1 to Day 111 with KRP203 and a follow-up period up to 365 days (from transplant). Subjects were admitted to the study site during the screening period (approximately 2 to 5 days before Baseline) and received the investigational product 3 mg KRP203 once daily from Day 1 until Day 111 along with cyclosporine A (CsA) given as standard of care (SOC) GvHD prophylaxis. HSCT was performed on Day 11. In addition to the investigational treatment with KRP203, the activities included conditioning, standard GvHD prophylaxis, infusion of the stem cells, pre and post-transplant supportive care and follow-up assessments according to the institutional practices. Subjects remained hospitalized for two to five days after bone marrow engraftment occurred. The subsequent study visits were ambulatory or in-house, if needed depending on the health status of the subject.

Part 2 comprised a screening period (Days -50 to -2), treatment period from Day 1 to Day 111 with KRP203 and follow-up visits. Myeloablative conditioning was performed between Day 2 and Day 10 as per SOC using chemotherapy. Subjects who met the eligibility criteria were randomized to receive either:
- 1 mg KRP203 once daily for 111 days and CsA was given as SOC GvHD prophylaxis, or
- 3 mg KRP203 once daily for 111 days and tacrolimus (Tac) was given as SOC GvHD prophylaxis.

As in Part 1, HSCT was performed on Day 11, all other procedures/visits were same as in Part 1.

The study was terminated early due to strategic considerations. This decision was not related to any safety concern of KRP203. The study was continued for the subjects who signed the informed consent form as of date the decision taken to terminate the study prematurely, with reduced follow-up period of ongoing patients while maintaining appropriate safety follow-up of six months after the last dose of KRP203.

A total of 25 subjects enrolled in the study, of which 23 subjects received study treatment. Two patients did not receive study treatment because of donor not eligible and because of screening failure. In part I, 10 subjects received 3 mg KRP203 + CSA and in part II, 6 subjects received 1 mg KRP203 + CsA and 7 subjects received 3 mg + Tac.

Overall, 14/23 subjects completed the study, including all 10 subjects in part I, and 3/6 subjects in the 1 mg KRP203 + CsA arm and 1/7 subjects plus 3 mg + Tac arm, respectively in part II.

The primary reason for study discontinuation was withdrawal of consent (n=14, 17%), followed by AEs (n=3, 13%) and death (n=2, 9%).

### Most frequent adverse events

### Part 1

Overall, all subjects in Part 1 reported AEs after 30 days of study treatment, nine subjects reported AEs up to 30 days of transplant and seven subjects reported AEs from start of treatment until just before transplant.

The majority of AEs were related to system organ class gastrointestinal disorders (6, 60%) from start of treatment until just before transplant. From transplant up to 30 days of transplant, the most frequent system organ classes affected were gastrointestinal disorder (7, 70%), respiratory, thoracic and mediastinal disorders (4, 40%), vascular disorders (3, 30%), immune system disorders (3, 30%) and nervous system disorders (3, 30%). The most frequent AEs were pertaining to immune system disorders (10, 100%), general disorders and administration site conditions (6, 60%) and infection and infestations (5, 50%) after 30 days of transplant.

From start of treatment until just before transplant, the most frequent AEs in at least two subjects) were vomiting (4, 40%), nausea (2, 20%), and bradycardia (2, 20%). The events of bradycardia (2, 20%), hypertension (1, 10%) and sinus arrest (1, 10%) were suspected related to study treatment by the Investigator.

From transplant up to 30 days of transplant, the most frequent AEs (in at least two subjects) were vomiting (6, 60%), nausea (2, 20%), acute GvHD in intestine (3, 30%), pleural effusion (2, 20%) and capillary leak syndrome (2, 20%). Of these events, the events of pleural effusion (2, 20%) and capillary leak syndrome (2, 20%), were suspected related to study treatment by the Investigator.

After 30 days of transplant, the most frequent AEs (in at least two subjects) were chronic GvHD (7, 70%), chronic GvHD in liver (5, 50%), edema peripheral (4, 40%), acute GvHD in skin, acute GvHD in intestine and chronic GvHD in skin (3, 30% each) followed by diarrhea, nausea, rash, eyelid edema, and chronic GvHD in intestine (2, 20% each). Of these, the event of edema peripheral (2, 20%) was suspected related to study treatment by the Investigator.

### Part 2

All subjects (13 [100%]) randomized in Part 2 in both treatment arms experienced at least one AE from start of treatment until just before transplant, from transplant up to 30 days after transplant and after 30 days of transplant.

From start of treatment until just before transplant, the top three system organ classes affected (in both 1 mg KRP203 + CsA and 3 mg KRP203 + Tac treatment arms) were gastrointestinal disorders, investigations and general disorder and administration site conditions. Up to 30 days after the transplant, the top three system organ classes reported were vascular disorders along with gastrointestinal disorders and investigations. After 30 days of the transplant, the majority (top three) of the AEs were pertaining to system organ class immune system disorders, infections and infestations and general disorders and administration conditions.

The most frequent AEs (at least in two subjects in either 1 mg KRP203 + CsA or 3 mg KRP203 + Tac treatment arm) reported from start of treatment until just before transplant were nausea, vomiting, weight increased, blood creatinine increased and diarrhea. Of these, the event of blood creatinine increased in one subject (1 mg KRP203 + CsA treatment arm) was suspected related to study treatment by the Investigator.

The most frequent AEs (at least in two subjects in both 1 mg KRP203 + CsA or 3 mg KRP203 + Tac treatment arm) reported from transplant up to 30 days of transplant were vomiting, stomatitis, hypertension, thrombocytopenia, back pain, bone pain, C-reactive protein increased, diarrhea and hypomagnesemia. Of these, the event of C-reactive protein in two subjects (1 mg KRP203 + CsA treatment arm) and the event of hypertension (3 mg KRP203 + Tac treatment arm) were suspected related to study treatment by the Investigator.

The most frequent AEs (at least in two subjects in both 1 mg KRP203 + CsA or 3 mg KRP203 + Tac treatment arm) reported after 30 days of transplant were chronic GvHD, edema peripheral, acute GvHD in skin, acute GvHD in intestine, ALT increased, diarrhea and oral candidiasis. Of these, the events of edema peripheral in one subject (1 mg KRP203 + CsA arm) and ALT increased in two subjects (one each in 1 mg KRP203 + CsA and 3 mg KRP203 + Tac treatment arm) were suspected related to study treatment by the Investigator.

### Deaths and other serious or clinically significant adverse events

Overall, seven deaths were reported during the study, one-year and long-term follow-up period. Three subjects died due to Grade 3 or Grade 4 serious AEs of pneumonia aspiration, hepatic failure and Hodgkin's disease.

Both the hepatic failure and Hodgkin's disease were suspected to be related to study treatment by the Investigator. However, an independent pathologist and the data monitoring committee also reviewed the case of hepatic failure and based on the histopathologic findings, concluded that the liver injury was rather caused by alloimmune-mediated hepatitis in line with acute GvHD (and thus, the hepatic failure was unlikely to be related to KRP203). Regarding Hodgkin's disease, the Investigator suspected a relationship between Hodgkin's disease and KRP203. In the Investigator's opinion, KRP203 as an immunosuppression drug may have blunted the GVL effect which could have resulted in relapse of the underlying hematological disease.

### Serious or clinically significant adverse events

### Part 1

One subject (10%) reported a serious AE of vomiting from start of treatment until just before transplant.

Two subjects (20%) reported three serious AEs of acute GvHD, dyspnea and weight increased from transplant up to 30 days after the transplant.

There was a sharp surge in the number of serious AEs reported after 30 days of transplantation. Four subjects (40%) reported SAEs of system organ classes, mainly general disorders and administration site conditions (3, 30%), infections and infestations (2, 20%) and neoplasms benign, malignant and unspecified (incl cysts and polyps) (2, 20%). None of the serious AEs was reported in more than one subject during this time period.

### Part 2

No serious AE was reported from start of treatment until just before transplant. One subject (10%) in the 1 mg KRP203 + CsA treatment arm reported two serious AEs of blepharitis and cystoid macular edema from transplant up to 30 days after transplant.

There was a sharp surge in the number of serious AEs reported after 30 days of transplantation primarily due to events of GvHD. The most frequent serious AEs (at least in two subjects in Part 2) reported were acute GvHD in intestine (3, 23%), diarrhea (2, 15%) and general physical health deterioration (2, 15%).

### Serious adverse events related to study drug

### Part 1

Three serious AEs of cystoid macular edema, dyspnea exertional, and edema peripheral were reported in one subject (10%) after 30 days of transplant were suspected related to study treatment by the Investigator. All these events were of mild severities.

### Part 2

Two serious AEs of blepharitis (Grade 2) and cystoid macular edema (Grade 3) reported in one subject (17%) in the 1 mg KRP203 + CsA treatment arm from transplant up to 30 days after transplant were suspected related to study treatment by the Investigator.

Following 30 days after transplant 4 subjects (31 %) reported five serious AEs that were suspected to be related to the study treatment by the Investigator; 1 (8%) incidence each of febrile bone marrow aplasia (Grade 3), hepatic failure, Hodgkin's disease (Grade 3), macular ischemia (Grade 3) and retinal ischemia (Grade 3).

### Adverse events leading to discontinuation

Overall, two (29%) subjects in the 3 mg KRP203 + CsA arm, two subjects (33%) in the 1 mg KRP203 + CsA arm and one subject (14%) in the 3mg KRP203 + Tac arm had AEs that resulted in discontinuation of study drug. All the events that led to discontinuation of study drug were suspected related to study treatment except two serious events of dyspnea and weight increased in Part 1.

### Benefits of the invention

For patients with high-risk Acute Myeloid Leukemia, an allogeneic HSCT remains the only curative option. This protocol offers these patients the opportunity to receive a promising adjunctive treatment, which improves Overall survival, and/or refractory GVHD-free, Relapse-free Survival (rGRFS) and/or Quality of Life associated to HSCT. The benefit expectations are based on previous Phase IIb CKRP2032105 trial outcomes.

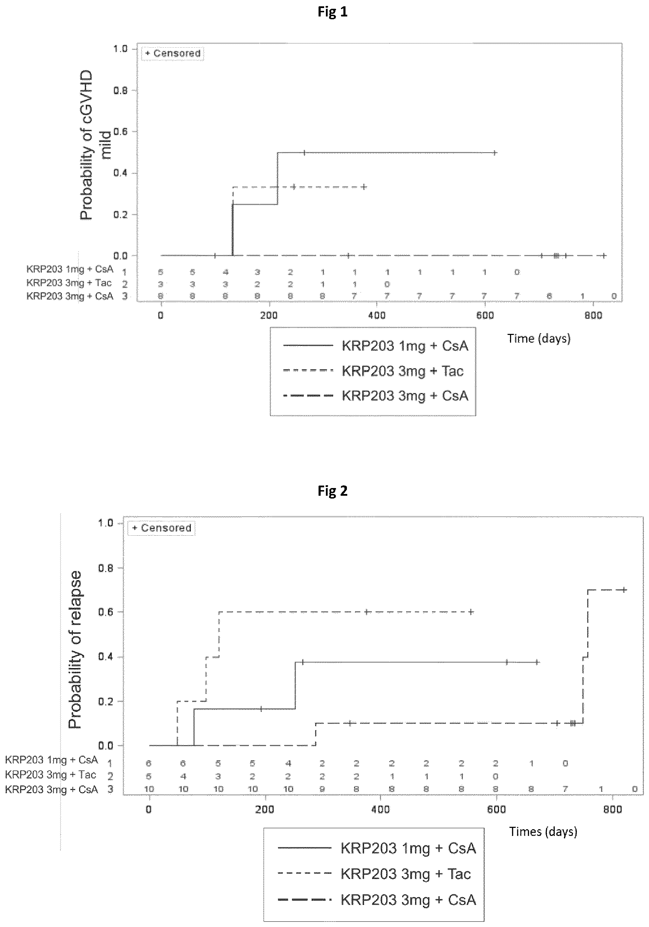

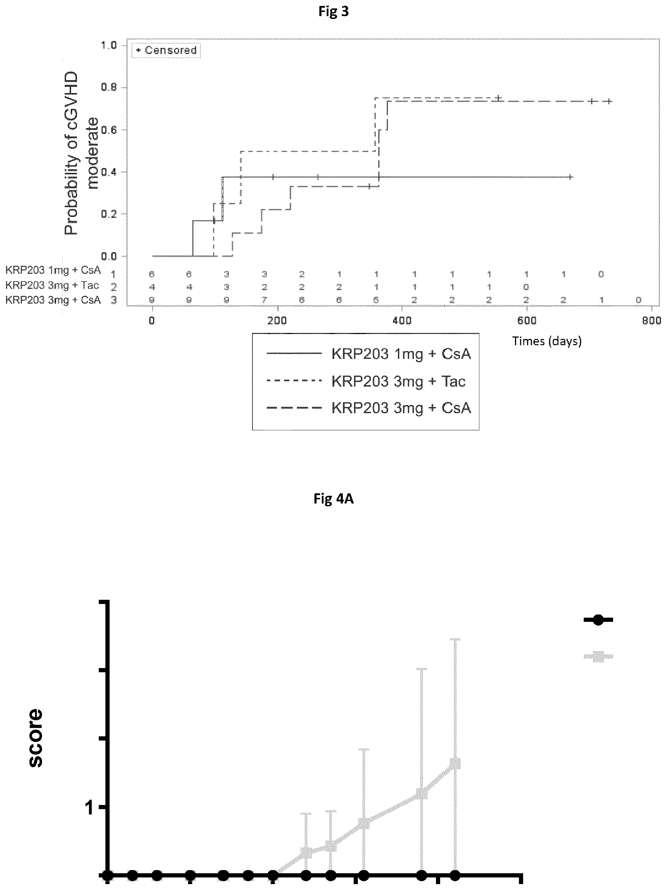

As shown in Fig 1, the incidence of mild chronic GVHD decreases in patients who have received 3 mg KRP203 and Ciclosporin A compared to patients who have received 3 mg KRP203 and Tacrolimus or 1 mg KRP203 and Ciclosporin A. Furthermore, the onset of moderate chronic GVHD is delayed in patients treated with 3 mg KRP203 and Ciclosporin A, compared to the other treatment groups (see Fig 3). The incidence of relapse among patients who received 3 mg KRP203 and Ciclosporin A is lower compared to patients who have received 3 mg KRP203 and Tacrolimus or 1 mg KRP203 and Ciclosporin A. Taken together, these first in human data support the long-term administration of 3 mg KRP203 for a period longer than 111 days for delaying the occurrence of chronic GVHD and preventing relapse of primary disease in AML patients undergoing HSCT.

### Assessment of Potential Risks and Benefits and risk management

The anticipated risks are based on mocravimod clinical experiences and are not expected to outweigh the anticipated benefit. Subjects will be informed thoroughly and clearly regarding the burden of participating in this clinical trial: the frequency of the screening and follow-up visits, the extended hospital stay, as well as any unscheduled visits that might be necessary. Subjects will also be informed about the frequency of blood tests and bone marrow sampling performed and requested to consider all information carefully before deciding to participate in the trial. Subjects will be monitored closely for the occurrences of any significant clinical events and treatment will only continue if it is considered safe and appropriate to do so. The early clinical development of mocravimod demonstrated that its use is generally safe and well tolerated and can justify evaluation of its use in the present Phase IIb trial. The data obtained from the Phase IIb trial also indicated that mocravimod has a potential for therapeutic activity in AML patients.

### TRIAL OBJECTIVES AND ENDPOINTS

### Objectives

The objective of this study is to compare the safety and efficacy of mocravimod vs control as adjunctive treatment to allogeneic HSCT in subjects with Acute Myeloid Leukemia. An additional objective of the study is to compare the effect of the two treatments on quality of life.

### Primary objectives

To assess mocravimod effect on relapse and GVHD as adjunctive treatment to HSCT in order to improve the safety and efficacy of HSCT. HSCT is performed with HLA-matched sibling or unrelated donor, in subjects with AML in first (CR1) or subsequent Complete Morphologic Remission (CR2).

### Secondary objectives

To assess mocravimod effect on overall survival at 2 years, disease progression free survival at 2 years, and Quality of Life

### Primary Endpoint

The primary endpoint of the study is Refractory GVHD-free, relapse-free survival (rGRFS).

rGRFS is defined as time from randomization until whatever occurs first:
- grade III/IV acute graft-versus-host disease (GVHD) refractory to at least 2 lines of treatment,
- extensive chronic GVHD refractory to systemic immunosuppressive treatment,
- disease relapse,
- death.

This composite endpoint captures both safety and efficacy. Composite endpoints such as GRFS acknowledge that rates of various critical events are clinically relevant when testing new therapies.

rGRFS is calculated similarly to conventional GRFS treating grade III to IV acute GVHD, chronic GVHD requiring systemic treatment, and disease relapse/progression as events, except that GVHD that resolved and do not require systemic treatment at the last evaluation is excluded as an event in rGRFS.

### Secondary Endpoints

The key secondary endpoint of the study is Overall survival.

Overall survival (OS) is defined as the time from randomization until death from any cause. Other secondary endpoints for efficacy evaluation are Progression-free survival (PFS) and Cumulative Incidence of Relapse. PFS is defined as the time from randomization until disease relapse or death from any cause. Cumulative Incidence of Relapse is defined as incidence of morphologic or clinical relapse from the time of random isation.

Secondary endpoints for safety evaluation are:
- Incidence of macular oedema
- Incidence of Liver function abnormality leading to dose reductions/treatment discontinuation
- Incidence of severe bradycardia
- Incidence of Post-Transplant Lymphoproliferative Disease
- Incidence of viral, bacterial and fungal severe infectious complications including EBV/CMV and incidence of pre-emptive, curative use of anti-viral therapy
- Cumulative incidence of NCI CTCAE grade 3-5 adverse events

Secondary endpoints for exploratory purposes are:
- Time to engraftment.
- Time to acute GvHD
- Cumulative incidence of grade II-IV and grade III-IV acute GVHD at one year
- Time to chronic GvHD
- Cumulative incidence of moderate and severe chronic GVHD at one year
- Cumulative incidence of chronic GVHD requiring systemic immunosuppressive treatment at 1 year
- Antibodies titers Response to Flu Vaccination
- Median duration and Average dose of GvHD prophylaxis by ciclosporin A in mg/kg/day over the first year of treatment
- Incidence of concomitant therapy and Cumulative dose of corticosteroids during the first year of treatment
- Incidence of concomitant therapy and Cumulative dose of Ruxolitinib over the first year of treatment
- Cumulative incidence of NCI CTCAE grade 2-5 and grade 3-5 infections

### Other Endpoints

Quality of Life evaluation is conducted via questionnaire with primary endpoint at 12 months.

The Foundation for the Accreditation of Cellular Therapy- Bone Marrow Transplantation questionnaire (FACT-BMT, version 4), the Short Form 36-item health survey (SF-36, version 2), and the MD Anderson Symptom Inventory (MDASI) will be scored at Screening, Month 3, Month 6, Month 12, and Month 24, provided that validated translations in local language are available.

### STUDY DESIGN.

### Overall Design

This is a Phase IIb randomized controlled multicentre open-label study comparing two parallel groups. After signing informed consent, a total of about 160 subjects will be randomized in a 1:1 fashion to receive either mocravimod in combination with GVHD prophylaxis based on CsA and MTX in the active arm or Standard of Care without mocravimod in the control arm.

Randomization will use minimization to balance treatment groups with respect to Complete Remission status (CR1 vs CR2) and center. A stochastic treatment allocation procedure will be used so that the treatment assignment is random for all subjects entered in the study.

Subjects randomized in the mocravimod group will receive 3 capsules dosed 1mg once a day starting at Day-11 before the HSCT for a duration of 12 months or until all immunosuppressants have been tapered and weaning is completed for at least 4 weeks.

Subjects who would have completed the 1^{st} year on treatment will be proposed to continue receiving study treatment for an additional year in an open label setting.

All patients will be followed up for 24 months post HSCT.

The study is designed to confirm the results of a non comparative Phase IIa clinical trial in hematologic malignancies and to compare the outcomes of patients receiving mocravimod as adjunctive therapy to HSCT to a control group of patients receiving a similar setting without mocravimod. The study aims at showing superiority in the mocravimod group compared to the control group.

### STUDY POPULATION

### Trial population

Male or female subjects aged 18-75 years with AML in Complete remission (CR1, CR2) who are eligible for a HSCT collected from peripheral blood of sibling or matched unrelated donor. In total, 160 patients are planned to be randomized and a number of 60 sites in Europe, Israel and North America are planned to enrol participants in the study.

### Inclusion Criteria

Each patient must meet the following criteria to be enrolled in this study:
- Subjects with a diagnosis of AML and related precursor neoplasms according to the WHO 2016 classification (excluding acute promyelocytic leukaemia), including secondary AML after an antecedent haematological disease (e.g. myelodysplastic syndrome) and therapy-related AML
- Subject is planned to undergo allogeneic HSCT from fully matched sibling donor or Unrelated Donor with an 8/8 match at HLA-A, -B, -C and DRB1 at high resolution by DNA-based typing, whereas Stem cell source is mobilized peripheral blood collected via apheresis by a compatible Donor. The minimum recommended CD34+ cell dose in the graft should be 2 × 106/kg, and the recommended target dose should be 5 × 106/kg.
- Subjects with AML in first cytomorphological remission (CR1) and ELN adverse classification or subjects in subsequent cytomorphological remission (CR2).
   CR is defined as leukaemia clearance (< 5% marrow blasts and no circulating peripheral blasts) in conjunction with normal values for absolute neutrophil count and platelet count, no extramedullary manifestation of leukaemia and no need for repeat blood transfusions.
   CRi is defined as meeting all CMR criteria except for an absolute neutrophil count < 1,000/µL or platelet count < 100,000/µL.
- Life expectancy ≥ 6 months at screening
- Karnofsky Performance Status (KPS) ≥ 70%
- Male or female, age ≥ 18 years and ≤ 75 years _
- Subjects ≥ 65 years must have a Sorror Score ≤ 3
- Able and willing to provide written informed consent and comply with the trial protocol and procedures
- For females of childbearing potential who are sexually active and males who have sexual contact with a female of childbearing potential: willingness to use reliable methods of contraception (oral contraceptives, intrauterine device, hormone implants, contraceptive injection or abstinence) during study participation.
   A female is considered of childbearing potential following menarche and until becoming post-menopausal unless permanently sterile. Women are considered post-menopausal and not of child bearing potential if they have had 12 months of natural (spontaneous) amenorrhea with an appropriate clinical profile (e.g. age appropriate, history of vasomotor symptoms) or have had surgical bilateral oophorectomy (with or without hysterectomy) or tubal ligation at least six weeks ago. In the case of oophorectomy alone, only when the reproductive status of the woman has been confirmed by follow up hormone level assessment, she is considered not of child bearing potential.
- Affiliation to a national health insurance scheme (according to applicable local requirements)

### Exclusion Criteria

Patients who meet any of the following criteria will be excluded from the study:
- Subjects having received prior allogeneic HSCT or recipient of a solid organ transplant.
- Subjects with acute promyelocytic leukaemia
- Diagnosis of any previous or concomitant malignancy is an exclusion criterion, except when the subject completed treatment (chemotherapy and/or surgery and/or radiotherapy) with curative intent for this malignancy at least 6 months prior to enrolment
- Blast crisis of chronic myeloid leukaemia
- Concurrent severe and/or uncontrolled medical condition including
   ∘ Clinically significant pulmonary fibrosis
   ∘ Tuberculosis, except for history of successfully treated tuberculosis or history of prophylactic treatment after positive PPD skin reaction
   ∘ Patients receiving chronic (daily) therapies for asthma
   ∘ Patients with any other types of clinically significant bronchoconstructive disease
   ∘ Uncontrolled diabetes mellitus as assessed by the investigator or diabetes complicated with organ involvement such as diabetic nephropathy or retinopathy.
   ∘ Uncontrolled seizure disorder
   ∘ Uncontrolled depression or history of suicide attempts/ideation
- Cardiac dysfunction as defined by:
   ∘ Myocardial infarction within the last 3 months of trial entry, or
   ∘ Reduced left ventricular function with an ejection fraction < 40% as measured by multi-gated acquisition (MUGA) scan or echocardiogram (echo) within 28 days before screening, or
   ∘ History or presence of stable or unstable ischemic heart disease (IHD), myocarditis, or cardiomyopathy, or
   ∘ New York Heart Association (NYHA) Class II-IV congestive heart failure, or
   ∘ Unstable cardiac arrhythmias including history of or presence of symptomatic bradycardia,
   ∘ Resting heart rate (physical exam or 12 lead ECG) <60 bpm
   ∘ History or current diagnose of ECG abnormalities indicating significant risk of safety such as: Concomitant clinically significant cardiac arrhythmias, e.g. sustained ventricular tachycardia, presence of a clinically relevant impairment of cardiac conduction including sick sinus syndrome, or sino-atrial heart block, clinically significant AV block, bundle branch block or resting QTc (Fridericia preferred, but Bazzet acceptable) > 450 msec for males and > 470 msec for females at Screening or Baseline ECG
   ∘ History or presence of symptomatic arrhythmia or arrhythmia requiring treatment or being otherwise of clinical significance
   ∘ Uncontrolled arterial hypertension; if controlled, the medication must be stable for three (3) months prior to baseline visit
   ∘ Treatment with medication that impairs cardiac conduction (e.g., beta blockers, verapamil-type and diltiazem-type calcium channel blockers, or cardiac glycosides)
   ∘ Concomitant use of agents known to prolong the QT interval unless they can be permanently discontinued for the duration of the study
   ∘ Treatment with quinidine
   ∘ History of syncope of suspected cardiac origin
   ∘ History of familial long QT syndrome or known family history of Torsades de Pointes
- Pulmonary dysfunction as defined by oxygen saturation < 90% on room air. Pulmonary function test (PFT) is required only in the case of symptomatic or prior known impairments within 28 days before screening - with pulmonary function < 50% corrected diffusing capacity of the lung for carbon monoxide (DLCO) and forced expiratory volume in 1 second (FEV1)
- Significant liver disease or liver injury or known history of alcohol abuse, chronic liver or biliary disease
- Hepatic dysfunction as defined by AST and/or ALT> 5 × ULN
- Renal dysfunction with Serum creatinine > 2.0 mg/dL (176 µmol/L)
- Vaccination with live, attenuated vaccines within 4 weeks prior to screening
- Immunosuppressive drugs for concomitant disease. Subjects must be able to be off prednisone or other immunosuppressive medications for at least 3 days prior to the start of treatment of the study
- History of stroke or intracranial haemorrhage within 6 months prior to screening
- Active infections (viral, bacterial or fungal) that requires specific therapy. Acute anti-infectious therapy must have been completed within 14 days prior to trial treatment
- History of human immunodeficiency virus (HIV) or active infection with hepatitis B virus (HBV) or hepatitis C virus (HCV) defined as a positive HIV antibody, Hepatitis B surface antigen or Hepatitis C
- History of a previous malignancy, treated or untreated, within the past 5 years, regardless of whether there is evidence of local recurrence or metastases, with the exceptions of localized basal cell carcinoma of the skin or in-situ cervical cancer
- Current concomitant chemotherapy, radiation therapy, or immunotherapy
- Major surgery within 4 weeks prior to screening or a major wound that has not fully healed
- Positive pregnancy test or breastfeeding of subject (women of childbearing age only)
- Estimated probability of surviving less than 3 months
- Known allergy to any of the components of mocravimod (e.g., excipient)
- Any contraindication for GVHD prophylaxis with ciclosporin A, or Methotrexate
- Diagnosis of macular edema during screening. Patients with a history of macular edema will be allowed to enter the study provided they do not have macular edema at the ophthalmic examination at screening
- Participation in another interventional clinical trial within 4 weeks prior to trial enrolment or participation in a concomitant interventional clinical trial
- Any other condition that, in the opinion of the investigator, makes the patient or donor ineligible for the study
- Subjects under legal protection measure (guardianship, trusteeship or safeguard of justice) and/or inability or unwillingness to comply with the requirements and procedures of this trial

### STUDY INTERVENTION

*Identity of the IMP and Accompanying non-investigational medicinal products (NIMPs).*

The IMP is mocravimod.

### IMP active substance:

### 2-amino-2-[2-[2-chloro-4-[[3-(phenylmethoxy)phenyl]thio]phenyl]ethyl]-1,3-propanediol hydrochloride

### Study Intervention Description.

All Patients in the trial will receive mocravimod with a regimen of a daily dose of 3 capsules by oral route starting at Day -11 for 1 year after HSCT.

NIMPS are products used in HSCT for conditioning, GVHD prophylaxis and infectious complications prophylaxis and are commercially available for human use under various brand names.

### Dosing and Administration

Mocravimod is formulated as a hard gelatin capsule and is an immediate release dosage form for oral administration. The hard gelatin capsules contain white to off-white powder in a pink opaque (Swedish orange) capsule, size #4.

The hard gelatin capsule is administered at a regimen of a daily dose of 3 capsules by oral route starting at Day -11 before and for 1 year after HSCT.

### Preparation/ Handling/ Storage/ Accountability.

In this trial, traceability of mocravimod is defined as the ability to locate and identify each individual unit of mocravimod during any step from storage, to distribution to the subject or disposal. This also implies the ability to identify the subject at the trial site, and the ability to locate and identify all relevant data relating to products and materials coming into contact with the mocravimod.

Accountability of the mocravimod will be documented at the trial centres. Detailed instructions on the accountability process are provided in the Pharmacy Manual.

In accordance with international guidelines, the sponsor will maintain records of all products dispensed worldwide. All waste materials that have been used for the preparation of mocravimod at the manufacturing facility and at the trial site will be destroyed according to local regulations on an ongoing basis.

Traceability of subjects is ensured by documenting the identity of subjects including their subject number at the trial site. Subject identities are protected and are only identified by code numbers that can be linked at the trial site to their full identity. Traceability of mocravimod batches from manufacturing until shipping to clinical sites is ensured by the procedures of the Contract Manufacturing Organization. Traceability of the mocravimod at the trial site is ensured by maintaining the mocravimod accountability log.

### Formulation, Appearance, Packaging, and Labelling.

Mocravimod is prepared as hard gelatin capsules for oral administration as immediate release dosage form. In addition to the drug substance, the capsules contain the following standard pharmacopoeial excipients: Mannitol; microcrystalline cellulose/cellulose, microcrystalline; sodium starch glycolate; magnesium stearate and colloidal silicon dioxide/silica; colloidal anhydrous.

The hard gelatin capsules contain white to off-white powder in a pink opaque (Swedish orange) capsule, size #4.

The packaging is HDPE bottles,each bottle contains 30 capsules, i.e. treatment for 10 days.

### Product Storage and Stability.

Available stability data demonstrate that Mocravimod 1 mg hard gelatin capsules stored at 5°C/ambient RH is stable for 48 months when packaged in HDPE bottles and is stable for 24 months when packaged in PVC/PCTFE/PVC (Aclar) blister packs.

Additionally, Mocravimod 1 mg hard gelatin capsules are stable for 36 months in HDPE bottles following storage at 25°C/60% RH. The drug product is also stable for 6 months when packaged in both HDPE bottles at 40°C/75% RH.

### Prior and Concomitant Medication and Therapy

All concomitant prescription medications used during the study, in addition to the study intervention, will be considered concomitant. The following concomitant medications should be recorded in the electronic case report form (eCRF):
- Medication for the treatment of GVHD
- Medication for the treatment of infections, including prophylactic or pre-emptive use of anti-infective medication
- Medication for the treatment of disease relapse
- Medication for the treatment of other reported AEs
- Post-transplant prophylactic use of immunosuppressive medication except Ciclosporin A and Methotrexate in the mocravimod arm.
- Medication for prophylaxis against conditioning-induced AEs
- Additional hematopoietic stem cell transplantation
- Donor Lymphocyte infusions
- Hematopoietic growth factors
- Vaccinations

Post-transplant immunosuppressive therapy by corticosteroids in the absence of GVHD should be avoided unless medically indicated.

Subjects in the mocravimod arm will receive an immunosuppressive regimen for GVHD prophylaxis during approx. 6 months based on Ciclosporin A and Methotrexate. Tapering and weaning will be conducted according to the clinical site institutional practice.

Post-transplant prophylaxis against GVHD other than Ciclosporin A and Methotrexate will not be permitted in the mocravimod arm.

To prevent infections with cytomegalovirus (CMV), patients who are CMV positive or have a CMV positive donor can be given prophylactic treatment including ganciclovir, valganciclovir, letermovir and foscarnet, and all patients will be subject to regular quantitative PCR monitoring.

The following dosing schedule is recommended in case of prophylactic CMV treatment (Day 0 is the day of HSCT):
- From Day -9 through Day -2: ganciclovir 5 mg/kg IV q12h.
- From Day 4 through Day 20: foscarnet 90 mg/kg IV q24h.
- From Day 21 until Day 100: valganciclovir 900 mg PO daily 5 days a week, or ganciclovir 5 mg/kg IV q24h 5 days a week.
- Dosage is to be adjusted depending on renal function.

If quantified viral DNA levels exceed the institutional threshold for treatment of CMV (as established for each study center before participation in the study), patients should be treated pre-emptively with ganciclovir (recommended dose 5 mg/kg IV q12h) or valganciclovir (recommended dose 900 mg PO twice daily).

To prevent infections with Epstein-Barr virus (EBV), patients who are EBV positive or have an EBV positive donor will be subject to regular quantitative PCR monitoring followed by adequate (pre-emptive) treatment if indicated. If quantified viral DNA levels exceed the institutional threshold for treatment of EBV (as established for each study center before participation in the study), patients should be treated with rituximab. It is also recommended to start rituximab if a patient who was EBV positive in the past, demonstrates enlarged lymph nodes, even if PCR for EBV is low or negative.

The following schedule is recommended:
- Immediately after the rise in EBV DNA is detected, rituximab (anti-CD20) 375 mg/m2 IV is started once weekly, until PCR for EBV becomes negative.
- If PTLD is suspected on the basis of clinical symptoms, CT scans of thorax, abdomen and pelvis, as well as bone marrow aspiration and biopsy, and -when possible- lymph node biopsy should be conducted. If results of the CT scan, bone marrow examinations, and lymph nodes demonstrate PTLD, rituximab is repeated weekly for at least 2 weeks.

Other viral, fungal and bacterial prophylaxis will be given according local, institutional guidelines.

Given that KRP203 is anticipated to be metabolized in the liver by CYP3A4 and 2D6 only to a limited extent (based on in vitro data), and the lack of effect of KRP203 and KRP203-P on cytochromes P450 enzymes and transporters, the risk of a PK DDI between KRP203 and CsA or TAC or other similar co-medications is considered low. The weak inhibitory effect of ciclosporin on hepatic CYP3A4 is unlikely to relevantly impact the PK of KRP203. As a result, even if Drug-Drug Intractions (DDIs) cannot be excluded, it is more likely to affect the PK of CsA or Tacrolimus (TAC) than that of KRP203, notably because of other co-medications that can alter the PK of CsA or TAC.

Nonetheless, for conservative reasons, caution has to be exerted when combining KRP203 with CsA or TAC. In trials where KRP203 is co-administered with CsA or TAC (e.g. in study CKRP203A2105), the PK properties of KRP203 and KRP203-P as well as concentrations of CsA and TAC were continuously assessed to mitigate the DDI risk.

### INVESTIGATIONAL PLAN

### Trial Phases

The trial for each subject will consist of 4 phases:
- Informed consent
- Screening and enrolment phase
- Treatment phase
- Follow-up phase, with an EOT visit at 24 months post-HSCT

### Treatment Phase

The treatment phase corresponds to the start of the IMP regimen at Day -11 before HSCT in the mocravimod arm until the last_IMP intake. The treatment phase in the control arm starts the day of the HSCT until1 year post HSCT.

Enrolled subjects in the mocravimod arm will take 3 capsules on a daily basis for 1 year after HSCT. A window of 7 additional days is tolerated in case the HSCT cannot happen as planned after conditioning for medical reasons (e.g. infections, AE monitoring).

On Day 0, subjects will receive HSCT. The last IMP intake corresponds to the end of treatment in the mocravimod arm.

The treatment phase will start in an outpatient setting followed rapidly by an inpatient (hospitalised) setting according to institutional practices for conditioning and HSCT. Subjects will be hospitalised from the start of the conditioning regimen until being discharged upon Investigator's decision. Therefore, the treatment phase will be starting on Day -11 prior to HSCT and before conditioning to be administered on Days -6, -5, - 4, and -3 prior to HSCT.

### Efficacy Assessments.

### Standard of Care Study Procedures.

Eligible subjects are randomized to either the mocravimod group or the control group. Subjects in both groups will undergo a conditioning regimen followed by HSCT as part of regular standard of clinical care. In this section the conditioning regimens and HSCT procedures are described.

### Conditioning Regimens

All patients will undergo a conditioning regimen before HSCT.

One of the following conditioning regimens is to be administered (day numbers are relative to the day of HSCT). Scheduled deviations from these conditioning regimens are to be discussed between the principal investigator and the Medical Monitor of the Study.
- Fludarabine; 30 mg/m2 IV once daily for 5 days on Day -8 to -4 (150 mg/m2)
- Thiotepa; 5 mg/kg IV twice daily for 1 day on Day -7 (10 mg/kg)
- Melphalan; 60 mg/m2 IV once daily for 2 days on Day -2 and -1 (120 mg/m2)

Dose variations in the conditioning regimen are allowed to accommodate for patient's condition and/or local practice:
According to institutional practices, the 3 following conditioning regimens are allowed next to the standard regimen described hereabove.
- Melphalan can be substituted by busulfan.
- Thiotepa can be substituted by cyclophosphamide
   1. Busulfan + fludarabine + cyclophosphamide:
      - Busulfan 110 mg/m2 IV on Day -7 to -4 (440 mg/m2)
      - Fludarabine 25 mg/m2 for 5 days from Day -6 to -2 (125 mg/m2)
      - Cyclophosphamide 14.5 mg/kg IV on Day -3 and -2 (29 mg/kg)
   2. Busulfan + fludarabine + thiotepa:
      - Busulfan 3.2 mg/kg/day IV for 3 days on Day -5 to -3 (9.6 mg/kg)
      - Fludarabine 50 mg/m2/day IV for 3 days on Day -5 to -3 (150 mg/m2)
      - Thiotepa 5 mg/kg/day IV for 2 days on Day -7 and -6 (10 mg/kg)
   3. Melphalan + thiotepa + fludarabine:
      - Melphalan 100 or 140 mg/m2 IV on Day -6
      - Fludarabine 40 mg/m2 IV for 4 days on Days -5 to -2 (160 mg/m2)
      - Thiotepa 5-10 mg/kg IV on Day -7
(day numbers are relative to the day of HSCT)

### Disease Assessment

rGRFS is a primary endpoint for efficacy assessment. Progression-Free Survival and Cumulative incidence of Relapse are secondary endpoints for efficacy assessment.

The status of the AML haematologic disease will be assessed at the local laboratory by BM biopsy or aspirate for morphology at the Screening Visit, Month 3, Month 6 and Month 12 Treatment Visits, Month 18 and 24 Follow-up Visits, unless relapse has already been confirmed. AML disease assessment will also be performed in case of suspected relapse.

A morphologic relapse is defined as *morphological* evidence of leukemia in Bone Marrow, or at other extra-medullary sites.

Details of relapse or disease progression will be recorded on the Relapse/Disease Progression assessment page of the eCRF.

In case a bone marrow biopsy cannot be obtained, it may be replaced by a bone marrow aspirate. If a bone marrow aspirate and/or biopsy had already been obtained within 6 weeks prior to a scheduled visit, the assessment does not need to be repeated. In addition, in case of suspected relapse post HSCT, chimerism will be assessed to support the diagnosis.

### Graft-Versus-Host Disease Assessment

GVHD events will be diagnosed and classified based on the NIH criteria (Filipovich et al., Biol Blood Marrow Transplant. 2005 Dec;11(12):945-56; Jagasia et al., Biol Blood Marrow Transplant 2015 Mar;21(3):389-401.e1) as summarized in Table 3.

**Table 3 Categories of acute and chronic GVHD**

| **Category** | **Time of symptoms after HSCT** | **Presence of acute GVHD features** | **Presence of chronic GVHD features** |
|---|---|---|---|
| **Acute GVHD** | | | |
| Classic acute GVHD | ≤ 100 days | Yes | No |
| Persistent, recurrent, or late-onset acute GVHD | > 100 days | Yes | No |

| **Chronic GVHD** | | | |
|---|---|---|---|
| Classic chronic GVHD | No time limit | No | Yes |
| Overlap syndrome | No time limit | Yes | Yes |

Acute GVHD will be graded according to the modified Glucksberg scale (Harris et al., Biol Blood Marrow Transplant 2016 Jan;22(1):4-10) (see below). Chronic GVHD will be graded according to NIH criteria (Filipovich et al., Biol Blood Marrow Transplant. 2005 Dec;11(12):945-56; Jagasia et al., Biol Blood Marrow Transplant 2015 Mar;21(3):389-401.e1) (see below). Whenever deemed possible, tissue biopsies will be obtained to confirm the diagnosis of GVHD diagnosis and to assess its severity. However, acute and chronic GVHD remain clinical diagnoses, which are considered present when diagnosed and treated, even in the absence of biopsy confirmation.

Details of all GVHD events will be recorded on the GVHD AE page of the eCRF, including start date, stop date, NCI CTCAE severity grade, GVHD grade, outcome, and action taken. For chronic GVHD events it will also be recorded whether they require systemic immunosuppressive treatment. The start date of the GVHD event is defined as the date of initiation of GVHD treatment or the date of biopsy confirmation of GVHD, whichever is earlier. Resolution of the GVHD event is defined as complete response, i.e. resolution of all signs and symptoms.

### Mortality

After subject death, the following information must be recorded in the eCRF:
- Date of death
- Cause of death (specification)
- Investigator classification of cause of death into:
- Disease relapse
- Disease progression
- Transplant-related mortality (TRM) defined as death due to causes other than disease relapse or disease progression

All death cases will be subject to independent adjudication.

### Quality of Life.

The Foundation for the Accreditation of Cellular Therapy- Bone Marrow Transplantation questionnaire (FACT-BMT, version 4 and the MD Anderson Symptom Inventory (MDASI) will be scored at Screening, Day+14, Day + 28, Day + 60, Month 3, Month 6, Month 12, and Month 24, provided that validated translations in local language are available.

### STATISTICAL CONSIDERATIONS

### Statistical Hypotheses

A separate statistical analysis plan (SAP), which will provide the technical details of the statistical analysis outlined below, will be prepared and approved before trial data analysis. Any deviations from these analyses will be justified in the clinical study report.

For the primary endpoint rGRFS, interest focuses on whether the hazard rate in the active group significantly differs from that in the control group. Hence the null and alternative hypotheses are:
Ho Hazard ratio mocravimod/control = 1
H₁ Hazard ratio mocravimod/control 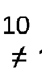 1

### Sample Size Determination

On the primary endpoint, rGRFS, it is anticipated that the 12-month rGRFS will be at least 60% in the mocravimod arm versus 40% at most in the control group (a hazard ratio equal to 0.55). A power of 0.8 will be required for this magnitude of treatment effect, and the two-tailed significance level will be set at 0.05.

Based on these assumptions, the sample size required is 140 subjects, and 116 events are required for the final analysis of rGRFS to take place. To compensate for potential drop off and losses to follow-up at one year, a total of 160 subjects will be enrolled into this study.

### Populations for Analyses

All enrolled patients who were randomized will be included in the analyses. The following analysis datasets will be discerned:

### Intention-to-treat (ITT) population

The ITT population consists of all randomized patients. The ITT population is the primary efficacy dataset for the primary and secondary endpoints.

### Modified intention-to-treat (MITT) population

The MITT population consists of all randomized patients who received an HSCT and mocravimod (active group) or at least one dose of GVHD prophylaxis (control group).

### Per protocol (PP) population

Prior to locking the database, the sponsor will define a PP population as a subset of the MITT population of patients without major protocol deviations. The PP population will be used to (1) shed light on potential reasons why the primary analysis of the ITT population may have failed to reach significance, or (2) investigate how major protocol deviations may have had an impact on the magnitude of the treatment benefit. The definition of "major" protocol deviations will be agreed upon, and all cases of such major deviations adjudicated prior to database lock, by a team blinded to treatment allocation.

### Safety population

The safety population consists of all patients who received an HSCT.

### Statistical Analyses

### General Approach

For time to event endpoints, standard statistical methods will be used, including Kaplan-Meier curves, the logrank test and the Cox proportional hazards model. The assumption of proportional hazards will be tested. All analyses will be stratified by Complete Morphologic Remission status (CR1 vs CR2).

For binary endpoints, treatment groups will be compared through the Cochran-Mantel-Haenszel test, stratified by CR1 vs CR2. The impact of prognostic factors upon these endpoints will be assessed through logistic regression models.

Unless specified otherwise, descriptive statistics (cumulative incidences or proportion of patients free of the event of interest) will be presented for time to event endpoints at 3 months, 6 months, 12 months and 24 months. The 95% confidence intervals will be calculated for the treatment contrast.

The following missing data handling strategies for primary efficacy endpoint and secondary endpoints will be used:
- There will be no imputation of missing values.
- There will be no "administrative" censoring for any reason, i.e. all events will be used in the analyses. The only censored observations will be for patients who have not experienced the event of interest. For instance, patients who died due to disease relapse or disease progression will be censored in the analysis of time to TRM.

### Analysis of the Primary Efficacy Endpoint

The primary endpoint of the study is rGRFS, treated as a time to event variable. The primary approach for the primary efficacy analysis will be a randomization test to reflect the treatment allocation procedure. The randomization test will be based on a large number of simulated trials, say S, in which the treatments are re-allocated to the patients actually entered in the study (in the same order of entry) using the same minimization algorithm. Each simulated trial uses a different seed for the random number generator. The test statistic, say Δ, is calculated for the actual trial ( Δobs) and for each simulated trial (Δi , i = 1, ... , S). The significance probability (P-value) of the randomization test is calculated directly from the empirical distribution of Δ under the null hypothesis. Let s be the number of ΔI_'s for which |Δi| ≥ |Δobs|. The two-sided randomization P-value is equal to s/S. The number of simulated trials S will be chosen to ensure that the P-value is estimated correctly to the second significant digit (Buyse M., Stat Med. 2010 Dec 30;29(30):3245-57).

### Analysis of the Secondary Endpoints

Secondary endpoints will be tested for significance using a Hochberg procedure. The procedure works as follows: let p1, p2, p3 and p4 be the p-values of the tests for each of the four secondary endpoints, with p1 ≥ p2 ≥ p3 ≥ p4. Let α be the significance level (e.g. 5%) appropriate for the analysis. If p1 ≤ α, all four secondary endpoints show a significant treatment effect. If p1 > α and p2 ≤ α/2, the endpoints corresponding to p2, p3 and p4 show a significant treatment effect. If p1 > α and p2 > α/2 and p3 ≤ α/3, the endpoints corresponding to p3 and p4 show a significant treatment effect. If p1 > α and p2 > α/2 and p3 > α/3 and p4 ≤ α/4, the endpoint corresponding to p4 shows a significant treatment effect. If p1 > α and p2 > α/2 and p3 > α/3 and p4 > α/4, no secondary endpoint shows a significant treatment effect.

### LIST OF ABBREVIATIONS AND DEFINITIONS OF TERMS

World Health Organization (WHO) 2016 classification of AML and related precursor neoplasms (Arber DA, Semin Hematol. 2019 Apr;56(2):90-95).

### Table A. WHO classification of AML and related neoplasms

### Acute myeloid leukaemia (AML) and related neoplasms

AML with recurrent genetic abnormalities
   AML with t(8;21)(q22;q22.1);*RUNX1-RUNX1T1*
   AML with inv(16)(p13.1q22) or t(16;16)(p13.1q22);*CBFB-MYH11*
   APL with *PML-RARA*
   AML with t(9;11)(p21.3;q23.3);*MLLT3-KMT2A*
   AML with t(6;9)(p23;q34.1);*DEK-NUP214*
   AML with inv(3)(q21.3q26.2) or t(3;3)(q21.3;q26.2); *GATA2, MECOM*
   AML (megakaryoblastic) with t(1;22)(p13.3;q13.3);*RBM15-MKL1*
   *Provisional entity: AML with BCR-ABL1*
   AML with mutated *NPM1*
   AML with biallelic mutations of *CEBPA*
   *Provisional entity: AML with mutated RUNX1*
AML with myelodysplasia-related changes
Therapy-related myeloid neoplasms
AML, NOS
   AML with minimal differentiation
   AML without maturation
   AML with maturation
   Acute myelomonocytic leukaemia
   Acute monoblastic/monocytic leukaemia
   Pure erythroid leukaemia
   Acute megakaryoblastic leukaemia
   Acute basophilic leukaemia
   Acute panmyelosis with myelofibrosis
Myeloid sarcoma
Myeloid proliferations related to Down syndrome
   Transient abnormal myelopoiesis (TAM)
   Myeloid leukaemia associated with Down syndrome

### MISCELLANEOUS

### Disease Risk Index (DRI)

The DRI is in the public domain and therefore available for public use (Armand P., et al. Validation and refinement of the Disease Risk Index for allogeneic stem cell transplantation. Blood. 2014; 123(23): 3664-3671).

**Table B: The DRI**

| DRI group | Disease (stage) |
|---|---|
| LOW | AML favorable cytogenetics CR |
| INTERMEDIATE | AML intermediate cytogenetics CR |
| HIGH | AML favorable cytogenetics (advanced stage) |
| | AML adverse cytogenetics CR |
| | AML intermediate cytogenetics (advanced stage) |
| | ALL CR2 |
| VERY HIGH | AML (advanced stage) |
| | AML adverse cytogenetics (advanced stage) |

| | |
|---|---|
| CR = complete remission ALL = Acute lymphoblastic leukaemia AML cytogenetics *Favorable:* Inv(16); *Intermediate:* Normal, All other abn.; *Adverse:* Complex (≥4 abn.) Sources: Armand *et al.* 2010; Armand *et al.* 2014; Armand *et al.* 2012 | |

### European Group for Blood and Marrow Transplantation (EBMT) risk score

The EMBT risk score is in the public domain and therefore available for public use (Gratwohl A. The EBMT risk score. Bone Marrow Transplant. 2012; 47(6):749-56).

**Table C: The EBMT Risk Score¹**

| Risk factor | 0 points | 1 point | 2 points |
|---|---|---|---|
| Age (yr) | < 20 | 20 - 40 | > 40 |
| Disease stage* | Early | Intermediate | Late |
| Time interval from diagnosis to transplantation (months) | < 12 | > 12 | - |
| Donor type | HLA- identical sibling | Unrelated donor, other | - |
| Donor recipient sex combination | Any other combination | Female donor, male recipient | - |

| | | | |
|---|---|---|---|
| ¹The EBMT risk score is calculated as the sum of the scores for each of the five risk factors * Classification of disease stage for calculation of EBMT risk score | | | |

### Disease stages of AML:

| | |
|---|---|
| Early | In first complete remission |
| Intermediate | In second complete remission |
| Late | In all other disease stages |

### Glucksberg Acute Graft versus Host (GVHD) Score

The Glucksberg acute GVHD score is in the public domain and therefore available for public use (Gratwohl A. The EBMT risk score. Bone Marrow Transplant. 2012; 47(6):749-56).

### Skin

| | |
|---|---|
| Stage 0: | no active (erythematous) GVHD rash |
| Stage 1: | maculopapular rash involving < 25% of the body surface |
| Stage 2: | maculopapular rash involving 25-50% of the body surface |
| Stage 3: | maculopapular rash involving > 50% of the body surface |
| Stage 4: | generalised erythroderma (> 50% of the body surface) *plus* bullous formation and desquamation (> 5% of the body surface) |

### Liver

Stage 0: bilirubin < 2 mg/dL
Stage 1: bilirubin 2-3 mg/dL
Stage 2: bilirubin 3.1-6 mg/dL
Stage 3: bilirubin 6.1-15 mg/dL
Stage 4: bilirubin > 15 mg/dL

### Upper Gastrointestinal (GI)

Stage 0: no or intermittent nausea, vomiting, or anorexia
Stage 1: persistent nausea, vomiting or anorexia
Stage 2: n/a
Stage 3: n/a
Stage 4: n/a

### Lower GI (diarrhea stool output/day for adults)

| | | | | | |
|---|---|---|---|---|---|
| Stage 0: | < 500 | mL/day | or | < 3 | episodes/day |
| Stage 1: | 500-999 | mL/day | or | 3-4 | episodes/day |
| Stage 2: | 1000-1500 | mL/day | or | 5-7 | episodes/day |
| Stage 3: | > 1500 | mL/day | or | > 7 | episodes/day |
| Stage 4: severe abdominal pain with or without ileus or grossly bloody stool (regardless of stool volume) | | | | | |

**Table D: Glucksberg Acute GVHD Score¹**

| Grade | Skin | Liver | Lower GI | Upper GI |
|---|---|---|---|---|
| I | Stage 1-2 | Stage 0 | Stage 0 | Stage 0 |
| II | Stage 3 and/or | Stage 1 and/or | Stage 1 and/or | Stage 1 |
| III | Stage 0-3 | Stage 2-3 and/or | Stage 2-3 | Stage 0-1 |
| IV | Stage 4 and/or | Stage 4 and/or | Stage 4 | Stage 0-1 |

| | | | | |
|---|---|---|---|---|
| ¹Overall grading of acute GVHD is based on the most severe target organ involvement | | | | |

### National Institutes of Health (NIH) Chronic GVHD Score

The NIH chronic GVHD score is in the public domain and therefore available for public use (Filipovich et al. National Institutes of Health consensus development project on criteria for clinical trials in chronic graft-versus-host disease: I. Diagnosis and staging working group report. Biol Blood Marrow Transplant. 2005;11(12):945-56; Jagasia et al. National Institutes of Health consensus development project on criteria for clinical trials in chronic Graft-versus-Host Disease: I. The 2014 Diagnosis and Staging Working Group report. Biol Blood Marrow Transplant. 2015;21(3):389-401).

The NIH global scoring system for chronic GVHD reflects the clinical effect of chronic GVHD on the patient's functional status. Eight organs or sites (skin, mouth, eyes, gastrointestinal tract, liver, lungs, joint and fascia, and genital tract) are considered for calculating global score. Elements included in the proposed global scoring include both the number of organs or sites involved and the severity score within each affected organ. Performance status scoring is not incorporated into the global scoring system. The global descriptions of mild, moderate, and severe were chosen to reflect the degree of organ impact and functional impairment due to chronic GVHD. Note that the global scoring system can be applied only after the diagnosis of chronic GVHD is confirmed by either (1) the presence of a diagnostic feature or, if a diagnostic feature is not present, (2) at least 1 distinctive manifestation of chronic GVHD with the diagnosis supported by histologic, radiologic, or laboratory evidence of GVHD from any site.
- Mild chronic GVHD:
   ∘ 1 or 2 organs involved with no more than score 1 **PLUS** lung score 0
- Moderate chronic GVHD:
   ∘ 3 or more organs involved with no more than score 1 **OR**
   ∘ at least 1 organ (not lung) with a score of 2 **OR**
   ∘ lung score 1
- Severe chronic GVHD:
   ∘ At least 1 organ with a score of 3 **OR**
   ∘ lung score of 2 or 3

**Table E. The NIH chronic GVHD scoring system for individual organs**

| **Organ** | **Score** | **0 Score 1** | **Score 2** | **Score 3** |
|---|---|---|---|---|
| Skin | No symptoms | <18% of BSA no sclerotic features and | 19-50% of BSA or superficial sclerosis (able to pinch) | >50% of BSA or deep sclerosis (unable to pinch) or impaired mobility, ulceration, or severe pruritus |
| Mouth | No symptoms | Mild signs/symptoms limiting oral intake not | Moderate signs/symptoms with partial limitation of oral intake | Sever signs/symptoms with major limitation of oral intake |
| Eyes | No symptoms | Mild dry-eye symptoms (using eye drops ≤3X day) or asymptomatic, signs of keratoconjunctivitis sicca / but | Moderate dry-eye symptoms partially affecting ADL (using eyedrops ≥:3X / day or punctuate plugs), no visual impairment | Severe dry-eye symptoms significantly affecting ADL or unable to work or loss of vision |
| GI tract | No symptoms | Symptoms without significant weight loss | Symptoms with weight loss of 5-15% | Symptoms with weight loss >15% requiring nutritional supplementation or need for esophageal dilation |
| Liver | Normal LFT | Bilirubin, AP, AST, or ALAT <2X of ULN | All 2-5X of ULN or bilirubin >3mg/dl | All ≥5X of ULN |
| Lungs¹ | No symptoms | Mild symptoms (SOB after 1 flight of steps); FEV₁ 60-79 % or LFS 2 | Moderate symptoms (SOB after walking on flat ground); FEV1 40-59% or LFS 6-9 | Severe symptoms (SOB at rest or requiring supplement O₂); FEV1 <39% or LFS 10-12 |
| Joint/ fascia | No symptoms | Mild tightness of arms or legs, mildly decreased ROM and not affecting ADL | At least 1 of the following: tightness of arms or legs, joint contractures,erythema due to fasciitis, moderately decreased ROM and mild-moderate limitation of ADL | Contractures with significantly decreased ROM and significant limitation of ADL |
| Genital tract | No symptoms | Mild signs/symptoms and no effect on coitus/minimal discomfort on examination | Moderate signs/symptoms and mild dyspareunia/ discomfort with examination | Advanced signs (strictures, labial fusion or severe ulceration) and severe pain with coitus/inability to insert vaginal speculum |

### Example 3: KRP 203 prevents chronic GVHD in mice

### Methods

To investigate whether KRP203 has a beneficial effect on the development of chronic GVHD, a clinically relevant, well-established murine model of cutaneous chronic GVHD was used. Briefly, Balb/c mice underwent conditioning with 5.5-6 Gy of total body irradiation (TBI), which was followed by intravenous injection of 8×10⁶ bone marrow (BM) supplemented with 2.5×10⁷ splenocytes from allogeneic B10.D2 donors to induce chronic GVHD. No GVHD controls received donor cells from syngeneic Balb/c mice. Some recipients in the GVHD group received 3 mg/kg KRP203 starting on day -1 (i.e., one day prior to bone marrow transplant (BMT) and chronic GVHD induction on day 0) until final analysis. Chronic GVHD was evaluated at different time points post-BMT up to day 42. Classical pathological changes of chronic skin GVHD, e.g., reduced hair follicles and fibrosis, were analyzed in harvested skin samples.

To investigate whether treatment with KRP203 can ameliorate established chronic GVHD, allogeneic mice were treated with 3 mg/kg KRP203 from day 21 until day 42. Some allogeneic mice received vehicle. No GVHD controls received donor cells from syngeneic BALB/c mice.

Chronic GVHD was evaluated by overall survival, GVHD score, chronic GVHD skin score, lacrimal excretory test, histological analysis of the skin and lacrimal glands and Sircol collagen assay of the skin and lacrimal glands. The effect of KRP203 on immune cell trafficking was analyzed by immune cell phenotyping of the spleen, skin and lacrimal glands.

### Results

### KRP203 as prophylaxis for chronic GVHD

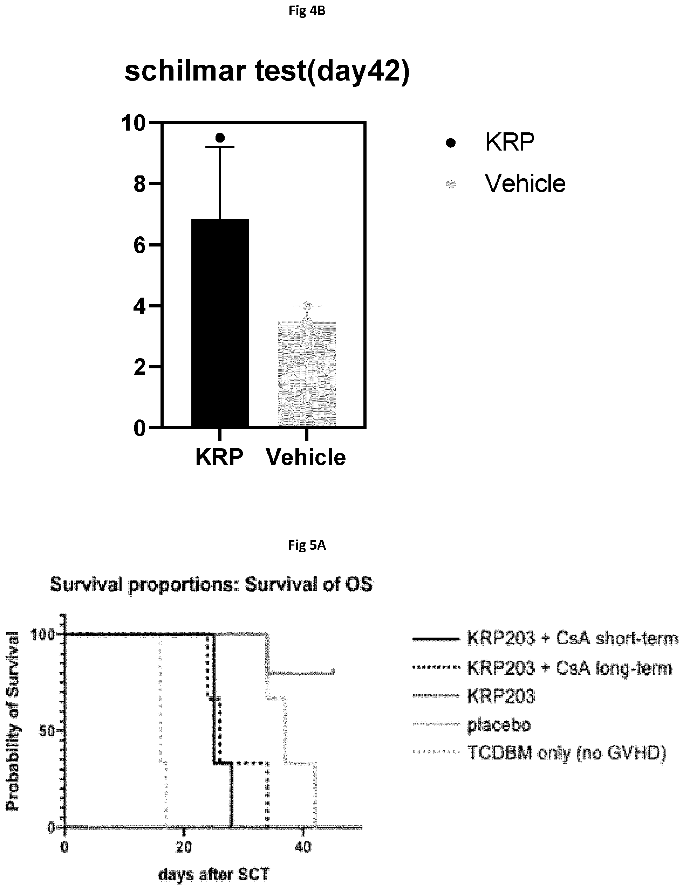

Skin pathology of GVHD recipients was evaluated from day 0 to 42. Mice who received KRP203 as prophylactic treatment starting on day -1 did not show any chronic GVHD-caused pathological skin changes and fur appeared normal similar to no GVHD control recipients (Figure 4A). This was in contrast to mice suffering from chronic GVHD who received vehicle treatment from day -1 onwards. These mice suffered from chronic GVHD with pathological skin changes and visible hair loss. Consequently, their skin clinical score was increased compared to mice who received KRP203 as chronic GVHD prophylaxis. Furthermore, a Schirmer test performed on day 42 post-bone marrow transplant to determine aqueous tear production, showed a low test result (<5mm) in GVHD mice who received vehicle, supporting the diagnosis of ocular chronic GVHD with severe dry eyes (Figure 4B). Prophylactic treatment with KRP203 improved this condition.

Taken together, these data demonstrate that KRP203 can be used as a prophylactic treatment for chronic GVHD resulting in reduced clinical pathology and improved survival.

### Conclusions

KRP203 treatment from day -1 to 42 (i.e., final analysis) was efficient to prevent development of chronic GVHD in mice who received bone marrow and T cells from genetically mismatched donors. This demonstrates that long-term treatment with KRP203 is beneficial to ameliorate quality of life and improves transplantation outcome.

### Example 4: Preparation of pharmaceutical composition comprising the S1P receptor modulator

12 excipients have been selected for the compatibility study of KRP203 (the S1P receptor modulator). In order to select the optimal combination of excipients and considering the risk of loss of stability, the following 17 blends have been prepared according to the table 4 below.

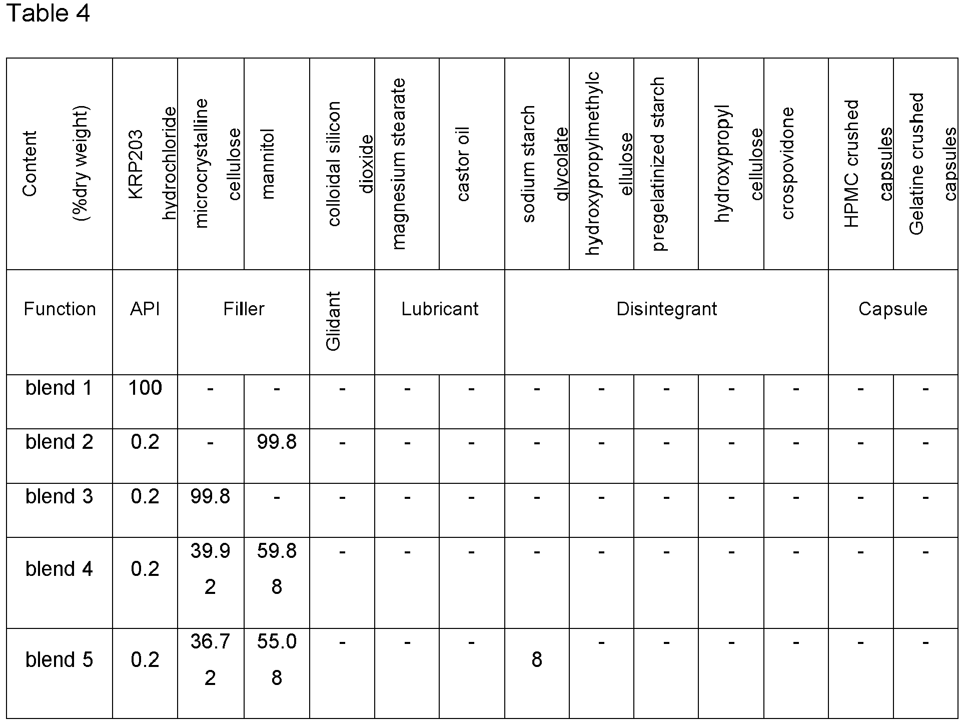

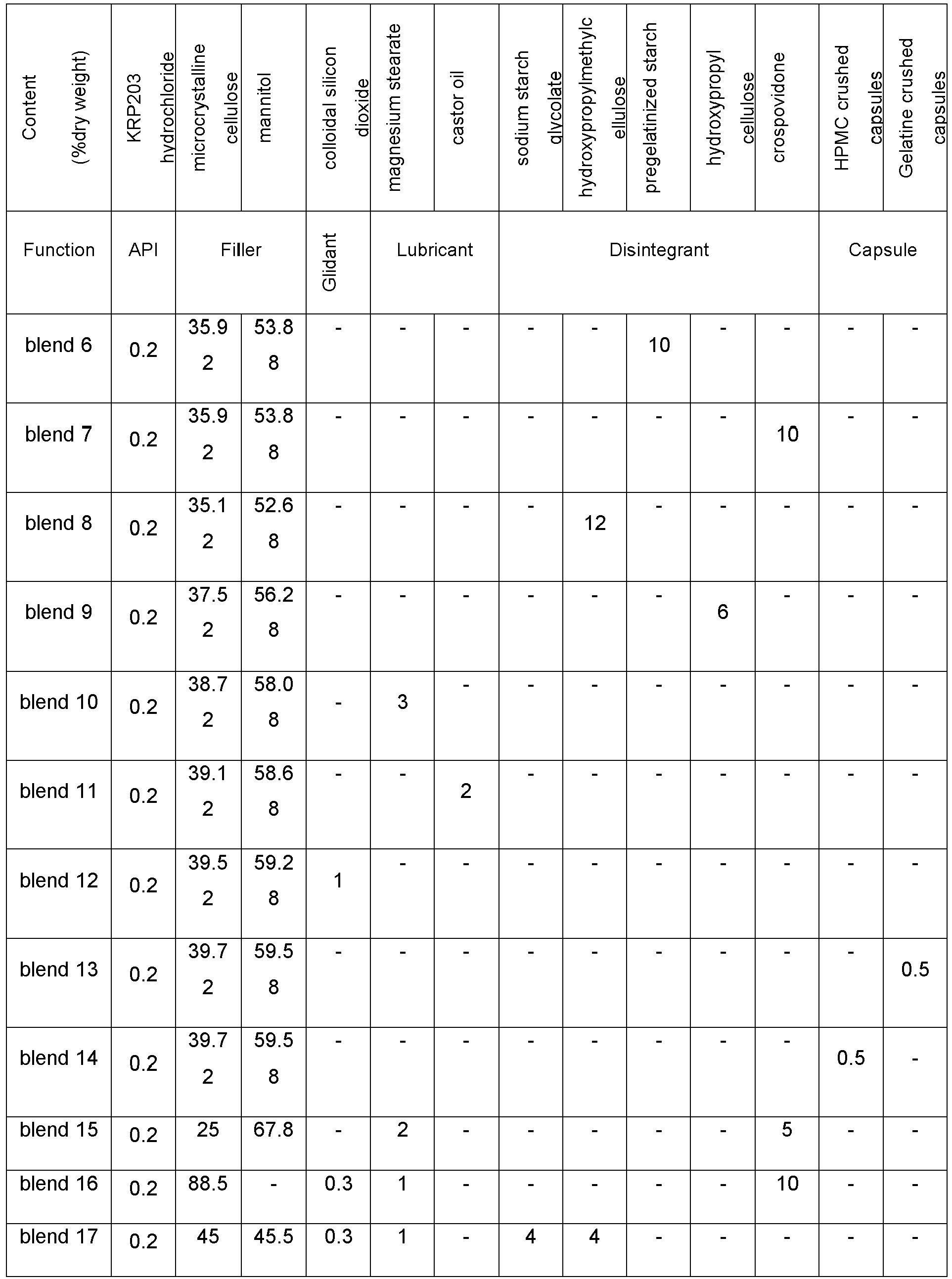

### Example 5: Stability data of the pharmaceutical compositions of example 1 stored at 50°C

The 17 blends of example 1 have been prepared in both dry and wet condition and stored at defined conditions for 6 weeks. The 17 blends were stored at defined conditions for 3, 6 weeks and were analyzed for the assay and the degradation products.

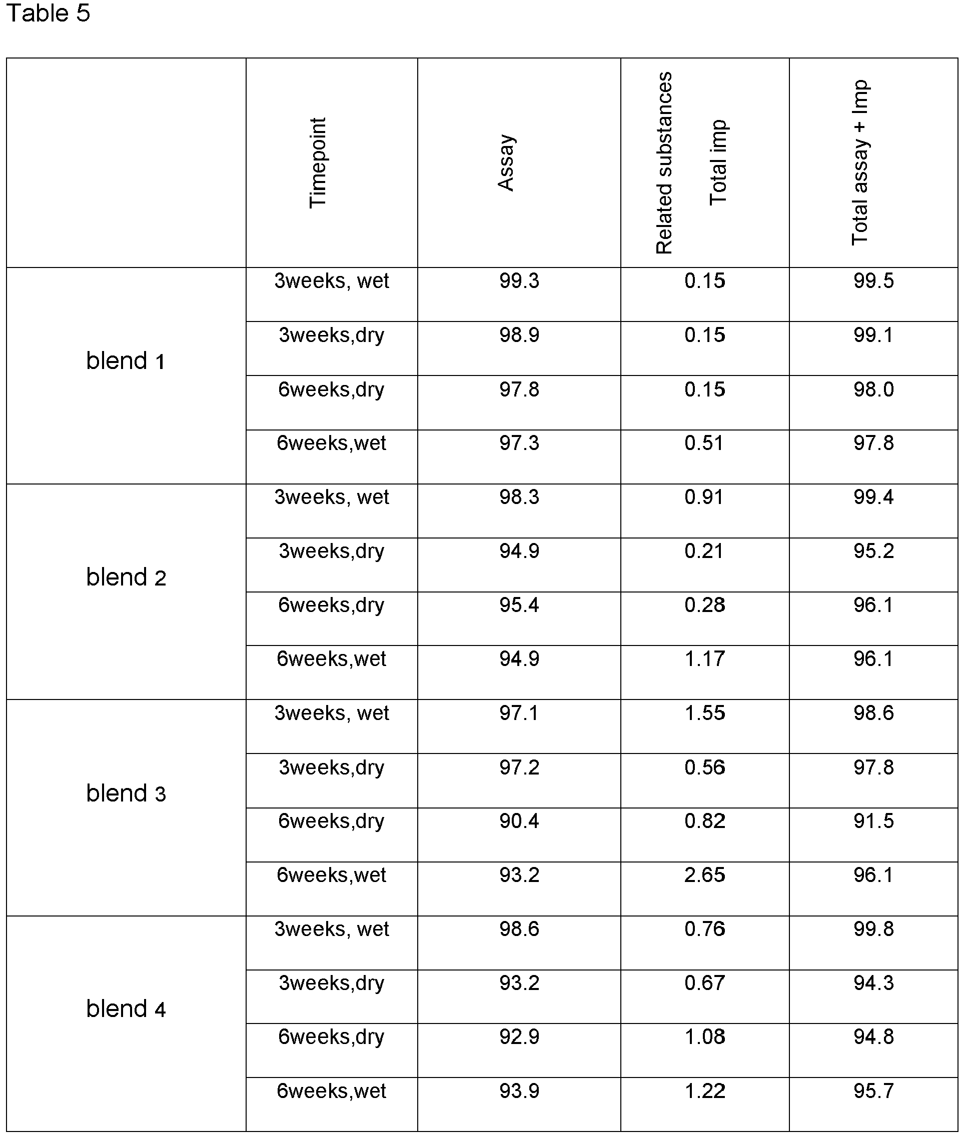

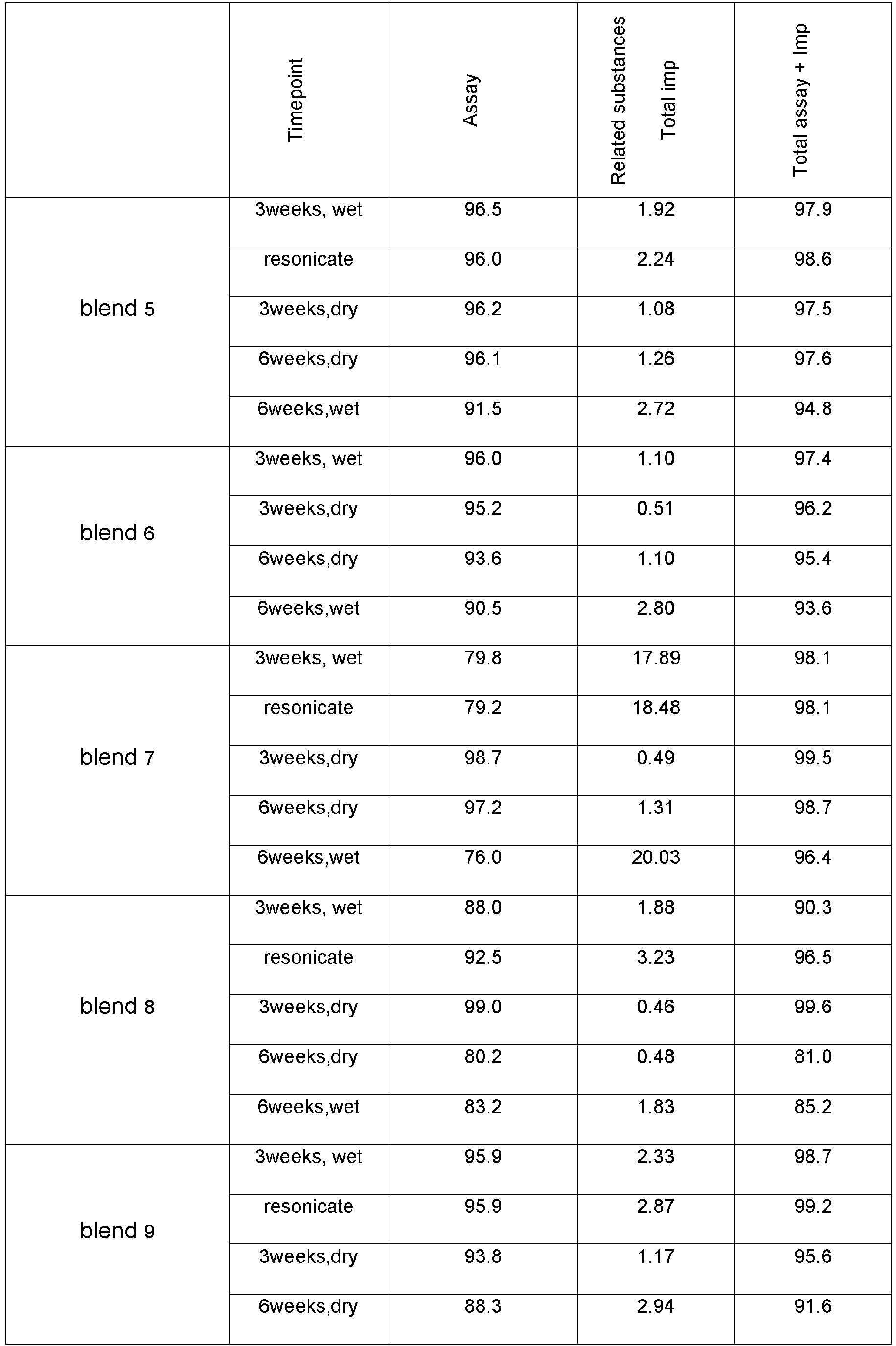

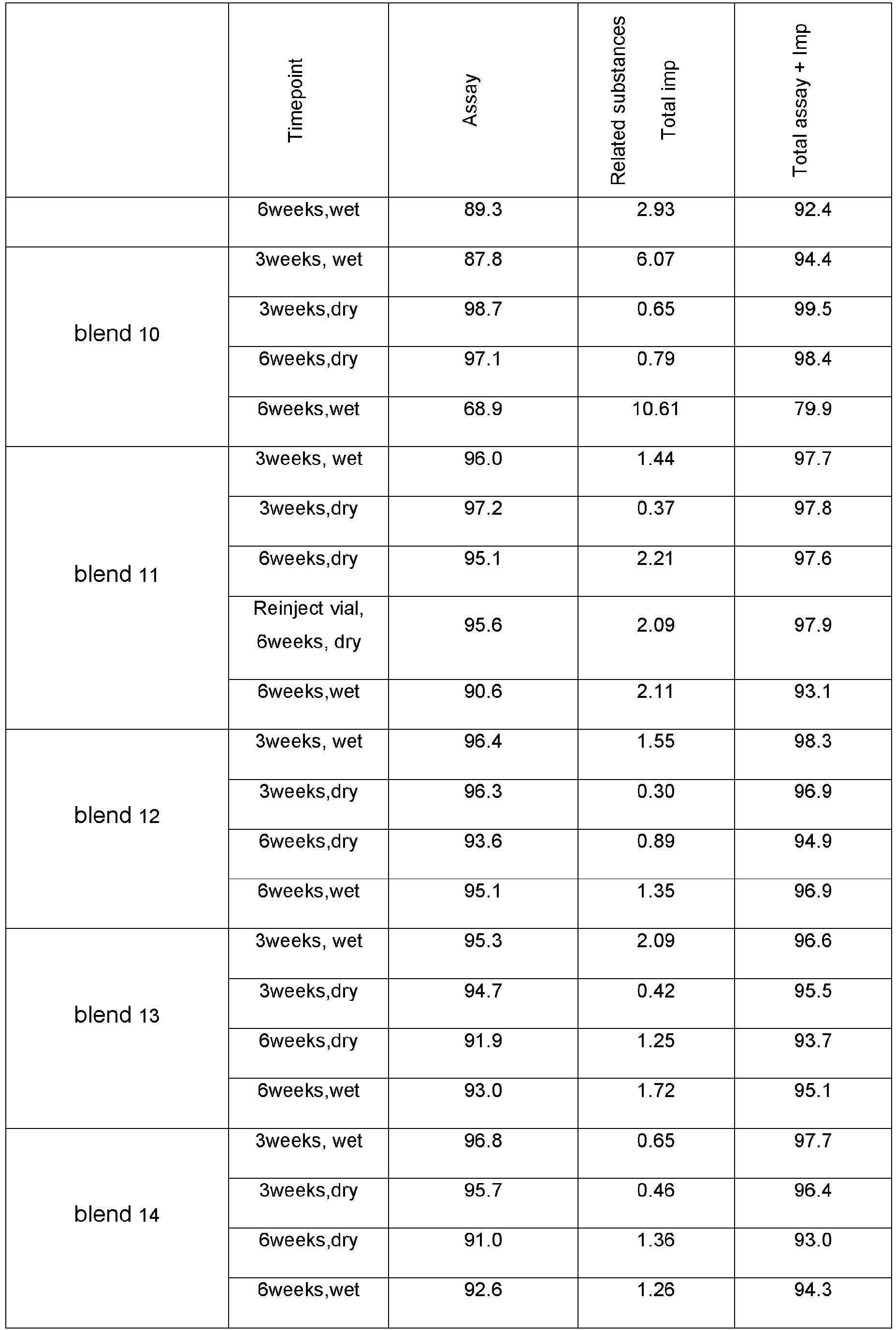

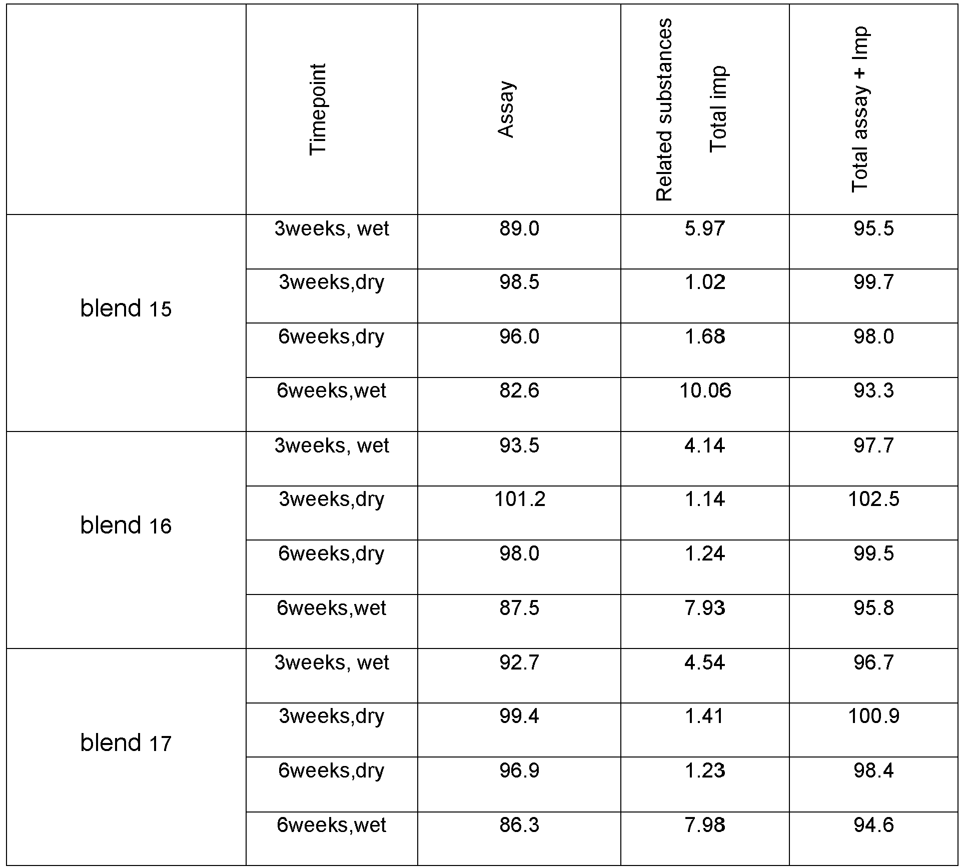

The generic blends in both dry and wet state did show some incompatibility and the extent of degradation was more pronounced in case of wet conditions. Hence, wet granulation as a process was not chosen for the development. Among the different combinations of dry blend, blends 7, 10, 16 and 17 were found to be highly compatible with assay data of 98.7%, 98.4%, 99,5% and 98.4% respectively at the end of 6 weeks. Among these, the blend 17 was found to be the optimal blend from the selection as it showed the best stability while including the required excipient for a capsule or tablet formulation of the S1P receptor modulator, namely, a filler, a disintegrant, a lubricant, and a glidant.

### Example 6: The pharmaceutical composition according to the invention

**Table 6 Formulation composition of 1 mg KRP203 capsules derived from blend 17 of examples 4 and 5**

| Excipient | Amount %w/w | Amount (kg) |
|---|---|---|
| KRP203 hydrochloride | 1.08 | 0.119 |
| mannitol | 68.23 | 7.505 |
| microcrystalline cellulose | 25.19 | 2171 |
| sodium starch glycolate | 4.00 | 0.440 |
| magnesium stearate | 1.00 | 0.110 |
| colloidal silicon dioxide | 0.50 | 0.055 |

**Table 7 Sub-lots of microcrystalline cellulose**

| Sub-lot | Amount (kg) |
|---|---|
| Sub-lot 1 | 0.100 |
| Sub-lot 2 | 0.100 |
| Sub-lot 3 | 0.100 |
| Sub-lot 4 | 1.200 |
| Sub-lot 5 | 1.271 |

The following process was used to manufacture the batch of 1 mg KRP203 capsules:
- Dispense the required amount of KRP203 hydrochloride, colloidal silicon dioxide, mannitol, sodium starch glycolate and magnesium stearate into appropriately labelled containers/bags.
- Dispense the required amount of microcrystalline cellulose into five separate sub-lots as detailed in Table 7.
- Place the charge bottle into the VSE
- In the VSE add the first sub-lot of microcrystalline cellulose, KRP203 hydrochloride and the colloidal silicon dioxide to a grey polypropylene container and blend in the Turbula mixer for 3 mins at 24 rpm.
- In the VSE sieve the blend from the polypropylene container through a 500 micron mesh into a PE bag.
- Sub-lot 2 of the cellulose rinse out the polypropylene container and then sieve sub-lot 2 into the PE bag.
- Transfer the sieved blend into the charge bottle.
- Sieve sub-lot 4 of the microcrystalline cellulose directly into 50L IBC
- Clean down the outside of the charge bottle before removing from the VSE.
- Transfer the blend from the charge bottle to the 50L IBC using the Ezi-dock system.
- Sieve sub-lot 3 of the microcrystalline cellulose into the used PE bag and then transfer into the charge bottle.
- Agitate the charge boille to ensure it is rinsed sufficiently and then transfer the cellulose from the charge bottle to the 50L IBC using the Ezi-dock system.
- Sieve sub-lot 5 of cellulose into the 50L IBC.
- Blend at 22rpm for 18 mins
- Unload the blend into a PE bag and transfer to the VSE, manually sieve through a 500 micron mesh into a PE bag.
- Transfer the blend from the PE bag to the 50L IBC.
- Manually sieve the pre-weighed Mannitol through the 500 micron mesh and add to the sieved blend in the 50L IBC.
- Blend at 22rpm for 9 mins
- Unload the blend into a PE bag and transfer to the VSE, manually sieve through a 500 micron mesh into a PE bag.
- Transfer the blend from the PE bag to the 50L IBC.
- Manually sieve the pre-weighed sodium starch glycolate through the 500 micron mesh and add to the sieved blend in the 50L IBC.
- Blend at 22rpm for 5 mins
- Manually sieve the pre-weighed magnesium stearate through the 500 micron mesh and add to the blend in the 50L IBC.
- Blend at 22rpm for 5 mins
- Remove the blend and place into labelled double Polyethylene Bags
- Transfer to building 6 for encapsulation
- A capsule filling speed challenge will be performed on the MG2 Labby and captured in the development record.
- Encapsulate the blend (100mg fill weight) into capsules (size 4) using the MG2 Labby Encapsulation machine. Perform in-process weight checks every 20 minutes on 20 capsules.
- De-dust the capsules manually.
- Pack the capsules into double PE bags and cable-tie closed.
- Place the PE bags into an aluminum foil bag and heat seal closed.

The manufacture ran smoothly, with no problems encountered.

### Example 7: The effect of KRP203 treatment with short- or long-term administration of cyclosporine A (CsA)

### Methods

***Mice:*** Female B6 (H-2b) and B6D2F1 (H-2b/d) mice were purchased from CLEA Japan (Tokyo, Japan) and used for allogeneic bone marrow transplantation at 8-12 weeks of age. All animal experiments were performed under the auspices of the Institutional Animal Care and Research Advisory Committee.

***Bone marrow transplantation**:* B6D2F1 recipients were lethally irradiated (11 to 13 Gy) and i.v. injected with 5x106 T-cell depleted bone marrow cells and 1x106 purified T cells from MHC-haploidentical B6 donors on day 0. Purification of T cells and T-cell depletion was performed using mouse Pan-T-cell Isolation kit II (Miltenyi Biotec, Auburn, CA) and anti-CD90-Microbeads (Miltenyi Biotec) on an AutoMACS Pro Separator (Miltenyi Biotec). To evaluate graft-versus-leukemia effects, 5x104 P815-luc+ cells were injected into mice on day 0 of stem cell transplant (SCT).

***Reagents:*** KRP203 (Priothera, Dublin, Ireland) was dissolved in sterile water and orally administered at a dose of 1-3 mg/kg from day 0 to day 14, 28 or final day of analysis after transplant. Cyclosporine A (CsA; Novartis, Tokyo, Japan), at a dose of 25 mg/kg/d was orally administered daily from day 0 to day 14 or 28 after transplant.

***Evaluation of graft-versus-host* disease:** Survival was monitored daily and clinical GVHD was assessed by using a GVHD scoring system with five parameters: body weight loss, activity, posture, fur texture, and alopecia. For pathological scoring, tissue samples were fixed in 10% formalin, embedded in paraffin, sectioned, and stained with hematoxylin and eosin (H&E). T-cell infiltration into target tissues including the liver, gut, and skin were assessed by flow cytometric analyses.

***Evaluation of graft-versus-leukemia* effects:** In vivo bioluminescence imaging (BLI) was conducted weekly after transplant. Mice were subcutaneously injected with 500 µg d-luciferin (Promega, Madison, WI), and in vivo imaging was done 5 min later. Luciferase+ cells were detected using IVIS Imaging System ver. 4.3.1 (Perkin Elmer, Waltham, MA). Light emission is presented as photons per second per square centimetre per steer radiant (ph/s/cm2/sr).

### Results

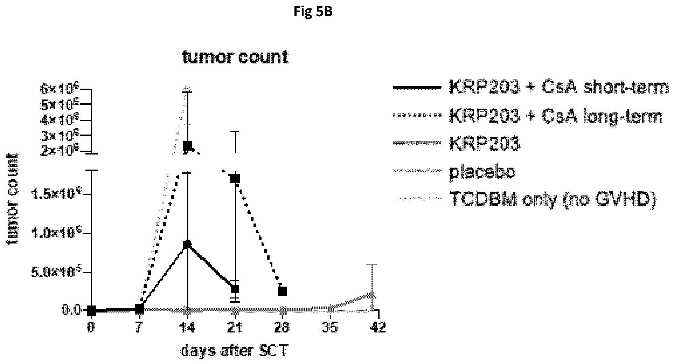

Mice with GVHD received either KRP203 treatment alone, KRP203 + Cyclosporine A short-term (i.e., up to day 14), KRP203 + Cyclosporine A long-term (i.e., for the whole observation period until final analysis) or placebo. No GVHD control mice received T-cell depleted bone marrow only without T cells. While short-term application of Cyclosporine A did not show any survival benefits compared to long-term CsA treatment, GVHD+ mice who received KRP203 treatment alone had highest survival (Figure 5A). No GVHD control mice who did not receive T cells, do not have any graft-versus-leukemia (GVL) effect, hence tumor growth was highest in this group and eventually all mice died because of leukemia (Figure 5B). Amongst mice with GVHD, tumor growth was best controlled in the group receiving KRP203 treatment alone with no leukemia death recorded in this group. Quantification of tumor counts showed that short-term Cyclosporine A on top of KRP203 resulted in fewer tumor cells compared to long-term Cyclosporine A on top of KRP203 treatment. Mice did not die of GVHD. These data indicate that Cyclosporine A can be stopped early with no impairment of GVHD, but better graft-versus-leukemia when KRP203 treatment is continued.

### Conclusions

Tumor growth is better controlled when Cyclosporine A is reduced or stopped early compared to long-term administration in combination with KRP203.

### Example 8: Clinical Study for treating AML patients undergoing HSCT

Provided herein is a prophetic example describing a prospective randomized, double-blind, placebo-controlled, multi-center phase IIb study to evaluate the efficacy and safety of mocravimod as an adjunctive and maintenance treatment in adult acute myeloid leukemia (AML) patients undergoing allogeneic hematopoietic stem cell transplant (HSCT).

### INTRODUCTION

### Rationale for use of Mocravimod

Allogeneic HSCT is a standard curative treatment for AML. The major limitation for successful outcome of allogeneic HSCT is disease relapse. Graft-versus-leukemia (GVL) is critical to prevent disease relapse and is mediated by donor T cells contained in the HSCT graft that trigger immune responses against leukemic cells. The off-target effect of the desirable GVL effect - graft-versus-host disease (GVHD) - remains a major complication of HSCT with significant morbidity and mortality, particularly when refractory to initial management. New strategies are being designed to sustain or reimpose GVL in order to reduce disease relapse, while preventing GVHD. With its novel mechanism of action, mocravimod's potential to preserve the desirable GVL effect while decreasing GVHD is a promising way to maintain the prospect of a cure while decreasing transplant-related mortality and morbidity in AML patients undergoing allogeneic HSCT.

The positive effects of mocravimod on disease relapse, stem cell engraftment, and GVHD inhibition have been demonstrated in previous preclinical and clinical studies. The dosing regimen of mocravimod in the setting of HSCT for hematologic malignancies in adults has been established in a phase Ib study CKRP203A2105 (ClinicalTrials.gov identifier: NCT01830010), in which mocravimod (1 mg/day or 3 mg/day) was administered for a treatment duration of around 3 months in combination with various immunosuppressive GVHD prophylaxis regimens. The data support proceeding with a pivotal clinical study to assess the efficacy and safety of mocravimod as an adjunctive and maintenance therapy for allogeneic HSCT for a longer duration of treatment up to 1 year, which may improve survival.

To date, there is no approved comparator across regions for the proposed indication. Therefore, a placebo-controlled design has been chosen for this study. The purpose of this study is to assess the efficacy and safety of mocravimod (1 mg/day and 3 mg/day) compared with the placebo as an adjunctive and maintenance treatment for allogeneic HSCT in AML patients in CR1 or CR2 with a treatment period of 1 year. In this study, the treatment with mocravimod or placebo will start 2 days prior to conditioning and will end 12 months after first dose.

### Objectives and Endpoints

| **Objectives** | | | **Endpoints** |
|---|---|---|---|
| Primary | | | |
| | • To compare the efficacy of mocravimod, in AML subjects in CR1 or CR2 after a 12-month treatment period to that of placebo | | • Relapse-free survival (RFS) at Months 12 (landmark analysis at Month 12) |

| Key Secondary | | | |
|---|---|---|---|
| | • To compare mocravimod's effect on overall survival (OS) at 24 months, (following a 12-month treatment and a 12-month follow-up period) to that of placebo | | • Overall survival (OS) at Month 24 after start of IMP intake |
| | • To assess the efficacy of the second dose of mocravimod in comparison to placebo, if applicable | | • RFS at Month 12 |

| Other Secondary | | | |
|---|---|---|---|
| | • To assess mocravimod's effect on the occurrence of GVHD in comparison to placebo | | • Survival free from refractory acute GVHD (aGVHD) at Month 24 |
| | | | • Time to aGVHD |
| | | | • Survival free from moderate/severe chronic GVHD (cGVHD) at Month 24 |
| | | | • Time to cGVHD |
| | • To assess mocravimod's effect on the occurrence of non-relapse mortality in comparison to placebo | | • Non-relapse related mortality at Month 12 and at Month 24 |

| | **Objectives** | | **Endpoints** |
|---|---|---|---|
| | • To assess mocravimod's effect on GVHD and GVL, as compared to placebo | | • rGRFS at Month 12 and Month 24 |
| | • To assess mocravimod's effect on time to relapse, as compared to placebo | | • Time to relapse |
| | • To assess the safety and tolerability of mocravimod | | • Type, frequency, seriousness, and severity of AEs according to the National Cancer Institute Common Toxicity Criteria for Adverse Events (NCI CTCAE) |
| | | | • Incidence of adverse events of special interest (AESI) |
| | • To assess Quality of Life (QoL) after a 12-month treatment period | | • Patient reported outcomes utilizing the Foundation for the Accreditation of Cellular Therapy - Bone Marrow Transplantation questionnaire (FACT-BMT, version 4) |

| Exploratory | | | |
|---|---|---|---|
| | • To investigate mocravimod's effect on engraftment kinetics | | • Time to engraftment (neutrophil recovery is defined as neutrophil count ≥ 0.5×10⁹/L for 3 consecutive days and platelet recovery is defined as platelet count ≥ 20×10⁹/L for 3 consecutive days, without transfusion) within prior 7 days. |

| | Objectives | | Endpoints |
|---|---|---|---|
| | • To investigate mocravimod's effect on the response of the immune system to various events | | • Immunosuppressant-free survival (IFS) at Month 12 after HSCT |
| | | | • Immunophenotyping of lymphocyte subpopulations |
| | • To explore the subsequent treatments that are used during the first year of the study | | • Subsequent treatments during the first year of next treatment |
| | • To assess the relationship between clinical safety as well as efficacy and exposure data for mocravimod | | • Mocravimod trough level at every PK visits and blood concentrations-time profile (Day -9 analyzed by model dependent (popPKPD) approach |
| | • To assess the relationship between relapse occurrence and exposure data with cross comparative analysis of CsA/TAC and mocravimod | | • Mocravimod and CsA/TAC trough level at every PK visits |
| | • To explore the relationship between mocravimod and the stratified and non-stratified subgroups | | • RFS at Month 12 |
| | | | • OS at Month 24 after start of IMP intake |

### Study design

This phase IIb study is designed as follows:
- A prospective, multicenter, randomized, double-blind, placebo-controlled, and parallel group study
- Approximately 300 subjects will be screened to randomize approximately 249 subjects with AML in CR1 or CR2, undergoing allogeneic HSCT
- This study will be conducted at approximately 80 study sites in North America, Europe, and possibly Asia-Pacific
- Eligible subjects will be stratified by the complete remission status (CR1 versus CR2) and the treatment (CsA versus TAC) used for the prophylaxis of GVHD. An Interactive Voice/Web Response System (IxRS) will be used for randomization (mocravimod 1 mg, mocravimod 3 mg or matching placebo [1:1:1 ratio])
   ∘ 1 mg/day mocravimod (1 mg arm): Approximately 83 subjects will receive 1 mg of mocravimod orally once per day from Day -9 ± 1 day to 12 months post first investigational medicinal product (IMP) intake
   ∘ 3 mg/day mocravimod (3 mg arm): Approximately 83 subjects will receive 3 mg of mocravimod orally once per day from Day -9 ± 1 day to 12 months post first IMP intake
   ∘ Placebo (placebo arm): Approximately 83 subjects will receive placebo orally once per day from Day -9 ± 1 day to 12 months post first IMP intake
- Subjects will be required to record each administration of the IMP in the subject diaries to ensure treatment compliance.
- The study duration will be approximately 24 months, comprising the following phases:
   ∘ Screening and enrollment phase (screening phase of up to 28 days until randomization on the day of start of study treatment or the day before)
   ∘ Double-blind treatment phase (2 days before start of conditioning to end of Month 12 or until relapse or death, or the end of treatment due to the onset of intolerable toxicity or due to any other reason).
   ∘ RFS Follow-up phase (for subjects who discontinue study treatment prematurely but did not relapse or die; until up to the end of Month 12)
   ∘ OS Follow-up phase (treatment free period from either end of treatment at 12 months, or end of RFS Follow-up phase, or relapse, for an additional 12 months)
- Subjects will be hospitalized 2 days before start of consolidation for randomization and the start of the administration of the IMP, followed by the conditioning regimen and allogeneic HSCT (Day 0). The length of hospitalization post-HSCT will depend on the subject's condition, investigator's judgment, and local practices
- The study consists of the following visits:
   ∘ Screening and randomization phase: Screening visit within 28 days before start of study treatment; randomization on the day of the start of study treatment or the day before.
   ∘ Double-blind treatment phase:
      ▪ Day 2 before start of consolidation (Hospitalization, baseline and start of IMP 2 days before the start of conditioning regimen)
      ▪ Mocravimod PK blood collection (pre-treatment and 24 h after the first IMP administration)
      ▪ Day -7 ± 1 day (start of conditioning regimen)
      ▪ Day -5 (mocravimod PK blood collection)
      ▪ Day -2 (CsA/TAC PK blood collection)
      ▪ Day 0 (HSCT)
      ▪ Day 14 ± 2 days
      ▪ Month 1 (Day 28) ± 2 days
      ▪ Month 2 (Day 56) ± 2 days
      ▪ Month 3 (Day 84) ± 2 days
      ▪ Month 4 (Day 112) ± 2 days
      ▪ Month 5 (Day 140) ± 2 days
      ▪ Month 6 (Day 168) ± 2 days
      ▪ Month 7(Day 196) ± 2 days
      ▪ Month 8 (Day 224) ± 2 days
      ▪ Month 9 (Day 252) ± 2 days
      ▪ Month 10 (Day 280) ± 2 days
      ▪ Month 11 (Day 308) ± 2 days
      ▪ Month 12 (Day 336) ± 2 days (End of treatment [EOT]/Early Discontinuation [ED] visit)
   ∘ RFS Follow-up phase:
      ▪ Monthly visits as in the double-blind treatment phase
   ∘ OS Follow-up phase:
      ▪ Month 13 (Day 364) ± 7 days (28 days after last IMP intake)
      ▪ Month 15 (Day 420) ± 7 days
      ▪ Month 18 (Day 504) ± 7 days
      ▪ Month 21 (Day 588) ± 7 days
      ▪ Month 24 (Day 672) ± 7 days (end of study [EOS]/ED)
   ∘ Unscheduled visit(s) may be arranged when necessary
- Safety reviews will be performed by an Independent Data Monitoring Committee (IDMC). An adjudication of the primary endpoint will be performed by an Independent Adjudication Committee (IAC).
- A futility interim analysis will be performed when 14 events of disease relapse or death will have occurred. It will only be performed if no treatment arm has been stopped due to safety findings earlier and if there are at least 20 subjects pending recruitment. Recruitment will be frozen once the events are reached, and restarted once the analysis will be finalized. A dedicated unblinded team at the Clinical Research Organization (CRO) will perform the analyses and will share the results with the IDMC who will assess and make the decision to stop or not stop the futile arm.

### Scientific rationale for study design

This study is designed as a prospective, multicenter, randomized, double-blind, placebo-controlled, and parallel group study. The purpose of this study is to assess the efficacy and safety of mocravimod (1 mg/day and 3 mg/day) compared with placebo as an adjunctive and maintenance treatment for AML patients in CR1 or CR2 undergoing allogeneic HSCT. Local standard of care GVHD prophylaxis of MTX plus CsA or MTX plus TAC will be used. To date, there is no approved comparator for the proposed indication. Therefore, a placebo-controlled design has been chosen for this study to prospectively assess the magnitude of changes in the efficacy and safety that may occur in.

The treatment assignment is blinded to the investigator, the study teams who are involved in the conduct of the study, and the subjects throughout the double-blind treatment phase to reduce potential bias during data collection and evaluation of study endpoints. The study data will be unblinded once all subjects have completed the EOT visit or the RFS Follow-up phase.

A futility interim analysis will be performed when 14 events of disease relapse or deaths have occurred. It will only be performed if no treatment arm has been stopped due to safety findings earlier and if there are at least 20 subjects pending recruitment. The purpose of the futility interim analysis is to investigate the efficacy of the 1 mg arm against 3 mg arm, to avoid exposing subjects to a potentially inferior treatment.

### Justification for dose

In this study, the treatment with mocravimod (1 mg/day or 3 mg/day) or placebo will start 2 days before start of conditioning and will end 12 months after first study drug intake.

In the phase Ib study (CKRP203A2105) of mocravimod (1 mg/day plus CsA or 3 mg/day plus CsA or TAC) the safety profile was comparable between the 1 mg and 3 mg mocravimod dose groups. A model-based PK/PD analysis was performed with the data from this study. Due to sparsity of available data, the exposure-response analysis could not inform on a potential difference between the 1-mg and 3-mg dose with regard to the GRFS efficacy endpoint of this study. However, in the modelling and simulation analysis, when relating the surrogate endpoints Absolute Lymphocyte Count (ALC), CD4+ and CD8+ T cell counts to dose, a more pronounced CD8+ T-cell count reduction with the 3-mg dose given with SOC plus CsA was revealed, while maximum or near-maximum cell count reduction was seen for CD4+ and ALC counts.

The clinical experience with mocravimod includes single doses up to 40 mg in healthy volunteers and multiple doses up to 3 mg/day in healthy volunteers and patients. A total of 325 healthy volunteers participated in phase I placebo-controlled studies of mocravimod, where mocravimod was well tolerated, demonstrating a favorable safety profile. Mocravimod was also well tolerated in phase II studies in patients with ulcerative colitis, subacute cutaneous lupus erythematosus, and Crohn's disease. In the phase Ib/IIa study, mocravimod was administered for up to around 3 months in patients with several different hematologic malignancies receiving allogeneic HSCT. Both dose levels that were evaluated in this trial, 1 mg and 3 mg, did not show any significant differences in terms of safety and efficacy. However, patient numbers were low and efficacy results warrant more data in a more homogeneous patient population. To gather more data on both doses, this phase IIb study will investigate mocravimod at two different dose levels, a daily dose of 1 mg as well as of 3 mg.

In the study population that was chosen for this trial, most relapses occur early (< 1 year) and most GVHD events will have arisen by 1 year. Therefore, a treatment duration of 1 year is chosen for the present study.

Dosing of mocravimod in this study is not body weight-related as no dose-dependent effects on safety and preliminary signs of efficacy were observed in the phase Ib/IIa study.

### Study population

### Inclusion criteria

Each subject must meet the following criteria to be enrolled in this study:

### Type of Subject and Disease Characteristics

1. Subjects with a diagnosis of AML (excluding acute promyelocytic leukemia) according to the World Health Organization 2016 classification of AML and related precursor neoplasms, including secondary AML after an antecedent hematological disease (e.g. myelodysplastic syndrome) and therapy-related AML.
2. Subjects with ELN high risk AML in CR1 or any other AML in CR2. (Complete remission with incomplete count recovery [CRi] is also allowable).
   ∘ Complete remission is defined as leukemia clearance (< 5% marrow blasts and no circulating peripheral blasts) in conjunction with normal values for absolute neutrophil count and platelet count, no extramedullary manifestation of leukemia and no need for repeat blood transfusions.
   ∘ CRi is defined as meeting all complete remission criteria except for an absolute neutrophil count < 1,000/µL or platelet count < 100,000/µL.
3. Subjects planned to undergo allogeneic HSCT, with all of the following parameters met:
   ∘ Use of fully matched related or unrelated donor (10/10 HLA-matched), and
   ∘ Use of granulocyte colony-stimulating factor (G-CSF) mobilized peripheral blood stem cells, *and*
   ∘ Planned use of protocol-approved (myeloablative) conditioning regimen, *and*
   ∘ Planned use of MTX plus CsA or MTX plus TAC as GVHD prophylaxis
4. Life expectancy ≥ 6 months at screening.
5. Karnofsky Performance Status (KPS) ≥ 70%.

### Gender and Age

6. Male or female, age ≥ 18 years and ≤ 75 years.
Subjects ≥ 65 years must have a Sorror (hematopoietic cell transplantation-specific comorbidity index [HCT-CI]) Score ≤ 3.

### Informed Consent

7. Able and willing to provide written informed consent and comply with the trial protocol and procedures.

### Contraceptive/Barrier Requirements

8. For females of childbearing potential who are sexually active and males who have sexual contact with a female of childbearing potential: willingness to use reliable methods of contraception (oral contraceptives, contraceptive injection, male vasectomy, condom with spermicidal agent, or sexual abstinence). Contraception is to be used from the Screening visit until the EOT visit, and in any case for at least 6 months after the last dose of IMP.
A female is considered of childbearing potential following menarche and until becoming post-menopausal unless permanently sterile. Women are considered post-menopausal and not of childbearing potential if they have had 12 months of natural (spontaneous) amenorrhea with an appropriate clinical profile (e.g. age appropriate, history of vasomotor symptoms) or have had surgical bilateral oophorectomy (with or without hysterectomy) or tubal ligation at least 6 weeks before IMP treatment. In the case of oophorectomy alone, only when the reproductive status of the woman has been confirmed by follow-up hormone level assessment, she is considered not of childbearing potential.
Women of childbearing potential must be willing to undergo pregnancy testing on a monthly basis.

9. Sexually active males must use a condom during intercourse from the Screening visit until at least 6 months after the last dose of IMP and should not father a child in this period. A condom is required to be used also by vasectomized men in order to prevent delivery of the IMP via seminal fluid.

### Other Inclusion

10.Affiliation to a national health insurance scheme (according to applicable local requirements).

### Exclusion criteria

Subjects who meet any of the following criteria will be excluded from the study:

### Prior/Concomitant Therapy

1. Planned use of anti-thymocyte globulin (ATG), post-transplantation cyclophosphamide, or mycophenolate mofetil for GVHD prophylaxis.
2. Planned use of serotherapy during conditioning, including ATG and alemtuzumab.
3. Planned ex vivo major graft manipulation, including T-cell depletion or CD34+ selection.
4. Subjects having received prior allogeneic HSCT or recipients of a solid organ transplant.
5. Vaccination within 4 weeks prior to randomization.
6. Immunosuppressive drugs for concomitant disease. Subjects must be able to be off prednisone (> 10 mg/day) or other immunosuppressive medications for at least 3 days prior to the start of treatment of the study. Physiologic replacement dosing of hydrocortisone is permissible.
7. Major surgery within 4 weeks prior to randomization or a major wound that has not fully healed.
8. Require any of the following treatments for cardiac dysfunction:
   ∘ Treatment with medication that impairs cardiac conduction (e.g. beta blockers, verapamil-type and diltiazem-type calcium channel blockers, or cardiac glycosides), or
   ∘ Concomitant use of agents known to prolong the QT interval unless they can be permanently discontinued for the duration of the study, or
   ∘ Treatment with quinidine.

### Medical Conditions

9. Subjects with acute promyelocytic leukemia.
10. Diagnosis of any previous or concomitant malignancy, except subjects diagnosed with localized basal cell carcinoma of the skin or *in situ* cervical cancer, or subjects who have completed treatment (chemotherapy and/or surgery and/or radiotherapy) with curative intent for the malignancy at least 3 years prior to enrollment.
11. Blast crisis of chronic myeloid leukemia.
12. Concurrent severe and/or uncontrolled medical condition including:
   ∘ Clinically significant pulmonary fibrosis
   ∘ Tuberculosis, except for history of successfully treated tuberculosis or history of prophylactic treatment after positive purified protein derivative (PPD) skin reaction
   ∘ Subjects receiving chronic (daily) therapies for asthma
   ∘ Subjects with any other types of clinically significant obstructive pulmonary disease
   ∘ Uncontrolled diabetes mellitus as assessed by the investigator or diabetes complicated with organ involvement such as diabetic nephropathy or retinopathy
   ∘ Uncontrolled seizure disorder
   ∘ Uncontrolled depression or history of suicide attempts/ideation.
13. Cardiac dysfunction as defined by:
   ∘ Myocardial infarction within the last 3 months of trial entry, or
   ∘ Reduced left ventricular function with an ejection fraction < 40% as measured by multi-gated acquisition (MUGA) scan or echocardiogram (echo) within 6 weeks before signing informed consent, or
   ∘ History or presence of stable or unstable ischemic heart disease (IHD), myocarditis, or cardiomyopathy, or
   ∘ New York Heart Association (NYHA) Class II-IV congestive heart failure, or
   ∘ Unstable cardiac arrhythmias including history of or presence of symptomatic bradycardia, or
   ∘ Resting heart rate (physical exam or 12-lead electrocardiogram [ECG]) < 60 bpm, *or*
   ∘ History or current diagnosis of ECG abnormalities indicating significant risk of safety such as: Concomitant clinically significant cardiac arrhythmias, e.g. sustained ventricular tachycardia, presence of a clinically relevant impairment of cardiac conduction including sick sinus syndrome, or sino-atrial heart block, clinically significant atrioventricular (AV) block, bundle branch block or resting QTc (Fridericia preferred, but Bazett acceptable) > 450 msec for males and > 470 msec for females at Screening or Baseline ECG, or
   ∘ History or presence of symptomatic arrhythmia or arrhythmia requiring treatment or being otherwise of clinical significance, or
   ∘ Uncontrolled arterial hypertension; if controlled, the medication must be stable for 3 months prior to baseline visit, or
   ∘ Requiring treatment with prohibited medication listed under 'Exclusion criteria - prior/concomitant therapy'
   ∘ History of syncope of suspected cardiac origin, or
   ∘ History of familial long QT syndrome or known family history of Torsades de Pointes.
14. Pulmonary dysfunction as defined by oxygen saturation < 90% on room air. Pulmonary function test (PFT) is required only in the case of symptomatic or prior known impairments within 6 weeks before signing informed consent - with pulmonary function < 50% corrected diffusing capacity of the lung for carbon monoxide (DLCO) and < 50% predicted forced expiratory volume in 1 second (FEV₁).
15. Significant liver disease or liver injury or known history of alcohol abuse, chronic liver or biliary disease. Hepatic dysfunction as defined by aspartate aminotransferase (AST) and/or alanine aminotransferase (ALT) >2.5 × upper limit of normal (ULN); or total bilirubin > 1.5 mg/dL, unless attributable to Gilbert's syndrome, in which case < 3 mg/dL.
16. Renal dysfunction with estimated creatinine clearance <60 ml/min/m² by the Modification of Diet in Renal Disease (MDRD) method
17. History of stroke or intracranial hemorrhage within 1 year prior to screening.
18. Active clinically significant infection (viral, bacterial, or fungal) that requires ongoing antimicrobial therapy and in the judgment of the investigator represents a risk to proceeding with HSCT.
19. History of human immunodeficiency virus (HIV) or active infection with hepatitis B virus (HBV) or hepatitis C virus (HCV) defined as a positive HIV antibody, hepatitis B surface antigen or hepatitis C antigen.
20. Subjects who are breastfeeding or have positive pregnancy test (serum pregnancy test is mandatory at screening for women of childbearing potential ).
21. Known allergy to any of the components of mocravimod (e.g. excipient), the non-investigational medicinal products (NIMPs), including conditioning regimen agents and GVHD prophylaxis, and concomitant medications and therapies possibly used in this trial.
22.Any contraindications to the NIMPs, including the conditioning regimen agents and the GVHD prophylaxis, and the concomitant medications and therapies possibly used in this trial, as stated in the local prescribing information for NIMP medicinal products.
23. Diagnosis of macular edema during screening. Subjects with a history of macular edema will be allowed to enter the study provided they do not have macular edema at the ophthalmic examination at screening.

### Prior/Concurrent Clinical Study Experience

24. Participation in another interventional clinical trial within 4 weeks prior to randomization or participation in a concomitant interventional clinical trial.

### Other Exclusions

25. Any other condition that, in the opinion of the investigator, makes the subject ineligible for the study.
26. Subjects under legal protection measure (guardianship, trusteeship, or safeguard of justice) and/or inability or unwillingness to comply with the requirements and procedures of this trial.

### Study treatment and concomitant therapy

Study treatment is defined as any investigational treatment(s), marketed product(s), and placebo intended to be administered to a study subject according to the study protocol. IMPs include mocravimod and placebo. NIMPs include products used in HSCT for conditioning and standard of care GVHD prophylaxis (MTX plus CsA or MTX plus TAC).

### Investigational Medicinal Products Administered per Arm

| **Treatment Name** | Mocravimod (KRP203) 1 mg/day | Mocravimod (KRP203) 3 mg/day | Placebo |
|---|---|---|---|
| **Type** | Drug | Drug | Drug (placebo) |
| **Dose Formulation** | Mocravimod (S1P receptor modulator) in hard gelatin capsule | Mocravimod (S1P receptor modulator) in hard gelatin capsule | Partially pregelatinized maize starch in hard gelatin capsule |
| **Unit Dose Strength(s)** | 1 mg of mocravimod/capsule | 1 mg of mocravimod/capsule | None |
| **Dosage Level(s)** | 1 mg of mocravimod | 3 mg of mocravimod | - |
| **Route of Administration** | Oral | Oral | Oral |

| **IMP or NIMP** | IMP | IMP | IMP |
|---|---|---|---|
| **Sourcing** | Provided centrally by the sponsor | Provided centrally by the sponsor | Provided centrally by the sponsor |
| **Packaging and Labeling** | Mocravimod will be provided in blisters. Each blister contains 30 capsules and will be labeled as required per country requirement. | Mocravimod will be provided in blisters. Each blister contains 30 capsules and will be labeled as required per country requirement. | Placebo will be provided in blisters. Each blister contains 30 capsules and will be labeled as required per country requirement. |

| | | | |
|---|---|---|---|
| *IMP: investigational medicinal product; NIMP: non-investigational medicinal product;* *S1P: sphingosine 1-phosphate* | | | |

### Treatment Arm(s)

| **Arm Title** | 1 mg Arm | 3 mg Arm | Placebo Arm |
|---|---|---|---|
| **Arm Type** | Experimental | Experimental | Placebo |
| **Arm Description** | Subjects will receive 1 mg of mocravimod (1 capsule of mocravimod and 2 capsules of placebo) orally once per day from 2 days prior to conditioning and up to 12 months after first dose | Subjects will receive 3 mg of mocravimod (3 capsules of mocravimod) orally once per day from 2 days prior to conditioning and up to 12 months after first dose | Subjects will receive 3 capsules of placebo orally once per day from 2 days prior to conditioning and up to 12 months after first dose |

### Non-investigational medicinal products

NIMPs include products used in HSCT for conditioning and GVHD prophylaxis (MTX plus CsA or MTX plus TAC). They are commercially available for human use under various brand names and will be administered as per local practice, unless otherwise stated.

### Conditioning regimen

All subjects will undergo a conditioning regimen before HSCT. One of the following conditioning regimens is to be administered. Conditioning regimen should start from Day -7 ±1 day. Dose and timing variations in the conditioning regimen are allowed to accommodate for subject's condition and/or local practice. Any change in dosage or timing in the conditioning regimen are to be discussed between the investigator and the medical monitor and to be recorded in the electronic case report form (eCRF).

One of the following regimens must be used:
1. Melphalan + fludarabine + thiotepa:
   - Fludarabine 120-180 mg/m² intravenous (IV)
   - Melphalan 110-140 mg/m² IV
   - Thiotepa 5-10 mg/kg IV
2. Busulfan + fludarabine:
   - Busulfan 9.6-12.8 mg/kg IV
   - Fludarabine 120-180 mg/m² IV
3. Busulfan + cyclophosphamide:
   - Busulfan 9.6-12.8 mg/kg IV
   - Cyclophosphamide 120 mg/kg IV
4. Busulfan + fludarabine + thiotepa:
   - Busulfan 9.6-12.8 mg/kg IV
   - Fludarabine 150-180 mg/m² IV
   - Thiotepa 5-10 mg/kg IV

When busulfan is utilized as a component of the conditioning regimen, appropriate PK monitoring with as needed dose-adjustments should be performed based on institutional standard of care. Suggested AUC range for daily exposure is 3600-6000 µM × min (-14.4-24.6 mg × h/L). Dosing frequency (e.g. daily versus q6h) is at the discretion of the investigator, but all busulfan must be administered intravenously.

### Graft-versus-Host-Disease prophylaxis

### • Calcineurin inhibitor (CNI) therapy

CsA and TAC should be started on Day -1 or -2 prior to stem cell infusion. CNI prophylaxis should continue until at least 3 months after HSCT, with tapering according to institutional standards, with a goal of discontinuation by 6 months post-HSCT in the absence of GVHD.

Appropriate clinical monitoring and management of toxicities (hypertension, hyperglycemia, hypomagnesemia, neurologic complications, etc.) should occur based on institutional practice.

Care should be taken to adjust dose based on potential drug-drug PK interactions (e.g. azole antifungals).
∘ Cyclosporine A (CsA)
   ▪ CsA should be started at a dose of 3 mg/kg/day IV (divided between 2 bolus infusions twice a day). Conversion to oral administration should occur based on clinical circumstances, at a conversion ratio based on institutional practice. If CsA is initiated via the oral route, the starting dose is 12 mg/kg/day divided BID (Ruutu et al 2014).
   ▪ Drug monitoring: CsA trough levels should be measured 12 hours after a dose (just before the administration of the next scheduled dose), and monitored regularly; doses will be adjusted to keep target concentrations of 200-300 µg/L during the first 3-4 weeks post-HSCT. In the absence of GVHD, the dose is decreased to reach a concentration of 100-200 µg/L thereafter
∘ Tacrolimus (TAC)
   ▪ TAC should be started at a dose of 0.02-0.03 mg/kg/day (either as continuous infusion or divided between 2 bolus infusions twice a day). Conversion to oral administration should occur based on clinical circumstances, at a conversion ratio based on institutional practice.
   ▪ Drug monitoring: TAC trough levels should be measured 12 hours after a dose (just before the administration of the next scheduled dose), and monitored regularly; dose should be adjusted to maintain target concentrations of 5-10 ng/mL

### • MTX dose/schedule:

∘ Day +1: 15 mg/m² IV once.
∘ Days +3, +6, +11: 10 mg/m² IV once.
∘ Use of leucovorin rescue is allowable and encouraged (same dose as MTX given every 6 hours for 3 doses starting 24 hours after MTX dose; given orally or IV).

Modifications of this regimen to accommodate decreased clearance or MTX toxicity are allowable but should be discussed with medical monitor.

### Preparation, handling, storage and accountability

The IMP used in this study will be prepared, packaged, and labeled under the responsibility of a qualified person from the sponsor or designee according to the Standard Operating Procedures (SOPs) of the sponsor or designee, Pharmaceutical Inspection Co-operation Scheme (PIC/S) Good Manufacturing Practice (GMP) guidelines, the International Council for Harmonisation of Technical Requirements for Pharmaceuticals for Human Use (ICH) Good Clinical Practice (GCP) guidelines, and applicable local law/regulations. The product will be labeled with descriptions "Clinical trial use only" as well as other required information according to the local regulatory requirements in the local language.

The IMP will be supplied ready for use by the sponsor or designee to the study sites. No further preparation will be needed before the administration of IMP. Upon randomization, subjects will be provided with IMP corresponding to their assigned treatment arms sufficient to cover the period between visits of the double-blind treatment phase. The IMP can be stored at room temperature. See pharmacy manual for details.

Available stability data demonstrate that mocravimod 1 mg hard gelatin capsules and placebo capsules when packaged in high density polyethylene (HDPE) bottles are stable for 48 months when stored at 5°C/ambient relative humidity (RH); for 36 months when stored at 25°C/60% RH, and for 6 months when stored at 40°C/75% RH.

Upon receipt, the IMP will be stored locally at the clinical sites until dispensation to the subject, according to the storage requirements specified in the Pharmacy Manual. All IMP must be stored in a secure, environmentally controlled, and monitored (manual or automated) area in accordance with the labeled storage conditions with access limited to the investigator and authorized site staff.

Accountability of the IMP will be documented at the study sites. Each time the IMP is dispensed to a subject, this must be recorded on a drug dispensing/accountability log. At regular intervals the clinical research associate (CRA) will perform a `drug reconciliation visit', verifying that all IMP that has been shipped to the study site can be accounted for by records of receipt, dispensing, and destruction. Unused IMP that is not dispensed may only be destroyed following authorization by a representative of the assigned CRO, and destruction should be fully documented. Alternatively, the IMP may be returned to the sponsor. Refer to the Pharmacy Manual for details.

At the EOT, it must be possible to reconcile delivery records with records of usage and destroyed or returned stock. It is essential that the investigator or the study site account for IMP, and that any discrepancies are explained and documented.

### Measures to Minimize Bias: Randomization and Blinding

Each subject will receive a 7-digit subject number: a 4-digit study site number followed by a 3 digit individual subject number in consecutive order (e.g. 0001-001), which is assigned by the study site at the screening visit and will be used throughout the study.

### Randomization

This study is designed as a randomized, double-blind, placebo-controlled study. Subjects will be stratified by the complete remission status (CR1 or CR2) and the GVHD prophylaxis treatment (CsA versus TAC) and randomized to receive either 1 mg/day or 3 mg/day of mocravimod or matching placebo (1:1:1 ratio) as an adjunctive treatment for HSCT.

All eligible subjects will be centrally assigned to randomized IMP using an IxRS. Before the study is initiated, the telephone number and call-in directions for the Interactive Voice Response System (IVRS) or the log-in information and directions for the Interactive Web Response System (IWRS) will be provided to each site.

### Blinding

The treatment assignment is blinded to the investigator, the study teams who are involved in the conduct of the study, and the subjects throughout the double-blind treatment phase to reduce potential bias during data collection and evaluation of study endpoints. The study data will be unblinded once all subjects have completed the EOT visit or the RFS Follow-up phase.

A futility interim analysis will be performed when 14 events of disease relapse or death will have occurred. It will only be performed if no treatment arm has been stopped due to safety findings earlier and if there are at least 20 subjects pending recruitment. Recruitment will be frozen once the events are reached, and restarted once the analysis will be finalized. A dedicated unblinded team at the Clinical Research Organization (CRO) will perform the analyses and will share the results with the IDMC who will assess and make the decision to stop or not stop the futile arm.

Each study site will be supplied with IMP with identical packaging. Both mocravimod and placebo capsules are identical in appearance: white to off-white powder in a pink opaque (Swedish orange) capsule, size #4.

### Procedures for Emergency Unblinding

The IxRS will be programmed with blind-breaking instructions. In case of an emergency, the investigator has the sole responsibility for determining if unblinding of a subject's treatment assignment is warranted. Subject safety must always be the first consideration in making such a determination. If the investigator decides that unblinding is warranted, the investigator should make every effort to contact the sponsor prior to unblinding a subject's treatment assignment unless this could delay emergency treatment for the subject. If a subject's treatment assignment is unblinded, the sponsor must be notified within 24 hours of this occurrence. Email notifications can be enabled to inform pre-specified email recipients when subjects or inventory items are unblinded. The date and reason for the unblinding must be recorded.

The IMP must be discontinued after emergency unblinding and the subject will be followed in the OS Follow-up phase. The IMP may or may not be discontinued for any subject whose treatment code has been broken inadvertently or for any non-emergency reason at the investigator's discretion.

### Investigational Medicinal Product Compliance

Subjects will be required to record each administration of the IMP in the subject diaries to ensure treatment compliance. The investigator should promote compliance by instructing the subject to take the IMP exactly as prescribed and by stating that compliance is necessary for the subject's safety and the validity of the study. The subject will be instructed to contact the investigator if for any reason unable to take the IMP as described.

Treatment compliance will be accomplished by documenting in record (i.e. drug accountability, administration logs, and subjects' eCRF) information on, but not limited to: the batch number of IMP, potential discontinuation/interruption of treatment administration, total number of IMP units administered, and signatures of designated study personnel delivering the IMP to the subject as described in the Pharmacy Manual. Deviation(s) from the prescribed dosage regimen should be recorded.

### Standard of Care GVHD Prophylaxis Exposure:

The exposure data of the standard of care GVHD prophylaxis of MTX plus CsA or MTX plus TAC will be collected. When subjects are dosed at the site, they will receive MTX and CsA or MTX plus TAC directly from the investigator or qualified study site staff, under medical supervision. The dose, date, and time of each dose administered and the reasons for dose changes (if any) at the site will be recorded in the source documents and relevant forms.

When subjects self-administer CsA or TAC at home, the compliance will be assessed at each visit and documented in the source documents and relevant forms. Deviation(s) from the prescribed dosage regimen should be recorded. A record of the quantity of treatment dispensed to and administered by each subject must be maintained and reconciled with compliance records. Treatment start and stop dates, including dates for treatment delays and/or dose reductions will also be recorded.

CsA and TAC level will be closely monitored.

### Dose Modification

All dosages prescribed and dispensed to the subject and all dose changes made by the investigator during the study will be recorded.

IMP dose modification, including dose reduction (down titration) and dose increase will not be allowed during the study.

The IMP can be temporarily interrupted for documented reasons, such as AEs. The circumstances surrounding the interruption of IMP must be discussed with the medical monitor and the IMP is to be restarted as soon as possible. These changes must be recorded in the source documents and the eCRF.

### Continued Access to Investigational Medicinal Product after the End of the Study

Mocravimod is an IMP under development and will, consequently, not be available for treatment of the subjects after the double-blind treatment phase completion, after relapse, or in case of study discontinuation by the subject or the sponsor.

After participation, subjects will be managed in accordance with local clinical practice, i.e. as per local guidelines and standard of care.

### Treatment of Overdose

Any daily dose greater than 3 capsules is a protocol deviation, is considered as an overdose. Any AEs due to the overdose should be reported.

Sponsor does not recommend specific treatment for an overdose.

In the event of an overdose, the investigator should:
- Contact the medical monitor immediately.
- Increase monitoring of the subject by 12-lead ECG for any bradycardia events for at least 3 days.
- Evaluate the subject to determine, in consultation with the medical monitor, whether IMP should be interrupted.
- Obtain a plasma sample for PK analysis within 2 days from the date of the last dose of IMP if requested by the medical monitor (determined on a case-by-case basis).

Document the quantity of the excess dose as well as the duration of the overdose.

### Concomitant and Prohibited Medication and Therapy

Any medication or vaccine (including over-the-counter or prescription medicines, vitamins, and/or herbal supplements) or therapy that the subject is receiving at the time of enrollment or receives during the study will be considered concomitant, except the IMP, HSCT, conditioning regimen, and MTX plus CsA or TAC used for GVHD prophylaxis. All concomitant medication must be recorded in the eCRF.

The medical monitor should be contacted if there are any questions regarding concomitant or prior therapy.

### Concomitant Medication and Therapy

### Prophylactic Treatment for Cytomegalovirus Infection

To prevent infections with cytomegalovirus (CMV), subjects who are CMV positive or have a CMV positive donor can be given prophylactic treatment including ganciclovir, valganciclovir, letermovir, and foscarnet, per institutional practice. All subjects will be subjected to regular quantitative polymerase chain reaction (PCR) monitoring regardless of chemoprophylaxis strategy.

CMV prophylaxis, treatment and indication for treatment of CMV reactivation should follow local SoC.

### Pre-emptive Treatment for Epstein-Barr Virus Infection

To prevent infections with Epstein-Barr virus (EBV), subjects who are EBV seropositive and/or have an EBV seropositive donor will be subjected to regular quantitative PCR monitoring followed by appropriate (pre-emptive) treatment, if indicated. If quantified viral DNA levels exceed the institutional threshold for treatment of EBV reactivation, subjects should be treated with rituximab. It is also recommended to start rituximab if a subject was EBV positive in the past and demonstrates enlarged lymph nodes, even if PCR for EBV is low or negative.

The following schedule is recommended:
- Immediately after the rise in EBV DNA is detected, rituximab (anti-CD20 monoclonal antibody) 375 mg/m² IV is started once weekly, until PCR for EBV becomes negative.
- If post-transplant lymphoproliferative disorder (PTLD) is suspected on the basis of clinical symptoms, computed tomography (CT) scans of neck, thorax, abdomen and pelvis, as well as bone marrow aspiration and biopsy, and when possible, lymph node biopsy should be conducted. A PET/CT should also be considered for diagnostic and response assessments. If results of the CT scan, bone marrow examinations, and lymph nodes demonstrate PTLD, rituximab should be administered weekly for at least 2 weeks, with subsequent dosing and additional treatment based on response.

### Pre-emptive Treatment of Human Herpesvirus-6 Reactivation

Human herpesvirus-6 (HHV-6) PCR prospective monitoring will occur at the discretion of the investigator based on subject risk and institutional standard of care. HHV-6 reactivation should also be suspected in the event a subject develops otherwise unexplained fever, erythematous rash, delayed engraftment or post-engraftment cytopenias, pneumonitis, encephalitis, or hepatitis. HHV-6-related symptoms or sufficient viral load (in the investigator's judgment) should prompt initiation of appropriate antiviral therapy, including the following options based on subject clinical condition and comorbidities:
- Foscarnet 90 mg/kg IV q2h or 60 mg/kg IV q8h
- Cidofovir 5 mg/kg IV weekly or 1 mg/kg IV three times weekly (with probenecid)
- Ganciclovir or valganciclovir

### Donor Selection

HLA-identical siblings are the preferred donor. In the absence of this option, an unrelated donor that is matched at HLA-A, -B, -C, -DRB1, and -DQB1 (10/10) based on DNA-based typing, is the second choice. When more than one suitable donor option is available, institutional algorithms for donor selection should be used, incorporating non-HLA characteristics such as donor age, gender, ABO compatibility, CMV serostatus, and parity, among others.

### Stem Cell Source and Graft Infusion

Peripheral blood stem cells obtained by leukapheresis after high-dose hematopoietic growth factor mobilization are the preferred stem cell source. The minimum CD34+ cell dose is 2×10⁶ cells/kg of recipient weight with a target dose of 5×10⁶ cells/kg recipient weight. The stem cell graft should be infused into the patient using institutional standard practices for premedication.

While infusion of fresh peripheral blood stem cells (PBSCs) is preferred, cryopreservation of previously collected cells may be necessary due to factors outside the investigator's control, such as COVID-19-related restrictions, and is permissible.

### Hematopoietic growth factor

Routine pre-emptive use of hematopoietic growth factor post-HSCT is permitted but not required. This can be initiated at a minimum 24 hours after stem cell infusion and continued until adequate neutrophil recovery.

### Other supportive care

Appropriate antimicrobial prophylaxis and monitoring should be employed based on institutional practices. Other than viral monitoring and prophylaxis/pre-emptive therapy described elsewhere, there are no study-specified antimicrobial prophylaxis regimens, and institutional standards should be observed. However, it is suggested that all subjects receive chemoprophylaxis against fungal and bacterial organisms (during at least the neutropenic period), and Pneumocystis (while lymphopenic or on immunosuppressive therapy), based on patient-specific factors and timing after HSCT. Subjects who develop GVHD may require re-initiation or augmentation of antimicrobial prophylaxis, including antibacterial prophylaxis against encapsulated organisms (in the event of cGVHD). In addition, intravenous immunoglobulin (IVIg) supplementation is allowable based on investigator preference.

Administration of blood product support during periods of pancytopenia should adhere to institutional standards; however, routine use of granulocyte transfusions is not permitted.

### Prohibited Medication and Therapy

The following medications or therapies are **prohibited** during the study:
- Remission maintenance therapy:
   ∘ Targeted treatment FLT3 inhibitor
   ∘ Hypo-methylating agents
   ∘ IDH inhibitors
   ∘ Bcl-2-inhibitors
   ∘ HDAC-inhibitors
   ∘ Any other maintenance therapy after Allo-HSCT including immunotherapy or any investigational drug
   ∘ Donor lymphocyte infusion (DLI) for relapse prophylaxis
      ▪ For instance, pre-emptive use of DLI in the event of MRD positive disease is not permitted
- GVHD *prophylaxis:*
   ∘ Pre-transplant ATG
   ∘ Post-transplant cyclophosphamide
   ∘ Mycophenolate mofetil
- Use of serotherapy during conditioning:
   ∘ ATG
   ∘ Alemtuzumab
- CYP3A4 inhibitor and inducer:
   ∘ Mocravimod is mainly metabolized by the enzyme CYP3A4 and strong CYP3A4 inhibitors and inducers should be avoided when possible.
- Live or live attenuated vaccines are prohibited while subjects are taking study treatment and for 2 months after study treatment discontinuation.
- Antihypertensives
   ∘ As bradycardia is an AESI of mocravimod, agents that commonly result in decreased heart rate should be avoided, such as calcium channel blockers and β-blockers.
   ∘ In the event of a life-threatening circumstance, the safety of the subject should take precedence, and these agents should be used at the discretion of the investigator.
   ∘ For routine treatment of hypertension, other classes of agents should be employed, if possible. Calcium channel blockers and β-blockers can be utilized if other options are contraindicated, and the subject has not demonstrated bradycardia after IMP exposure, after discussion with the medical monitor.

### Dose Selection after Study Completion

Should no safety findings be observed in the 3 mg arm and should efficacy be comparable between 1 mg and 3 mg at the time of the analysis for the primary endpoint, 3 mg/day mocravimod will be selected for upcoming trials and submissions.

### Efficacy Assessments

### Disease Assessment

AML disease assessment will be assessed at the local laboratory by bone marrow aspirate and/or biopsy for morphology at visits, until confirmation of relapse. AML disease assessment will also be performed in case of suspected relapse happening in-between study visits. MRD positivity without morphologic relapse is not considered being relapse. For subjects with a history of extramedullary disease, radiographic (and/or cerebrospinal fluid [CSF]) evaluations will be included for disease evaluation.

A morphologic relapse is defined as morphological evidence of leukemia in the bone marrow (≥ 5% leukemic blasts) or appearance of blasts in the peripheral blood or at other extra-medullary sites. Details of relapse will be recorded in the eCRF.

If a bone marrow aspirate and/or biopsy had already been obtained between the monthly visits during the double-blind treatment phase or during the RFS follow up phase, or within 6 weeks prior to a scheduled visit during the OS follow-up phase, the assessment does not need to be repeated. In addition, in case of suspected relapse post allo-HSCT, chimerism will be assessed to support the diagnosis.

A subject, who relapses during the double-blind treatment phase will discontinue the IMP treatment. The subject will move to the OS follow-up phase and will be observed for an additional 12 months.

### Mortality Assessment

Mortality assessment will be conducted at visits.

After a subject's death, the following information must be recorded in the eCRF:
- Date of death
- Cause of death (specification)
- Investigator classification of cause of death:
   ∘ Leukemia relapse
   ∘ Transplant-related mortality (TRM) defined as death due to causes other than disease relapse or disease progression
   ∘ Other

### Graft-Versus-Host Disease Assessment

GVHD assessment will be conducted at visits. Overall GVHD events will be categorized based on consensus criteria. aGVHD will be graded according to the MAGIC scale (Harris et al 2016). cGVHD will be graded according to National Institutes of Health (NIH) criteria (Filipovich et al 2005; Jagasia et al 2015).

| **Category** | **Time of symptoms after HSCT** | **Presence of aGVHD features** | **Presence of cGVHD features** |
|---|---|---|---|
| **Acute GVHD (aGVHD)** | | | |
| Classic aGVHD | ≤ 100 days | Yes | No |
| Persistent, recurrent, or late-onset aGVHD | > 100 days | Yes | No |

| **Chronic GVHD (cGVHD)** | | | |
|---|---|---|---|
| Classic cGVHD | No time limit | No | Yes |
| Overlap syndrome | No time limit | Yes | Yes |

| | | | |
|---|---|---|---|
| GVHD: graft-versus-host disease Source: Harris et al 2016; Jagasia et al 2015 | | | |

Whenever deemed possible, tissue biopsies will be obtained to confirm the diagnosis of GVHD and to assess its severity. However, acute and chronic GVHD remain clinical diagnoses, which are considered present when diagnosed and treated, even in the absence of biopsy confirmation.

Details of all GVHD events will be recorded in the eCRF. For cGVHD and aGVHD events, the use of systemic immunosuppressive treatment will be recorded. The start date of the GVHD event is defined as the date of initiation of GVHD treatment or the date of biopsy confirmation of GVHD, whichever is earlier.

Treatment of GVHD will be at the discretion of the investigator, based on GVHD organ involvement, severity, and patient clinical condition. Details of GVHD-directed therapies will be recorded in the eCRF, including onset of treatment, dose, duration (start/stop), and timing and magnitude of best response. This includes non-absorbable enteral steroids for upper/lower GI GVHD and topical therapies for skin, oral, or ocular GVHD.

### Quality of Life Assessment

The Foundation for the Accreditation of Cellular Therapy-Bone Marrow Transplantation (FACT BMT, version 4) is a 47-item self-report questionnaire that assess multiple domains, including physical, functional, social/family, emotional well-being, and transplant-specific concerns. The questionnaire will be scored at visits.

### Exploratory Immune-related Assessments

### Engraftment

Engraftment will be assessed at visits.

Neutrophil engraftment is defined as neutrophil count of ≥ 0.5×109/L for 3 consecutive days and platelet recovery is defined as platelets count of ≥ 20×109/L for 3 consecutive days, without transfusion within prior 7 days. The first days of occurrence of both criteria will be recorded.

Primary graft failure is defined as lack of initial engraftment of donor cells. In this case, the subject never recovers from neutropenia (neutrophil count of < 0.5×109/L), resulting in pancytopenia and an urgent need for re-transplantation. Secondary graft failure is defined as loss of donor cells after initial engraftment. In this case, autologous recovery is common; however, marrow aplasia and pancytopenia may also develop.

### Chimerism

Chimerism will be assessed in whole blood by PCR amplification at the local laboratory at visits . Chimerism will also be assessed in case of suspected relapse.

### Immunophenotyping

Blood samples will be collected for immunophenotyping at visits. Biomarkers (T/B/NK panel) will include: CD3 (CD3 T cells), CD4 (CD4 T cells), CD8B (CD8 T cells), LRP5 (B cells), OSBPL5 (NK cells). Potential expansion of the T/B/NK panel may be considered in the future.

Immunophenotyping will be carried out by a central laboratory, using a validated analytical method and in accordance with SOP or laboratory manual. Details regarding the sample processing, handling, storage, and shipment will be provided separately in the study-specific central laboratory manual prior to the initiation of the study.

### Exploratory Pharmacokinetics Assessment

### Mocravimod

Blood samples will be collected for the determination of mocravimod systemic concentrations at visits. One pre-dose sample will be collected at each PK visit. Trough level will be determined at all PK visits and blood concentration-time profiles over 12 hours will be performed on Day -9, Month 1, and Month 6.

On Day -9, Month 1, and Month 6, a standard 12-lead ECG assessment followed by pulse and blood pressure measurements should be conducted 5 minutes before the PK blood sample collection at 6 h post-dose. The exact date and sampling time will be recorded.

The exact date and sampling time for blood samples collection and ECG assessment will be recorded in the eCRF.

Bioanalytical determinations will be carried out by a central laboratory, using a validated LC-MS/MS analytical method and in accordance with SOP and laboratory manual. All blood samples will be taken by either direct venipuncture or an indwelling cannula inserted in a forearm vein. Blood samples (2 mL) for PK evaluation will be collected into an EDTA tube. EDTA tubes will be stored in a freezer at site until shipment to the central laboratory. Details regarding the sample processing, handling, storage, and shipment will be provided separately in the study-specific central laboratory manual prior to the initiation of the study.

Blood samples for PK analyses may be used for exploratory metabolite identification using non-validated cold metabolite identification methods. The PK (and optional metabolic) evaluation will be reported separately.

Results will not be disclosed to the sponsor, the study staff, and the subject before study data unblinding.

### Cyclosporine AlTacrolimus

Blood samples will be collected for the determination of CsA/TAC systemic concentrations at visits. The assessment will be conducted per local practice. CsA/TAC target concentration ranges are described in Section "Graft-versus-Host Disease prophylaxis". The exact date and sampling time will be recorded in the eCRF.

### Statistical Analysis

In general, measured variables and derived parameters will be listed by subject and tabulated. Tabulation of results will be displayed by treatment arm and overall population, and by visit when applicable. Data of all study sites will be pooled for statistical analysis.

Unless otherwise specified, continuous variables will be summarized descriptively with number of subjects, mean, median, standard deviation (SD), interquartile range (IQR), range (minimum and maximum), and 95% confidence interval (CI) of mean and median (when appropriate). Categorical variables will be summarized by frequencies and percentages of subjects and/or number of events (if applicable). Number of missing values will also be specified, if any. For summary tables by visit, the number of subjects with missing values will include those subjects with a missing assessment or visit up to the treatment discontinuation visit.

The baseline value is defined as the last non-missing value prior to randomization, unless otherwise described.

Time-to-event endpoints will be analyzed using Kaplan-Meier methods to estimate the survival distribution, median time-to-event with 95% CI and survival probabilities at selected time points; numbers of subjects at risk, subjects with an event, subjects censored at selected timepoints will also be reported.

Unless specified otherwise, descriptive statistics (cumulative incidences or proportion of subjects free of the event of interest) will be presented for time to event endpoints.

All statistical tests will be 2-sided and carried out at the 0.05 α level, unless otherwise specified.

Protocol deviations, including what generally constitutes major (important) protocol deviations may be detailed in the SAP in accordance with the ICH guidelines. All protocol deviations will be reviewed and finally classified as either major or minor in a data review meeting prior to the primary analysis.

### Demographics and Baseline Characteristics

All background and demographic data, including baseline and disease characteristics, will be tabulated, and listed based on ITT.

Medical and surgical history data will be summarized by Medical Dictionary for Regulatory Activities (MedDRA) system organ class (SOC) and preferred term (PT), and included in data listings.

### Treatment Exposure

All study treatment data will be summarized using the SAF set, including the IMP and the standard of care GVHD prophylaxis backbone (MTX plus CsA or MTX plus TAC).

At least the following variables will be summarized, together with the changes in the dose schedule and the reasons of those changes:
- ***Overall treatment exposure*** (weeks) is the time interval between the date of last dosing and the date of first dosing and includes periods of temporary interruptions of study treatment.
- ***Clinically relevant dose interruptions*** are those dose interruptions of study treatment due to AEs and lasting > 15 days.
- ***Overall treatment duration*** (weeks) is the cumulative number of days during which subjects received the assigned study treatment (i.e. interruption periods are not included in this calculation).
- ***Actual dose intensity*** (mg/day) is the actual dose received during the whole study (total dose) divided by the duration of exposure. For dose interruption period, the dose is equal to zero. Where the *actual dose* is the cumulative daily dose (mg) over the period. Actual dose intensity will be summarized using descriptive statistics.

### Prior and Concomitant Medications

Medications that ended prior to the start of the IMP and medications taken after the start of the IMP will be summarized as frequency statistics by category of medication using Anatomical Therapeutic Chemical (ATC) code from World Health Organization (WHO) drug dictionary. Prohibited medication will be also summarized.

Concomitant medications taken during the double-blind treatment phase will be presented separately from those taken during the follow-up phases.

### Efficacy Analyses

Efficacy analyses will be performed based on ITT.

Safety review will be performed by an IDMC.

### 1)Primary Endpoint Analysis

The primary endpoint is RFS at Month 12. The 3 mg arm vs placebo will be assessed unless the 3 mg arm is discontinued due to safety findings; in that case, the 1 mg arm vs placebo will be assessed. The analysis is a landmark analysis at Month 12, i.e. only data from the first 12 months (the planned treatment period) of each subject will be included in the analysis. The variable for the primary endpoint is defined as the duration from randomization to the first occurrence of disease relapse or death (of any cause).

The primary efficacy variable will be described using visual representation of the Kaplan-Meier estimates as well as a table reporting those estimates by a quarterly interval. Estimates of median will be provided with two-sided 95% CIs, along with the 25th and 75th percentiles, together with the hazard ratio estimates.

A stratified log-rank test, using stratification factors as used for the subject randomization, will be performed to compare the mocravimod arm versus the placebo arm.

Additionally, after confirmation of proportional hazards, a Cox-regression model will be used to compare the treatment arms. The list of factors to be used for adjustment in the model may include but is not limited to the following: complete remission status (CR1 versus CR2), GVHD prophylaxis treatment (CsA versus TAC), age, donor type (sibling versus unrelated), donor recipient sex combinations, disease stage of AML, and time interval from diagnosis to transplantation.

Subjects without event at the time of the analysis (i.e. neither relapse or death) will be censored at the last date they were known to be relapse-free or alive or at the end of their 12 months since randomization, whichever comes first.

Time to censoring will also be described using a reverse Kaplan-Meier plot and presented and tabulated over the considered period by a monthly interval.

The distribution of the component defining the primary endpoint, i.e. the earliest, will be tabulated by treatment arm.

Additionally, the overall follow-up times from randomization will be described using a plot of Kaplan-Meier estimates, for each of the treatment arm and will be compared through a log-rank test.

The overall follow-up times are defined, respectively, as the time intervals (weeks) from randomization to the date of first occurrence of the event, or up to the date of censoring if no event occurred at the time of analysis.

It is noteworthy that if a treatment arm is stopped due to safety or lower efficacy, subjects who choose to switch doses will not be included in the primary analysis. The impact of the potential treatment switch will be explored and discussed in the SAP.

### 2)Sensitivity Analyses

The impact of the potential deviation from the proportional hazards assumed in this study will be assessed using the combinations of Fleming-Harrington weighted log-rank statistics.

Other sensitivity analyses will be detailed in the SAP.

### 3)Secondary Efficacy Endpoints Analyses

### Key Secondary Efficacy Analysis

The first key secondary endpoint is OS assessed after a 12-month OS Follow-up period of each subject, compared between the mocravimod arm and the placebo arm. The 3 mg arm vs placebo will be assessed unless the 3 mg arm is discontinued due to safety findings; in that case, the 1 mg arm vs placebo will be assessed. The second key secondary endpoint is the RFS assessed after the double-blind treatment period or the RFS Follow up period at Month 12, compared between the second mocravimod arm and the placebo arm, if applicable.

The corresponding analysis variable is defined as the time interval from date of randomization to date of death due to any cause within first 24 months, and the analysis will be performed when all subjects have completed the study (i.e. 24 months and includes the follow-up period).

OS analysis will be performed using the same approach as the primary endpoint. Subjects who are still alive at the end of the 24-month study follow-up or lost to follow-up will be censored at the last time they were known to be alive.

Information on death occurring within the 24 months will be collected for all subjects randomized in the study.

The statistical hypotheses and analysis for the second key secondary endpoint are the same as the primary endpoint.

### Other Secondary Analyses

The other secondary variables, to be analyzed in the ITT, will include:
- RFS after Month 24
- Time to relapse
- Cumulative incidence of relapse at Month 12 and at Month 24
- Non-relapse mortality at Month 12 and Month 24
- OS of second dose of Mocravimod versus placebo (only applicable if all 3 arms reach the primary analysis)
- Survival free from Grade III.IV aGVHD at Month 12
- Time to aGVHD
- Survival free from moderate/severe cGVHD at Month 24
- Time to cGVHD
- GRFS at Month 12 and at Month 24
- rGRFS at Month 12 and at Month 24
- Safety
- Quality of life

### Survival free from Grade III/IV aGVHD at Month 12

Survival free from Grade III/IV aGVHD is defined as the time interval (weeks) from randomization until death from any cause or the first occurrence of Grade III/IV aGVHD as defined by the MAGIC score, whichever occurs first.

If no event is reported for a subject, observation will be censored at the last date the subject was known to be free from Grade III/IV aGVHD.

Survival free from Grade III/IV aGVHD will be summarized by treatment arm using a Kaplan Meier plot and survival estimate summary. Stratified log-rank test, using stratification factors as used for the subject randomization, will be used to assess the existence of a statistically significant difference between each mocravimod arm and the placebo arm separately.

Additionally, as a supportive analysis, after confirmation of proportional hazards, a Cox-regression model will be used to compare the treatment arms. The list of factors to be used for adjustment in the model may include but is not limited to the following: complete remission status (CR1 versus CR2), GVHD prophylaxis treatment (CsA versus TAC), age, donor type (sibling versus unrelated), donor recipient sex combinations, disease stage of AML, and time interval from diagnosis to transplantation.

Grade III/IV aGVHD at Month 12 will be summarized as the cumulative proportion of subjects with at least one occurrence of a Grade III/IV aGVHD during the RFS follow-up period. Each mocravimod arm will be compared separately with the placebo using a stratified CMH test; the odds ratio and the corresponding 95% CI will be provided.

### Time to aGVHD

Time to aGVHD is defined as the time interval (weeks) from randomization until first occurrence of Grade III/IV aGVHD as defined by the MAGIC.

Time to aGVHD, only for patients with occurrence of Grade III/IV aGVHD, will be summarized by treatment arm descriptively, using mean, standard deviation, minimum, lower quartile, median, upper quartile, and maximum.

Should the descriptive summary be insufficient, due to imbalance, or insufficient events in the groups for comparison, then further exploration will be performed including all subjects. For this analysis, if a subject died before the occurrence of Grade III/IV aGVHD, the date of death will be used as a final follow-up time and death will be considered as a competing event. If no occurrence of Grade III/IV aGVHD or death is reported for a subject, Time to aGVHD will be censored at the last date the subject was known to be free from aGVHD.

Time to aGVHD will be summarized by treatment arm using a cumulative incidence function plot and survival estimate summary. A Gray test using the stratification factors used for the subject randomization, will be used to assess the existence of a statistically significant difference between the each mocravimod arm and the placebo arm separately.

A Competing risk model will be used to provide an adjusted estimate of treatment effect. The list of factors to be used for adjustment in the model may include but is not limited to the following: complete remission status (CR1 versus CR2), GVHD prophylaxis treatment (CsA versus TAC), age, donor type, donor recipient sex combinations, disease stage of AML, and time interval from diagnosis to transplantation.

### Survival free from moderate/severe cGVHD at Month 24

Survival free from moderate/severe cGVHD at Month 24 is defined as the time interval (weeks) from randomization until the first occurrence of moderate/severe cGVHD refractory to systemic immunosuppressive treatment during the whole study period.

If no event is reported for a subject, observation will be censored at the last date the subject was known to be free from moderate/severe cGVHD.

Survival free from moderate/severe cGVHD will be summarized by treatment arm using a Kaplan-Meier plot and survival estimate summary. Stratified log-rank test, using stratification factors as used for the subject randomization, will be used to assess existence of a statistically significant difference between each mocravimod arm and placebo separately.

A Cox-regression model will be used to provide an adjusted estimate of treatment effect on remission status, GVHD prophylaxis treatment (CsA versus TAC), age, donor type, donor recipient sex combinations, disease stage of AML, and time interval from diagnosis to transplantation.

Moderate/severe cGVHD at Month 24 will be summarized as the cumulative proportion of subjects with at least one occurrence of a moderate/severe cGVHD during the whole study period. Each mocravimod arm will be compared separately with the placebo using a stratified Cochran-Mantel-Haenszel (CMH) test; the odds ratio and the corresponding 95% CI will be provided.

### Time to cGVHD

Time to cGVHD is defined as the time interval (weeks) from randomization until first occurrence of moderate/severe cGVHD.

Time to cGVHD, only for patients with occurrence of moderate/severe cGVHD, will be summarized by treatment arm descriptively, using mean, standard deviation, minimum, lower quartile, median, upper quartile, maximum and CI of mean and median (when appropriate).

Should the descriptive summary be insufficient, due to imbalance, or insufficient events in the groups for comparison, then further exploration will be performed including all subjects. For this analysis, if a subject died before the occurrence of moderate/severe cGVHD, the date of death will be used as a final follow-up time and death will be considered as a competing event. If no occurrence of moderate/severe cGVHD or death is reported for a subject, Time to cGVHD will be censored at the last date the subject was known to be free from cGVHD.

Time to cGVHD will be summarized by treatment arm using a cumulative incidence function plot and survival estimate summary. A Gray test using the stratification factors used for the subject randomization, will be used to assess the existence of a statistically significant difference between each mocravimod arm and the placebo arm separately.

A Competing risk model will be used to provide an adjusted estimate of treatment effect. The list of factors to be used for adjustment in the model may include but is not limited to the following: complete remission status (CR1 versus CR2), GVHD prophylaxis treatment (CsA versus TAC), age, donor type, donor recipient sex combinations, disease stage of AML, and time interval from diagnosis to transplantation.

### Non-relapse mortality at Month 12 and at Month 24

Non-relapse mortality is defined as death without evidence of leukemia recurrence.

If sufficient events occur, the cumulative incidence of non-relapse mortality at Month 12 will be summarized by treatment arm with 95% confidence interval.

A CMH stratified test, using stratification factors as used for the subject randomization, will be performed and odds-ratio will be presented comparing each mocravimod arm with placebo arm separately.

If a subject relapses, the date of relapse will be used as a final follow-up time and relapse will be considered as a competing event. If no occurrence of non-relapse mortality is reported for a subject, Time to non-relapse mortality will be censored at the last date the subject was known to be free from mortality.

Non-relapse mortality, if sufficient events occur, will be summarized by treatment arm using a cumulative incidence function plot and survival estimate summary. A Gray test using the stratification factors used for the subject randomization, will be used to assess the existence of a statistically significant difference between the each mocravimod arm and the placebo arm separately.

A Competing risk model will be used to provide an adjusted estimate of treatment effect. The list of factors to be used for adjustment in the model may include but is not limited to the following: remission status (CR1 versus CR2), GVHD prophylaxis treatment (CsA versus TAC), age, donor type, donor recipient sex combinations, disease stage of AML, and time interval from diagnosis to transplantation.

Additionally, non-relapse mortality at Month 24 will be analyzed using same approach as for the non-relapse mortality at Month 12 analysis.

### GRFS at Month 12 and at Month 24

GVHD is defined as Grade III/IV aGVHD or moderate/severe cGVHD.

Survival free from GVHD at Month 12 and Month 24 is defined as the time interval (weeks) from randomization until death from any cause, or the first occurrence of GVHD, or relapse, whichever occurs first.

If no event is reported for a subject, observation will be censored at the last date the subject was known to be free from GVHD.

Survival free from GVHD will be summarized by treatment arm using a Kaplan-Meier plot and survival estimate summary. Stratified log-rank test, using stratification factors as used for the subject randomization, will be used to assess existence of a statistically significant difference between each mocravimod arm and placebo separately.

GVHD at Month 24 will be summarized as the cumulative proportion of subjects with at least one occurrence of a moderate/severe cGVHD during the whole study period. Each mocravimod arm will be compared separately with the placebo using a stratified CMH test; the odds ratio and the corresponding 95% CI will be provided.

### rGRFS at Month 12 and at Month 24

rGRFS at Months 12 and Month 24 will be summarized as the cumulative proportion of subjects with at least one occurrence of the events defining rGRFS during the whole study period. rGRFS is defined as the duration (weeks) from randomization to the first occurrence of (Kawamura et al 2018):
- Grade III/IV active aGVHD not resolved despite treatment
- Active cGVHD not resolved despite requiring systemic treatment
- Disease relapse
- Death from any cause

GVHD that resolved and did not require systemic treatment at the last evaluation is not considered as an event.

rGRFS will be summarized by treatment arm using a Kaplan-Meier plot and survival estimate summary. Stratified log-rank test, using stratification factors as used for the subject randomization, will be used to assess existence of a statistically significant difference between each mocravimod arm and placebo separately.

A stratified CMH test, using stratification factors as used for the subject randomization, for Month 12 and Month 24 separately, between the each mocravimod arm and the placebo arm separately will be performed; and the odds ratio and the corresponding 95% CI will be provided.

### Other Efficacy Analyses

### Relapse-free survival at Month 24

RFS is defined as the time interval (weeks) from randomization until disease relapse or death from any cause, whichever occurs first, and will be analyzed at Month 24.

If no death nor relapse is reported for a subject, RFS will be censored at the last date the subject was known to be free from relapse.

RFS will be summarized by treatment group using a Kaplan-Meier plot and survival estimate summary. A log-rank test using the stratification factors used for the subject randomization, will be used to assess existence of a statistically significant difference between each mocravimod arm and the placebo arm separately.

Additionally, after confirmation of proportional hazards, a Cox-regression model will be used to compare the treatment arms. The list of factors to be used for adjustment in the model may include but is not limited to the following: complete remission status (CR1 versus CR2), GVHD prophylaxis treatment (CsA versus TAC), age, donor type (sibling versus unrelated), donor recipient sex combinations, disease stage of AML, and time interval from diagnosis to transplantation.

### Cumulative Incidence of Relapse at Month 12 and Month 24

The cumulative incidence of relapse at Month 12 and Month 24 will be summarized by treatment arm with 95% confidence interval.

A CMH stratified test, using stratification factors as used for the subject randomization, between each mocravimod arm and placebo separately, will be performed and odds-ratio will be presented.

Additionally, cumulative incidence of relapse at Month 24 will be analyzed using same approach as for the cumulative incidence of relapse at the primary analysis.

### Time to relapse (TTR)

TTR is defined as the time interval (weeks) from randomization until disease relapse.

TTR, only for subjects with relapse, will be summarized by treatment arm descriptively, using mean, standard deviation, minimum, lower quartile, median, upper quartile, maximum.

Should the descriptive summary be insufficient, due to imbalance, or insufficient events in the groups for comparison, then further exploration will be performed including all subjects. For this analysis, if a subject died before the occurrence of relapse, the date of death will be used as a final follow-up time and death will be considered as a competing event. If no occurrence of relapse or death is reported for a subject, Time to relapse will be censored at the last date the subject was known to be free from relapse.

TTR will then be summarized by treatment arm using a cumulative incidence function plot and survival estimate summary.

A Competing risk model may also be used. The model may include factors such as, but not limited to remission status (CR1 versus CR2), GVHD prophylaxis treatment (CsA versus TAC), age, donor type, donor recipient sex combinations, disease stage of AML, and time interval from diagnosis to transplantation.

## Claims

1. An S1P receptor modulator, for use in treating hematological malignancies, in a subject undergoing allogeneic hematopoietic stem cell transplant (HSCT), wherein said S1P receptor is daily administered for at least 6 months.

2. An S1P receptor modulator, for use according to Claim 1, wherein said hematological malignancy is acute myeloid leukemia (AML).

3. The S1P receptor modulator, for use according to claim 1 or 2, wherein said S1P receptor modulator is selected among mocravimod, FTY720, siponimod, fingolimod, ozanimod, ponesimod, etrasimod, AKP-11, cenerimod, amiselimod, CBP-307, OPL-307, OPL-002, BMS-986166, SCD-044, BOS-173717, CP-1050.

4. The S1P receptor modulator, for use according to any one of claims 1-3, wherein the S1P receptor modulator is a S1P receptor agonist of the following formula (I) or (II) or (IIa) or (IIb): 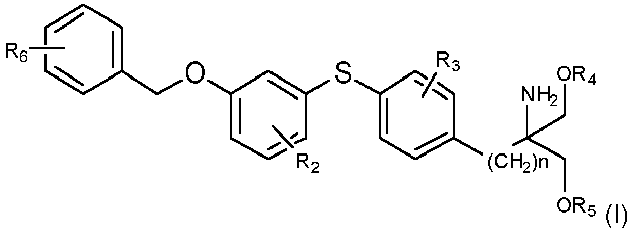 wherein
R₂ is H, halogen, trihalomethyl, C₁₋₄alkoxy, C₁₋₇alkyl, phenethyl or benzyloxy;
R₃ is H, halogen, CF₃, OH, C₁₋₇alkyl, C₁₋₄alkoxy, benzyloxy, phenyl or C₁₋₄alkoxymethyl;
each of R₄ and R₅, independently is H or a residue of formula (a) 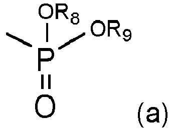
wherein each of R₈ and R₉, independently, is H or C₁₋₄alkyl optionally substituted by halogen;
and n is an integer from 1 to 4; and
R₆ is hydrogen, halogen, C₁₋₇alkyl, C₁₋₄alkoxy or trifluoromethyl, 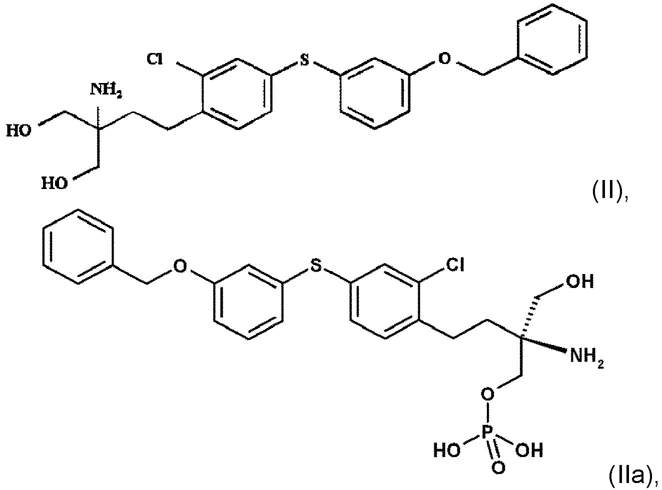 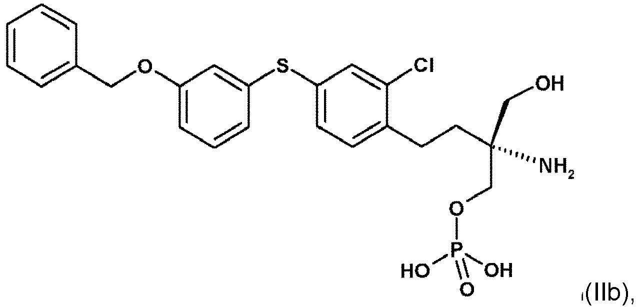
or pharmaceutically acceptable salts thereof.

5. The S1P receptor modulator, for use according to any one of claims 1-4, which is mocravimod, or a pharmaceutically acceptable salt thereof.

6. The S1P receptor modulator for use according to any one of claims 1-5, wherein said S1P receptor modulator is daily administered for at least 12 months, or more, or until relapse.

7. The S1P receptor modulator for use according to any one of claims 1-6, wherein said S1P receptor modulator is daily administered from a starting day between 1-14 days prior to HSC transplant, preferably 11 days before HSC transplant.

8. The S1P receptor modulator for use according to any one of claims 1-7, wherein said S1P receptor modulator is mocravimod, and said mocravimod is administered at a daily dose of 3mg.

9. The S1P receptor modulator for use according to any one of claims 1-7, wherein said S1P receptor modulator is mocravimod, and said mocravimod is administered at a daily dose of 1mg.

10. The S1P receptor modulator for use according to any one of claims 1-7, wherein said hematological malignancy is acute myeloid leukemia, said S1P receptor modulator is mocravimod, said mocravimod is administered at a daily dose of 1mg or 3 mg for at least 12 months.

11. The S1P receptor modulator for use according to any one of claims 1-10, wherein said S1P receptor modulator is mocravimod, and said mocravimod is formulated as a solid dosage form, said solid dosage form comprising:
- mannitol;
- microcrystalline cellulose ;
- sodium starch glycolate;
- magnesium stearate; and
- colloidal silicon dioxide.

12. The S1P receptor modulator for use according to any one of claims 1-11, wherein said S1P receptor modulator is mocravimod, and said mocravimod is formulated as a solid dosage form, said solid dosage form comprising:
- mannitol at a content from 48 to 88 mg/unit;
- microcrystalline cellulose at a content from 5 to 45 mg/unit;
- sodium starch glycolate at a content from 1 to 8 mg/unit;
- magnesium stearate at a content from 0.025 to 4 mg/unit; and
- colloidal silicon dioxide at a content from 0.125 to 2 mg/unit.

13. The S1P receptor modulator for use according to any one of claims 1-10, wherein said S1P receptor modulator is mocravimod, and said mocravimod is formulated as a solid dosage form, said solid dosage form comprising:
- mannitol at a content from 58 to 78mg/unit;
- microcrystalline cellulose at a content from 15 to 35 mg/unit;
- sodium starch glycolate at a content from 2 to 6 mg/unit;
- magnesium stearate at a content from 0.5 to 2 mg/unit; and
- colloidal silicon dioxide at a content from 0.25 to 1 mg/unit.

14. The S1P receptor modulator for use according to any one of claims 1-10, wherein said S1P receptor modulator is mocravimod, and said mocravimod is formulated as a solid dosage form, said solid dosage form comprising:
- mannitol at a content about 68 mg/unit;
- microcrystalline cellulose at a content about 25 mg/unit;
- sodium starch glycolate at a content about 4 mg/unit;
- magnesium stearate at a content at a content about 1 mg/unit; and
- colloidal silicon dioxide at a content about 0.5 mg/unit.
